(19) Europäisches Patentamt
European Patent Office
Office européen des brevets

(11) Veröffentlichungsnummer: **0 022 494**
**B1**

(12) # EUROPÄISCHE PATENTSCHRIFT

(45) Veröffentlichungstag der Patentschrift: **23.02.83**

(21) Anmeldenummer: **80103612.0**

(22) Anmeldetag: **26.06.80**

(51) Int. Cl.³: **C 07 D 499/64,** C 07 D 501/20, A 61 K 31/43, A 61 K 31/545 // C07D401/12, C07D403/12, C07D405/12, C07D405/14, C07D409/12, C07D409/14, C07D413/12, C07D417/12, C07D239/36

(54) **Neue Lactame, Verfahren zu ihrer Herstellung und diese Verbindungen enthaltende Arzneimittel.**

(30) Priorität: **13.07.79 DE 2928344**

(43) Veröffentlichungstag der Anmeldung: **21.01.81 Patentblatt 81/3**

(45) Bekanntmachung des Hinweises auf die Patenterteilung: **23.02.83 Patentblatt 83/8**

(84) Benannte Vertragsstaaten: **AT BE CH DE FR GB IT LI LU NL SE**

(56) Entgegenhaltungen:
**EP-A-0 003 814**
**EP-A-0 006 532**
**DE-A-2 450 668**
**DE-A-2 714 214**
**FR-A-2 370 052**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 720, D-7950 Biberach (Riss) (DE)**

(72) Erfinder: **Wetzel, Bernd, Dr. Dipl.-Chem., Kapellenweg 21, D-7950 Biberach 1 (DE)**
Erfinder: **Woitun, Eberhard, Dr. Dipl.-Chem., Stecherweg 17, D-7950 Biberach 1 (DE)**
Erfinder: **Reuter, Wolfgang, Dr. Dipl.-Chem., Nelkenweg 10, D-7951 Laupertshausen (DE)**
Erfinder: **Maier, Roland Dr. Dipl.-Chem., Bodelschwinghstrasse 39, D-7950 Biberach 1 (DE)**
Erfinder: **Lechner, Uwe, Dr., Panoramastrasse 12, D-7951 Ummendorf (DE)**
Erfinder: **Goeth, Hanns, Dr., Oberer Bühl 6, D-7950 Biberach 1 (DE)**

EP 0 022 494 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Lactame, Verfahren zu ihrer Herstellung und diese Verbindungen enthaltende Arzneimittel

Die Erfindung betrifft neue β-Lactame der allgemeinen Formel I

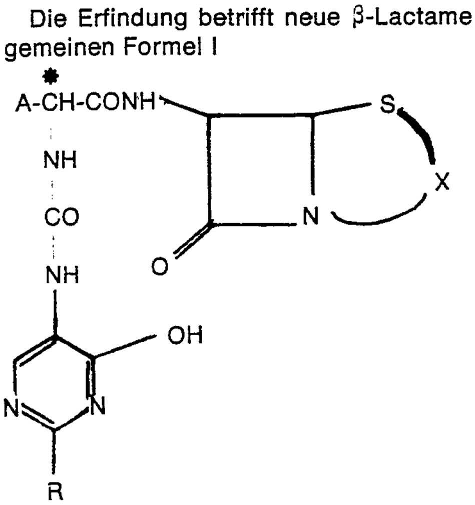

(I)

gegebenenfalls ihre physiologisch verträglichen Salze mit anorganischen oder organischen Basen, Verfahren zur Herstellung der Verbindungen und diese Verbindungen enthaltende Arzneimittel.

Die Verbindungen der allgemeinen Formel I zeichnen sich gegenüber den chemisch nächstverwandten Verbindungen der DE-A 2 714 214, FR-A 2 370 052 und DE-A 2 450 668 durch wesentlich bessere Wirkungen, insbesondere gegen gramnegative Problemkeime aus.

In der obigen allgemeinen Formel I bedeutet
A die Phenyl-, 4-Hydroxyphenyl-, 2- oder 3-Thienyl-, 2- oder 3-Furyl-, Cyclohexyl-, Cyclohexen-1-yl-, Cyclohexa-1,4-dien-1-ylgruppe sowie einen in 3,4-Stellung disubstituierten Phenylrest, wobei die Substituenten dieses Restes gleich oder voneinander verschieden und Chloratome, Hydroxy- oder Methoxygruppen sein können,

R eine Gruppe der Formel $NH(CH_2)_nR_1$, worin n 0 oder 1 und $R_1$ einen gegebenenfalls substituierten 5- oder 6gliedrigen heterocyclischen Rest mit vorzugsweise 1 bis 4, insbesondere 1 oder 2 gleichen oder verschiedenen Heteroatomen, wie Sauerstoff, Schwefel oder Stickstoff bedeutet, beispielsweise eine gegebenenfalls substituierte Thienyl-, Furyl-, Pyrrolyl-, Oxazolyl-, Isoxazolyl-, Thiazolyl-, Isothiazolyl-, Imidazolyl-, Pyrazolyl-, Oxdiazolyl-, Thiadiazolyl-, Triazolyl-, Tetrazolyl-, Pyridyl-, Pyrazinyl-, Pyridazinyl-, Pyrimidinyl- oder Tetrahydrofuranylgruppe, wobei diese Gruppen durch folgende Reste substituiert sein können:
durch Alkylgruppen, durch Halogenatome, wie Fluor, Chlor oder Brom, durch Nitro-, Cyano-, Amino-, Alkyl- oder Dialkylaminogruppen, durch Alkylcarbonylamino- oder Alkoxycarbonylaminogruppen, durch Hydroxy-, Alkoxy-, Alkylthio-, Alkylsulfinyl- oder Alkylsulfonylgruppen, durch Methylsulfonylamino-, Aminocarbonyl-, Alkylcarbonyloxy- oder Alkoxycarbonylgruppen, Aminosulfonyl-, Alkylamino- oder Dialkylaminosulfonylgruppen, wobei die Alkylgruppen in diesen Resten jeweils 1-4 Kohlenstoffatome enthalten können, sowie durch die Carbonsäure- oder Sulfonsäuregruppe;
und
X die Gruppen:

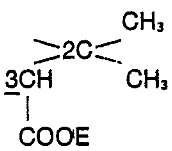

und

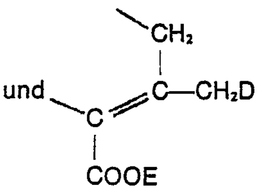

worin
D ein Wasserstoffatom, eine Hydroxy-, Acetoxy-, Aminocarbonyloxy-, Pyridinium oder 4-Aminocarbonyl-pyridiniumgruppe oder die Gruppe -SHet bedeutet, wobei Het die Tetrazol-5-yl-, 1-Methyl-tetrazol-5-yl-, 1,2,4-Thiadiazol-5-yl-, 3-Methyl-1,2,4-thiadiazol-5-yl, 1,3,4-Thiadiazol-2-yl-, 2-Methyl-1,3,4-thiadiazol-5-yl-, 2-Methylamino-1,3,4-thiadiazol-5-yl-, 2-Dimethylamino-1,3,4-thiadiazol-5-yl-, 2-Formylamino-1,3,4-thiadiazol-5-yl-, 2-Acetylamino-1,3,4-thiadiazol-5-yl-, 2-Methyl-1,3,4-oxadiazol-5-yl-, 1,2,3-Triazol-4-yl- oder die 1,2,4-Triazol-3-yl-gruppe darstellt und

E ein Wasserstoffatom oder eine in vitro oder in vivo leicht spaltbare Schutzgruppe darstellt. Als Carboxylschutzgruppen kommen im Sinne der Erfindung solche in Frage, welche auch bisher schon auf dem Gebiet der Penicilline und Cephalosporine eingesetzt wurden, insbesondere esterbildende Gruppen, die durch Hydrogenolyse oder Hydrolyse oder andere Behandlungen unter milden Bedingungen entfernt werden können, oder esterbildende Gruppen, welche leicht im lebenden Organismus abgespalten werden können. Beispiele für in vitro leicht spaltbare Schutzgruppen sind z.B. die Benzyl-, Diphenylmethyl-, Trityl-, t-Butyl-, die 2,2,2-Trichloräthyl- oder die Trimethylsilylgruppe.

Beispiele für in vivo leicht spaltbare Schutzgruppen sind z.B. Alkanoyloxyalkylgruppen, wie z.B. die Acetoxymethyl-, Propionyloxymethyl-, 2-Acetoxyäthyl- oder Pivaloyloxymethylgruppe oder die Phthalidyl- oder Indanylgruppe.

Bedeutet E ein Wasserstoffatom, so fallen unter den Anspruch auch pharmazeutisch verträgliche Salze mit anorganischen oder organischen Basen, wie z.B. die Alkali- oder Erdalkalisalze, beispielsweise die Natrium-, Kalium-, Magnesium- oder Calciumsalze, die Ammoniumsalze oder organische Aminsalze, z.B. mit Triäthylamin oder Dicyclohexylamin.

Steht D für Pyridinium oder Aminocarbonylpyridinium, dann haben die Verbindungen die allgemeine Formel Ia

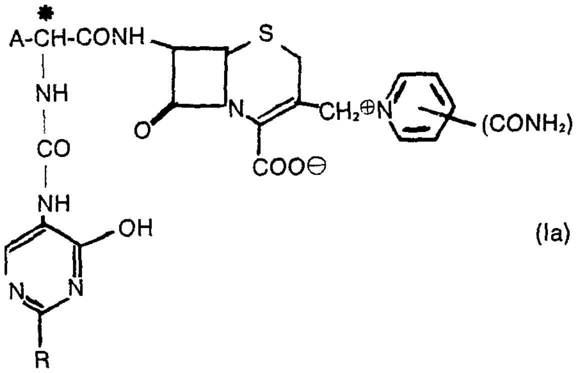

(Ia)

Bevorzugt sind diejenigen Verbindungen der allgemeinen Formel I, worin A für die Phenyl-, p-Hydroxyphenyl-, 3,4-Dihydroxyphenyl-, 2- oder 3-Thienyl- oder 2- oder 3-Furylgruppe, E für Wasserstoff, 2-Acetoxyäthyl- oder Pivaloyloxymethyl- steht,

D die oben angegebenen Bedeutungen besitzt,

sowie $(CH_2)_nR_1$ die folgenden Bedeutungen hat:

die 3-Pyridyl-, 6-subst.-3-Pyridyl-, 2-subst.-3--Pyridyl-, 2-, 3- oder 4-Pyridylmethyl-, 2- 4- oder 5-Pyrimidinylmethyl-, 2-subst.-5-Pyrimidinylmethyl-, 4-Hydroxy-5-pyrimidinyl-, 2-subst.-4-hydroxy-5-pyrimidinyl-, 5-Pyrimidinyl-, 2-subst.-5-Pyrimidinyl-, 4-subst.-2-Pyrimidinyl-, 4,6-disubst.-2-Pyrimidinyl-, 2-subst.-4-Pyrimidinyl-, 2,6-disubst.--4-Pyrimidinyl-, 5-subst.-2-Furyl-, 5-subst.-2-Thienyl-, 2- oder 3-Furylmethyl-, 2- oder 3-Thienylmethyl-, 5-subst.-2-Thienylmethyl-, eine 5-subst.--2-Furylmethylgruppe oder eine 4-subst.-2-Thiazolylgrupe, wobei die Substituenten in diesen Gruppen die oben angegebene Bedeutung Besitzen können,

weiter eine 2-Tetrahydrofurylmethylgruppe,

eine gegebenenfalls durch einen Methylrest substituierte 2-Imidazolyl-, 2-Imidazolylmethyl-, 2-Thiazolylmethyl-, 2-Oxazolyl-, 2-Oxazolylmethyl-, eine 1,2,4-Triazolyl- oder 1,2,4-Triazolylmethyl-, eine 5-Methyl-2-(1,3,4)-thiazolyl-, sowie eine Tetrazolylmethylgruppe.

Besonders bevorzugt sind diejenigen Verbindungen der allgemeinen Formel I, worin die Definition der Reste wie folgt lautet:

A steht für Phenyl-, p-Hydroxyphenyl-, 2-Thienyl- oder die 2- oder 3-Furylgruppe,

E steht für eine Wasserstoffatom oder die Pivaloyloxymethylgruppe

D steht für ein Wasserstoffatom, die Acetoxy- oder Aminocarbonyloxygruppe oder die Gruppe SHet, wobei Het die Tetrazol-5-yl-, die 1-Methyltetrazol-5-yl-, die 1,3,4-Thiadiazol-5-yl- oder die 2-Methyl-1,3,4-thiadiaol-5-yl-gruppe bedeutet,

und $(CH_2)_nR_1$ die folgenden Bedeutungen besitzt:

die 3-Pyridyl-, die 6-Methylsulfinyl-3-pyridylgruppe, die 6-Methylsulfonyl-3-pyridylgruppe, die 6-Hydroxy-3-pyridylgruppe, die 5-Pyrimidinylmethyl- oder die 2-Methyl-5-pyrimdinylmethylgruppe, die 2-Methyl- oder die 2-Hydroxy-5-pyrimidinylgruppe, die 6-Hydroxy-2-pyrimidinylgruppe, die 4,6-dihydroxy-2-pyrimidinylgruppe, die 3-Pyridylmethylgruppe, die 2-Furylmethylgruppe, die 2-Thienylmethylgruppe, die 5-Aminosulfonyl-2--thienylmethylgruppe, die 5-Aminocarbonyl- oder die 5-Äthoxycarbonyl-thienylgruppe.

Ganz besonders bevorzugte Bedeutungen für die Verbindungen der allgemeinen Formel I sind die, in denen

A die p-Hydroxyphenyl- oder 2-Thienylgruppe,

E Wasserstoff

D die Acetoxy- oder 1-Methyl-tetrazol-5-yl-gruppe

und $(CH_2)_nR_1$

die 3-Pyridylmethyl-, 6-Methylsulfinyl-3-pyridyl-, 6-Methylsulfonyl-3-pyridyl- oder 6-Hydroxy--3-pyridylgruppe, die 2-Methyl-5-pyrimidinylmethyl-, die 2-Hydroxy-5-pyrimidinyl-, die 4-Hydroxy-2-pyrimidinyl- oder die 4,6-Dihydroxy-2--pyrimidinylgruppe, die 5-Aminocarbonyl-thienyl-, 2-Thienylmethyl- oder 5-Aminosulfonyl-2--thienylmethyl- oder die 2-Furylmethylgruppe bedeutet.

Die β-Lactame der allgemeinen Formel I können in zwei tautomeren Formen (nämlich vom Lactim- und vom Lactamtyp) vorliegen. Es hängt besonders vom jeweiligen Lösemittel und von der Art des Substituenten R ab, welche der beiden Formen I oder I' überwiegt:

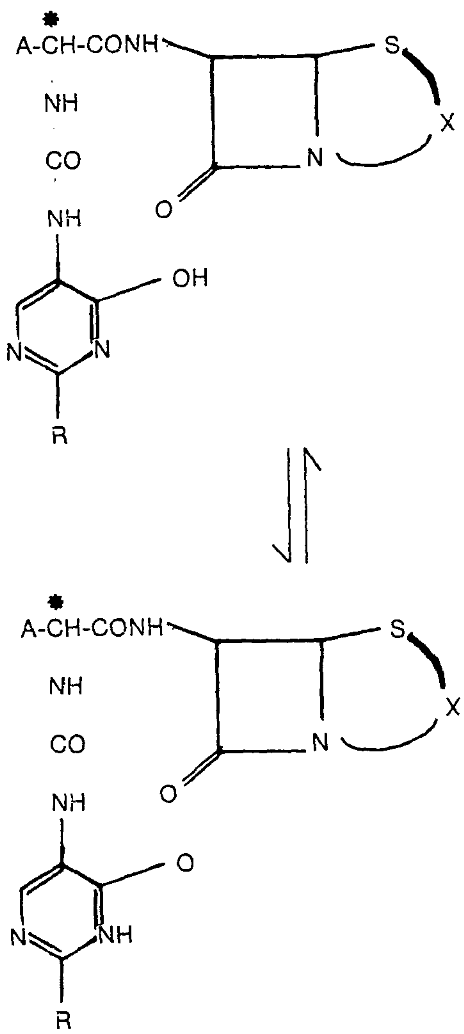

(I)

(I')

Es versteht sich von selbst, dass die eingangs angegebenen Verbindungen vom Typ I immer beide Tautomeren umfassen.

Die Verbindungen der allgemeinen Formel I können bezüglich des Chiralitätszentrums C+ in den beiden möglichen R- und S-Konfigurationen, jedoch auch als ein Gemisch dieser beiden Konfigurationen, vorliegen. Besonders bevorzugt sind solche Verbindungen, für welche die D=R-Konfiguration zutrifft. Wenn das Endprodukt in der D,L-Form anfällt, gelingt es, die reinen D- und L-Diastereomeren durch präparative Flüssigkeitschromatographie (HPLC) herzustellen.

Die Verbindungen der allgemeinen Formel I können wie folgt hergestellt werden:

1. Verbindungen der allgemeinen Formel I, in der D die angegebene Bedeutung mit Ausnahme der einer Pyridinium- oder Aminocarbonylpyridiniumgruppe besitzt, durch Umsetzung einer Verbindung der allgemeinen Formel

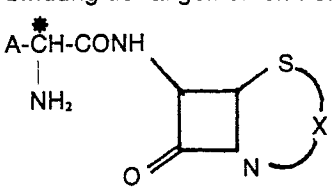

(II)

in der A und X die oben angegebene Bedeutung und D die oben angegebene Bedeutung mit Ausnahme der einer Pyridinium- oder Aminocarbonylpyridiniumgruppe besitzen, mit einem Pyrimidinderivat der allgemeinen Formel

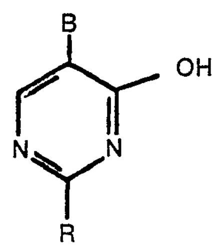

(III)

in der R wie oben definiert ist und B die Gruppe =NCO oder ein reaktives Derivat der Gruppe -NHCOOH bedeutet, wie z.B. die Gruppen

-NHCOCl, -NHCOBr oder -NH-COO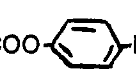-$NO_2$,

wobei die Gruppen =NCO und -NHCOCl besonders bevorzugt sind. Es können auch Gemische von solchen Pyrimidinderivaten der allgemeinen Formel III verwendet werden, in der B teils die eine und teils die andere der vorstehend genannten Bedeutungen besitzt, z.B. die Gruppen -NCO und -N(H)-COCl gleichzeitig nebeneinander.

Bedeutet E ein Wasserstoffatom, so können die Ausgangsprodukte der allgemeinen Formel II in Form ihrer anorganischen oder organischen Salze, z.B. als Triäthylammoniumsalz oder Natriumsalze, eingesetzt werden. Die Reaktion kann dann in beliebigen Mischungen von Wasser mit solchen organischen Lösungsmitteln, die mit Wasser mischbar sind, wie Ketonen, z.B. Aceton, cyclische Äther, z.B. Tetrahydrofuran oder Dioxan, Nitrilen, z.B. Acetonitril, Formamiden, z.B. Dimethylformamid, Diemethylsulfoxid oder Alkoholen z.B. Isopropanol oder in Hexametapol durchgeführt werden. Dabei hält man den pH der Reaktionsmischung durch Zusatz von Basen oder Verwendung von Pufferlösungen in einem pH-Bereich von etwa 2,0 bis 9,0, vorzugsweise zwischen pH 6,5 und 8,0. Es ist aber auch möglich, die Reaktion in wasserfreien organischen Lösungsmitteln, z.B. halogenierten Kohlenwasserstoffen, wie Chloroform oder Methylenchlorid unter Zusatz von Basen, vorzugsweise Triäthylamin, Diäthylamin oder N-Äthylpiperidin durchzuführen. Weiterhin lässt sich die Reaktion in einem Gemenge aus Wasser und einem mit Wasser nicht mischbaren Lösungsmitte,l wie Äther, z.B. Diäthyläther, halogenierten Kohlenwasserstoffen, z.B. Chloroform od. Methylenchlorid, Schwefelkohlenstoff, Ketonen, z.B. Isobutylmethylketon, Estern, z.B. Essigsäureäthylester, aromatischen Lösungsmitteln, z.B. Benzol ausführen, wobei es zweckmässig ist, kräftig zu rühren, und den pH-Wert durch Basenzusatz oder Verwendung von Pufferlösungen in einem Bereich von etwa pH 2,0 bis 9,0, vorzugsweise zwischen 6,5 und 8,0, zu halten. Man kann die Reaktion aber auch in Wasser allein in Gegenwart einer organischen oder anorganischen Base oder unter Zusatz von Pufferstoffen durchführen.

Bedeutet E die Trimethylsilylgruppe, d.h. verwendet man als Ausgangsprodukte für das erfindungsgemässe Verfahren die Silylderivate der Verbindungen der allgemeinen Formel II (z.B. Mono- oder zweckmässigerweise die an der Amino- und Carboxylgruppe silyierten Di-trimethylsilylderivate) und setzt sie mit Verbindungen der allgemeinen Formel III um, so arbeitet man im allgemeinen zweckmässigerweise in wasser- und hydroxyl-gruppenfreien Lösungsmitteln, beispielsweise in halogenierten Kohlenwasserstoffen, z.B. Methylenchlorid oder Chloroform, Benzol, Tetrahydrofuran, Aceton oder Dimethylformamid usw. Der Zusatz von Basen ist hierbei nicht notwendig, kann jedoch in einzelnen Fällen vorteilhaft sein, um die Ausbeute und Reinheit der Produkte zu verbessern. Als gegebenenfalls zugesetzte Basen werden zweckmässigerweise tertiäre aliphatische oder aromatische Amine, wie Pyridin oder Triäthylamin, oder durch sterische Hinderung schweracylierbare sekundäre Amine, wie Dicyclohexylamin, benützt. Bedeutet E eine der anderen obengenannten in vitro oder in vivo leicht spaltbaren Schutzgruppen, z.B. die Diphenylmethylestergruppe oder die Pivaloyloxymethylgruppe, so empfiehlt es sich ebenfalls in einem aprotischen Lösungsmittel zu arbeiten, z.B. in absolutem Methylenchlorid, Chloroform, Tetrahydrofuran oder Dimethylformamid.

Die Menge der verwendeten Basen ist z.B. durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich oder nicht sinnvoll ist, werden im Falle der Verwendung der nichtsilylierten Verbindungen der allgemeinen Formel II vorzugsweise 1,0 bis 2,0 Moläquivalente Basen eingesetzt. Im Falle der Verwendung der silylierten Verbindungen verwendet man vorzugsweise bis zu einem Moläquivalent Base.

Als Basen können prinzipiell alle in der organischen Chemie üblicherweise verwendeten organischen und anorganischen Basen verwendet werden, wie Alkali- und Erdalkalihydroxide, Erdalkalioxide, Alkali- und Erdalkalicarbonate und Hydrogencarbonate, Ammoniak, primäre, sekundäre und tertiäre aliphatische und aromatische Amine sowie heterocyclische Basen. Beispielhaft seien Natrium-, Kalium- und Calziumhydroxid, Cayziumoxid, Natrium- und Kaliumcarbonat, Natrium- und Kaliumhydrogencarbonat, Diäthylamin, Methyl-äthylamin, Triäthylamin, Hydroxyäthylamin, Anilin, Dimethylanilin, Pyridin und Piperidin genannt. Bei Verwendung der silylierten Ausgangsstoffe sollten jedoch die oben angegebenen Einschränkungen bezüglich der Art der Basen beachtet werden.

Als Puffersysteme können alle üblichen Puffermischungen verwendet werden, z.B. Phosphatpuffer, Citratpuffer und Tris(hydroxymethyl)-amino-methan-Puffer.

Die Reaktionstemperaturen können in einem grösseren Bereich variiert werden. Im allgemeinen arbeitet man zwischen −20 und +50°C, vorzugsweise zwischen 0 und +20°C.

Die Reaktionspartner der allgemeinen Formeln II und III können von vornherein in äquimolaren Mengen miteinander zur Reaktion gebracht werden. Es kann aber in einzelnen Fällen durchaus zweckmässig sein, einen der beiden Reaktionspartner im Überschuss zu verwenden, um sich damit die Reinigung des Endproduktes zu erleichtern oder um die Ausbeute zu steigern.

2) Verbindungen der allgemeinen Formel I, in der D die oben angegebenen Bedeutungen mit Ausnahme der einer Pyridinium- oder Aminocarbonylpyridiniumgruppe besitzt, durch Umsetzung von Ureidocarbonsäuren der allgemeinen Formel IV,

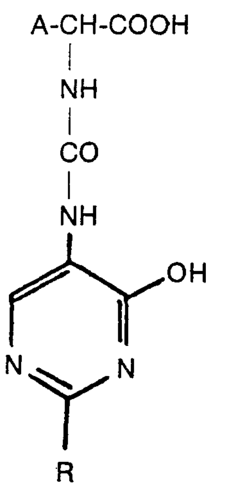

(IV)

in der A und R di oben angegebenen Bedeutungen besitzen, ihren Salzen oder reaktiven Derivaten, mit Verbindungen der allgemeinen Formel V,

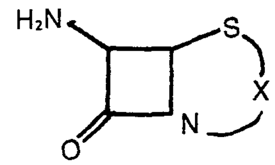

(V)

in der X die oben angegebenen Bedeutungen, mit Ausnahme der Formel, in der D für Pyridinium oder Aminocarbonylpyridinium steht, hat.

Als reaktive Derivate der Ureidocarbonsäuren der allgemeinen Formel IV kommen beispielsweise deren Säureanhydride wie z.B. die, die sich von Chlorameisensäureestern, z. B. Chlorameisensäureäthyl- oder isobutylester, ableiten, oder deren reaktive Ester, wie der p-Nitrophenylester oder der N-Hydroxysuccinimidester, oder deren reaktive Amide, wie das N-Carbonylimidazol, aber auch deren Säurehalogenide, wie das entsprechende Säurechlorid oder deren Säureazide in Frage.

Prinzipiell können jedoch alle Verknüpfungsmethoden, wie sie aus der β-Lactamchemie bekannt sind, verwendet werden.

Die 7-Aminocephalosporansäure oder Penicillansäurederivate der allgemeinen Formel V werden vorteilhafterweise in Form eines in vitro oder in vivo leicht spaltbaren Derivates eingesetzt. Hierfür kommen z.B. die Verbindungen der allgemeinen Formel V in Frage, bei denen E die eingangs gegebenen Bedeutungen mit Ausnahme der eines Wasserstoffatoms besitzt; besonders bevorzugte Derivate sind der Diphenylmethylester, der t-Butylester, der Trimethylsilylester oder das N,O-Bistrimethylsilylderivat.

Beispielsweise werden die Ureidocarbonsäuren, ihre Salze oder ihre reaktiven Derivate mit den 7-Aminocephalosporan- oder 6-Aminopenicillansäurederivaten in einem Lösungsmittel bei Temperaturen zwischen −40°C und +40°C, gegebenenfalls in Gegenwart einer Base, umgesetzt. Wird z. B. ein Anhydrid der Ureidocarbonsäure, beispielsweise das Anhydrid mit dem Äthylchloroformiat, eingesetzt, so wird die Reaktion unter Kühlung, beispielsweise bei −10°C bis +10°C in Gegenwart eines tertiären Amins, wie Triäthylamin oder N,N-Dimethylanilin, in einem Lösungsmittel, wie Aceton, Tetrahydrofuran, Dimethylformamid, Chloroform, Dichlormethan, Hexametapol oder in einem Gemisch dieser Lösungsmittel, durchgeführt. Setzt man beispielsweise einen N-Hydroxysuccinimidester der Ureidocarbonsäure mit Derivaten der allgemeinen Formel V um, so wird die Reaktion vorzugsweise bei 0 bis 20°C in Anwesenheit einer Base, wie z. B. Triäthylamin, in einem Lösungsmittel wie Dimethylformamid, Dichlormethan, Dioxan oder in einem Gemisch solcher Lösungsmittel durchgeführt.

Die Umsetzung einer Ureidocarbonsäure der allgemeinen Formel IV selbst oder ihrer Salze mit Verbindungen der allgemeinen Formel V erfolgt vorteilhafterweise in Gegenwart eines Kondensationsmittels, z.B. in Gegenwart von N,N'-Dicyclohexylcarbodiimid.

3) Cephalosporinderivate der allgemeinen Formel I, in der D die Bedeutungen einer -S-Het-, Pyridinium- oder 4-Aminocarbonylpyridiniumgruppe besitzt, durch Umsetzung einer Verbindung der allgemeinen Formel VI,

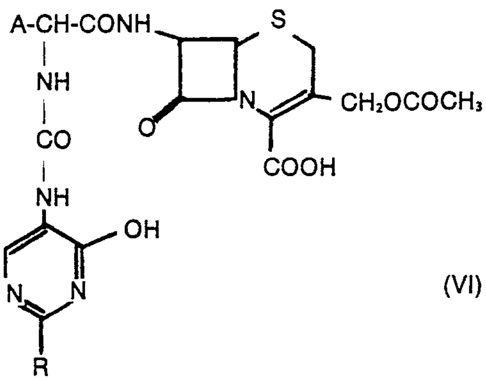

(VI)

in der A und R die oben angegebenen Bedeutungen haben, entweder mit einer Verbindung der allgemeinen Formel VII,

Het-S-M (VII)

in der Het die oben angegebenen Bedeutungen hat und M für ein Wasserstoffatom oder ein Alkalimetall oder ein Erdalkalimetall steht oder mit Pyridin bzw. 4-Aminocarbonylpyridin. Dazu wird z. B. eine Verbindung der Formel VI mit beispielsweise 5-Methyl-2-mercapto-1,3,4-thiadiazol in einem Lösungsmittel, wie z. B. Wasser, Methanol, Äthanol, Aceton, Methyläthylketon, Tetrahydrofuran, Acetonitril, Essigsäureäthylester, Dimethoxyäthan, Dimethylformamid, Dimethylsulfoxid, Chloroform oder einem Gemisch dieser Lösungsmittel umgesetzt. Vorzugsweise wird ein stark polares Lösungsmittel, wie Wasser, Acetonitril oder dergleichen, verwendet: Im Falle von Wasser als Lösungsmittel wird der pH-Wert der Reaktionslösung vorteilhafterweise auf 2-10 und insbesondere auf 4-8 gehalten. Der gewünschte pH-Wert kann durch Zugabe einer Pufferlösung, wie Natriumphosphat, eingestellt werden. Die Reaktionsbedingungen unterliegen keinen besonderen Beschränkungen. Normalerweise wird die Umsetzung bei einer Temperatur im Bereich von 0° bis 100°C während einer Zeitdauer von einigen Stunden durchgeführt.

Die nach den Verfahren 1-3 hergestellten erfindungsgemässen Verbindungen, in denen E für eine in vitro leicht abspaltbare Schutzgruppe steht, können nach bekannten Methoden der Cephalosporin- oder Penicillinchemie in die freien Carbonsäuren (E = Wasserstoff) der allgemeinen Formel I überführt werden. So kann beispielsweise die Trimethylsilylgruppe leicht durch wässrige Hydrolyse entfernt werden oder die Diphenylmethylestergruppe z. B. durch hydrolytische Spaltung mit Trifluoressigsäure. Diese Eliminierung der Schutzgruppen sind allgemein bekannt.

Ebenso können die Antibiotika der Formel I, worin E ein Wasserstoffatom bedeutet, in Acyloxyalkylester, worin E z. B. einen Pivaloyloxymethylrest

$-CH_2-OC-C(CH_3)_3$
$\|$
O

bedeutet, überführt werden, indem man ein Alkalisalz der freien Carbonsäure, beispielsweise ein Natrium- oder Kaliumsalz, mit einem Pivaloyloxymethylhalogenid der Formel

$Hal-CH_2-O-C-C(CH_3)_3$
$\|$
O

worin Hal für Chlor, Brom oder Jod steht, umsetzt. Weitere geeignete Acyloxyalkylhalogenide sind z. B. Chlormethylacetat, Brommethylpropionat oder 1-Bromäthylacetat.

Die Herstellung der Acyloxyalkylester der Formel I wird vorgenommen, indem man das jeweilige Alkalisalz der Stammsäure in einem inerten Lösungsmittel mit einem leichten molaren Überschuss des Jod-, Brom- oder Chloralkylesters, wie Pivaloyloxymethyljodid, bei Raumtemperatur oder leicht erhöhter Temperatur bis zu etwa 40 bis 45°C umsetzt. Als Lösungsmittel können beispielsweise Aceton, Tetrahydrofuran, Dioxan, Dimethylformamid oder Methylenchlorid verwendet werden.

Die Aufarbeitung der nach den genannten Verfahren erhaltenen Reaktionsgemische nach erfolgter Umsetzung wird nach den bei β-Lactam-Antibiotica gebräuchlichen Methoden vorgenommen; dasselbe gilt für die Isolierung und Reinigung der Endprodukte, beispielsweise für die Freisetzung der Säure oder ihre Überführung in andere Salze mittels anorganischer oder organischer Basen. Für die Herstellung der Kalium- oder Natriumsalze bewährt sich besonders die Fällung dieser Salze aus einer alkoholisch-ätherischen Lösung der freien Säure durch die Zugabe von Kalium- oder Natrium-2--äthylhexanoat oder die Umsetzung der freien Säure mit der entsprechenden Menge Natriumbicarbonat unter pH-Kontrolle und Kühlung sowie anschliessender Gefriertrocknung.

Die Ausgangsstoffe der Formel II (z. B. Ampicillin, Amoxycillin, Epicillin, Cefaloglycin, Cefalexin sowie ihre in vivo leicht spaltbaren Ester) sind bekannt oder können in Analogie zu bekannten Stoffen nach an sich bekannten Methoden hergestellt werden, z. B. durch Acylierung der bekannten Aminolactame der Formel IV und, falls erwünscht, anschliessende Umsetzung so erhaltener Cephalosporansäurederivate der Formel II (D = $-OCOCH_3$) mit Thiolen der Formel Het-SH.

Die Ausgangsstoffe der allgemeinen Formel III können beispielsweise durch Umsetzung der entsprechenden 5-Aminopyrimidine der allgemeinen Formel VIII,

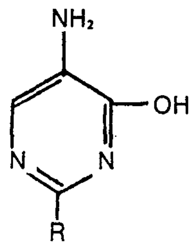

(VIII)

in der R wie oben definiert ist, mit Phosgen gewonnen werden. Diese Umsetzung erfolgt vorzugsweise in einem nicht Hydroxylgruppen-enthaltenden Lösungsmittel, wie Tetrahydrofuran, Methylenchlorid, Chloroform, Dimethoxyäthan oder Hexametapol bei Temperaturen zwischen −40° und +60°C, vorzugsweise zwischen −10° und +20°C. Dabei empfiehlt es sich, den entstehenden Chlorwasserstoff durch äquimolare Mengen einer inerten organischen Base, wie Triäthylamin oder Pyridin, zu binden. Als Lösungsmittel kann auch Pyridin im Überschuss verwendet werden. Sollten die betreffenden Aminopyrimidine der allgemeinen Formel VIII in einem der erwähnten Lösungsmittel schwer löslich sein, kann die Phosgenierung auch in heterogener Phase durchgeführt werden. Desweiteren können die Aminopyrimidine der allgemeinen Formel VIII durch Behandlung mit einem Silylierungsmittel, wie Hexamethyldisilazan oder Trimethylchlorsilan/Triäthylamin, Trimethylsilyldiäthylamin oder N,O-Bistrimethylsilylacetamid in ein im allgemeinen in den erwähnten Lösungsmitteln sehr leicht lösliches, einfach oder, entsprechend den vorhandenen austauschbaren Wasserstoffatomen, mehrfach silyliertes Aminopyrimidin überführt werden, das mit Phosgen dann zu den entsprechenden Verbindungen der allgemeinen Formel III reagiert. Je nach der Art des Lösungsmittels, der Höhe der Temperatur, der Menge und Art der eingesetzten Base entsteht entweder überwiegend das entsprechende Iesocyanat oder Carbaminsäurehalogenids oder ein Gemisch dieser beiden Verbindungen. Je nach den Reaktionsbedingungen kann die Verbindung der allgemeinen Formel III auch teilweise oder ganz als ein, den Isocyanaten isomeres Dihydrooxazolo-pyrimidin der allgemeinen Formel IIIa,

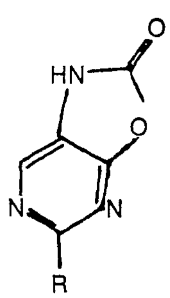

(IIIa)

oder bei vorhergehender Silylierung je nach Art des Substituenten R als ein- oder mehrfach silyliertes Analoges vorliegen.

Die durch Phosgenierung entstandenen Ausgangsprodukte der allgemeinen Formel III bzw. IIIa oder deren Gemische sind in den obenerwähnten Lösungsmitteln im allgemeinen gut löslich und können, nach Entfernung des überschüssigen Phosgens, ohne weitere Reinigung mit den entsprechenden β-Lactam-derivaten der allgemeinen Formel II direkt umgesetzt werden.

Es ist jedoch auch möglich, das Zwischenprodukt der allgemeinen Formel IIIa zu isolieren, es gegebenenfalls mit einem protischen Lösemittel, beispielsweise Wasser oder Methanol zu entsilylieren, es auf Grund seiner Löslichkeitseigenschaften zu reinigen und in der oben dargelegten Weise umzusetzen.

Die Ureidocarbonsäuren der allgemeinen Formel IV lassen sich leicht durch Umsetzung der Pyrimidinderivate der allgemeinen Formel III mit Glycerinderivaten der allgemeinen Formel IX,

$$A\text{-}\overset{+}{C}H\text{-}COOH \quad (IX)$$
$$\quad |$$
$$NH_2$$

in der A wie oben definiert ist, erhalten. Die Umsetzung erfolgt bei Temperaturen zwischen −20° und +40°C, vorzugsweise zwischen 0° und +20°C, in einem Lösungsmittel. Als Lösungsmittel können hierbei z. B. Gemische von Wasser und organischen Lösungsmitteln, die mit Wasser mischbar sind, verwendet werden, beispielsweise Aceton, Tetrahydrofuran, Dioxan, Acetonitril, Dimethylformamid, Äthanol, Dimethylsulfoxid. Gegebenenfalls wird die Verwendung eines halogenwasserstoffbindenden Mittels notwendig, als solche eignen sich beispielsweise Trialkylamine, wie Triäthylamin oder anorganische Basen, wie verdünnte Natronlauge.

Die Ausgangsverbindungen der allgemeinen Formel VI lassen sich leicht nach Verfahren 1 herstellen.

Die 2-substituierten 5-Amino-4-hydroxy-pyrimidine der allgemeinen Formel VIII lassen sich z. B. durch Umsetzung von 2-Äthylmercapto-4-hydroxy-5-nitro-pyrimidin (Vorbrüggen und Strehlke, Chem. Ber. 106, S. 3039 [1973]) mit den Aminen $NH_2(CH_2)_nR_1$ und anschliessende Reduktion der Nitrogruppe nach bekannten Methoden gewinnen. Statt der 5-Nitro-verbindung kann auch 2-Methylmercapto-4-hydroxy-5-benzoylamino-pyrimidin umgesetzt und anschliessend entbenzoyliert werden; des weiteren können die Aminopyrimidine der allgemeinen Formel VIII durch Umsetzung von 2-Chlor-4-hydroxy-5-nitropyrimidin mit den Aminen $R_1(CH_2)_nNH_2$ in wässriger Lösung und anschliessender Reduktion der Nitrogruppe erhalten werden. Zur Charakterisierung der so gewonnenen Ausgangsprodukte sollen hier typische Vertreter genannt werden:

5-Amino-4-hydroxy-2-(3'-pyridylamino)-pyrimidin
5-Amino-4-hydroxy-2-(6'-methoxy-3'-pyridylamino)-pyrimidin
5-Amino-4-hydroxy-2-(2'-pyridylmethylamino)-pyrimidin

5-Amino-4-hydroxy-2-(3'-pyridylmethylamino)--pyrimidin
5-Amino-4-hydroxy-2-(4'-pyridylmethylamino)--pyrimidin
5-Amino-4-hydroxy-2-(5'-pyrimidinylamino)-pyrimidin
5-Amino-4-hydroxy-2-(2'-amino-5'-pyrimidinylamino)-pyrimidin
5-Amino-4-hydroxy-2-(2'-methyl-5'-pyrimidinylmethylamino)-pyrimidin
5-Amino-4-hydroxy-2-(2'-methyl-5'-pyrimidinylamino)-pyrimidin
5-Amino-4-hydroxy-2-(6'-methylsulfinyl-3'-pyridylamino)-pyrimidin
5-Amino-4-hydroxy-2-(6'-methylsulfonyl-3'-pyridylamino)-pyrimidin
5-Amino-4-hydroxy-2-(6'-hydroxy-3'-pyridylamino)-pyrimidin
5-Amino-4-hydroxy-2-(4'-hydroxy-2'-pyrimidinylamino)-pyrimidin
5-Amino-4-hydroxy-2-(4',6'-dihydroxy-2'-pyrimidinylamino)-pyrimidin
5-Amino-4-hydroxy-2-(2',6'-dihydroxy-4'-pyrimidinylamino)-pyrimidin
5-Amino-4-hydroxy-2-(2'-hydroxy-4'-pyrimidinylamino)-pyrimidin
5-Amino-4-hydroxy-2-(2'-hydroxy-5'-pyrimidinylamino)-pyrimidin
5-Amino-4-hydroxy-2-(5'-äthoxycarbonyl-2'-thienylamino)-pyrimidin
5-Amino-4-hydroxy-2-(5'-aminocarbonyl-2'-thienylamino)-pyrimidin
5-Amino-4-hydroxy-2-(5'-methyl-2'-thienylamino)--pyrimidin
5-Amino-4-hydroxy-2-(2'-thienylmethylamino)-pyrimidin
5-Amino-4-hydroxy-2-(3'-thienylmethylamino)-pyrimidin
5-Amino-4-hydroxy-2-(5'-methyl-2'-thienylmethylamino)-pyrimidin
5-Amino-4-hydroxy-2-(5'-chlor-2'-thienylmethylamino)-pyrimidin
5-Amino-4-hydroxy-2-(2'-furylmethylamino)-pyrimidin
5-Amino-4-hydroxy-2-(3'-furylmethylamino)-pyrimidin
5-Amino-4-hydroxy-2-(5'-methyl-2'-furylmethylamino)-pyrimidin
5-Amino-4-hydroxy-2-(2'-tetrahydrofurylmethylamino)-pyrimidin
5-Amino-4-hydroxy-2-(2'-pyrrolylmethylamino)--pyrimidin
5-Amino-4-hydroxy-2-(4'-imidazolylmethylamino)-pyrimidin
5-Amino-4-hydroxy-2-(4'-methyl-2'-thiazolylamino)-pyrimidin
5-Amino-4-hydroxy-2-(4'-methyl-2'-thiazolylmethylamino)-pyrimidin
5-Amino-4-hydroxy-2-(5'-methyl-2'-thiadiazolylamino)-pyrimidin
5-Amino-4-hydroxy-2-(2'-triazolylmethylamino)--pyrimidin
5-Amino-4-hydroxy-2-(5'-aminosulfonyl-2'-thienylmethylamino)-pyrimidin
5-Amino-4-hydroxy-2-(5'-tetrazolylmethylamino)--pyrimidin

Es wurde gefunden, dass die Verbindungen der allgemeinen Formel I wertvolle pharmakologische Eigenschaften bei guter Verträglichkeit besitzen. Die erfindungsgemässen Wirkstoffe können daher zur Prophylaxe und Chemotherapie von lokalen und systemischen Infektionen in der Human- und Tiermedizin verwendet werden. Als Krankheiten, die durch die erfindungsgemässen Verbindungen verhindert bzw. geheilt werden können, seien beispielsweise solche der Atmungswege, des Rachenraumes und der Harnwege genannt; die Verbindungen wirken insbesondere gegen Pharyngitis, Pneumonie, Peritonitis, Pyelonephritis, Otitis, Cystitis, Endocarditis, Bronchitis, Arthritis und allgemeine systemische Infektionen. Weiter können diese Verbindungen als Stoffe zur Konservierung von anorganischen oder organischen Materialien, wie Polymeren, Schmiermittel, Farben, Fasern, Leder, Papier und Holz sowie von Lebensmitteln verwendet werden.

Dies wird dadurch ermöglicht, dass die Verbindungen der allgemeinen Formel I sowohl in vitro als auch in vivo gegen schädliche Mikroorganismen, insbesondere gegen grampositive und gramnegative Bakterien und bakterienähnliche Mikroorganismen sehr stark wirken, wobei sie sich besonders durch ein breites Wirkungsspektrum auszeichnen.

Mit diesen β-Lactamen können beispielsweise lokale und/oder systemische Erkrankungen behandelt und/oder verhindert werden, die durch die folgenden Erreger oder durch Mischungen der folgenden Erreger verursacht werden:

Micrococcaceae, wie Staphylokokken;
Lactobacteriaceae, wie Streptokokken;
Neisseriaceae, wie Neisserien;
Corynebacteriaceae, wie Corynebakterien;
Enterobacteriaceae, wie Escherichiae-Bakterien der Coli-Gruppe, Klebsiella-Bakterien, z. B. K. pneumoniae;
Proteae-Bakterien der Proteus-Gruppe z. B. Proteus vulgaris;
Salmonella-Bakterien, z. B. S.thyphimurium;
Shigella-Bakterien, z. B. Shigella dysenteriae;
Pseudomonas-Bakterien, z. B. Pseudomonas aeruginosa;
Aeromonas-Bakterien, z. B. Aeromonas lique faciens;
Spirillaceae, wie Vibrio-Bakterien, z. B. Vibrio cholerae;
Parvobateriaceae oder Brucellaceae, wie Pasteurella-Bakterien;
Brucella-Bakterien, z. B. Brucella abortus;
Haemophilus-Bakterien, z. B. Haemophilus influenzae;
Bordetella-Bakterien, z. B. Bordetella pertussis;
Moraxella-Bakterien, z. B. Moraxella lacunata;
Bacteroidaceae, wie Bacteroides-Bakterien;
Fusiforme-Bakterien, z. B. Fusobacterium fusiforme;

Sphaerophorus-Bakterien, z. B. Sphaerophorus necrophorus;
Bacillaceae, wie aerobe Sporenbildner, z. B. Bacillus anthracis;
anaerobe Sporenbildner-Chlostridien, z. B. Chlostridium perfringens;
Spirochaetaceae, wie Borrelia-Bakterien;
Treponema-Bakterien, z. B. Treponema pallidum;
Leptospira-Bakterien, wie Leptospira interrogans.

Die obige Aufzählung von Erregern ist lediglich beispielhaft und keineswegs beschränkend aufzufassen.

In den folgenden Tabellen 1 und 2 werden typische, besonders gut wirksame Penicilline und Cephalosporine gemäss vorliegender Erfindung aufgezählt. Die genannten Penicilline können nach dem Verfahren 1 oder 2, die Cephalosporine nach den Verfahren 1, 2 oder 3 gewonnen werden.

TABELLE 1: Penicilline

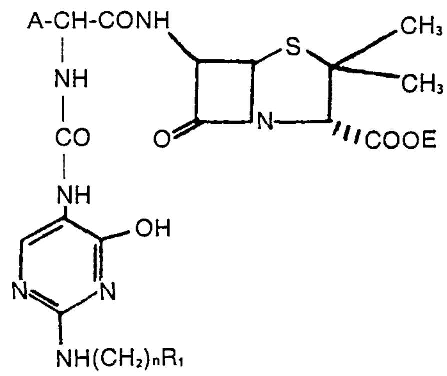

| | A | $(CH_2)_nR_1$ | E |
|---|---|---|---|
| 1 | p-HO-Phenyl | 2-Pyridyl | H |
| 2 | Phenyl | 3-Pyridyl | H |
| 3 | p-HO-Phenyl | 3-Pyridyl | H |
| 4 | p-HO-Phenyl | 6-Hydroxy-3-pyridyl- | H |
| 5 | p-HO-Phenyl | 2-Pyridylmethyl- | H |
| 6 | Phenyl | 3-Pyridylmethyl- | H |
| 7 | p-HO-Phenyl | 3-Pyridylmethyl- | H |
| 8 | p-HO-Phenyl | 4-Pyridylmethyl- | H |
| 9 | p-HO-Phenyl | 3-Pyridylmethyl- | $-CH_2OCOC(CH_3)_3$ |
| 10 | p-HO-Phenyl | 5-Pyrimidinyl- | H |
| 11 | p-HO-Phenyl | 2-Amino-5-pyrimidinyl- | H |
| 12 | p-HO-Phenyl | 2-Propyl-5-pyrimidinyl- | H |
| 13 | p-HO-Phenyl | 1,2,3,4-Tetrahydro-1,3-dimethyl-2,4-dioxo--5-pyrimidinyl | H |
| 14 | p-HO-Phenyl | 2-Cyclopropyl-4-hydroxy-5-pyrimidinyl- | H |
| 15 | p-HO-Phenyl | 4-Pyrimidinylmethyl- | H |
| 16 | p-HO-Phenyl | 2-Methyl-5-pyrimidinylmethyl- | H |
| 17 | p-HO-Phenyl | 5-Methyl-2-thienyl- | H |
| 18 | p-HO-Phenyl | 5-Äthoxycarbonyl-2-thienyl- | H |
| 19 | p-HO-Phenyl | 5-Aminocarbonyl-2-thienyl- | H |
| 20 | Phenyl | 2-Thienylmethyl- | H |
| 21 | p-HO-Phenyl | 2-Thienylmethyl- | H |
| 22 | p-HO-Phenyl | 2-Thienylmethyl- | $-CH_2OCOC(CH_3)_3$ |

TABELLE 1 (Fortsetzung)

| | A | $(CH_2)_nR_1$ | E |
|---|---|---|---|
| 23 | p-HO-Phenyl | 2-Thienylmethyl- | $-CH(CH_3)OCOC_2H_5$ |
| 24 | m,p-Di-OH-Phenyl | 2-Thienylmethyl- | H |
| 25 | 2-Thienyl | 2-Thienylmethyl- | H |
| 26 | 2-Furyl | 2-Thienylmethyl- | H |
| 27 | p-HO-Phenyl | 3-Thienylmethyl- | H |
| 28 | p-HO-Phenyl | 5-Methyl-2-thienylmethyl- | H |
| 29 | p-HO-Phenyl | 5-Chlor-2-thienylmethyl- | H |
| 30 | Phenyl | 2-Furylmethyl- | H |
| 31 | p-HO-Phenyl | 2-Furylmethyl- | H |
| 32 | p-HO-Phenyl | 2-Furylmethyl- | $-CH_2OCOC(CH_3)_3$ |
| 33 | p-HO-Phenyl | 2-Furylmethyl- | $-CH(CH_3)OCOC_2H_5$ |
| 34 | m,p-Di-HO-Phenyl | 2-Furylmethyl- | H |
| 35 | 2-Furyl | 2-Furylmethyl- | H |
| 36 | 3-Furyl | 2-Furylmethyl- | H |
| 37 | 2-Thienyl | 2-Furylmethyl- | H |
| 38 | 3-Thienyl | 2-Furylmethyl- | H |
| 39 | p-HO-Phenyl | 5-Methyl-2-furylmethyl- | H |
| 40 | p-HO-Phenyl | Tetrahydro-2-furylmethyl- | H |
| 41 | p-HO-Phenyl | 5-Aminosulfonyl-2-thienyl-methyl- | H |
| 42 | p-HO-Phenyl | 5-Aminosulfonyl-2-thienylmethyl- | $-CH_2OCOC(CH_3)_3$ |
| 43 | p-HO-Phenyl | 6-Methylsulfinyl-3-pyridyl | H |
| 44 | p-HO-Phenyl | 6-Methylsulfinyl-3-pyridyl | $CH_2OCOC(CH_3)_3$ |
| 45 | p-HO-Phenyl | 6-Methylsulfonyl-3-pyridyl | H |
| 46 | p-HO-Phenyl | 6-Methylsulfonyl-3-pyridyl | $CH_2OCOC(CH_3)_3$ |
| 47 | p-HO-Phenyl | 4-Methyl-2-thiazolyl- | H |
| 48 | p-HO-Phenyl | 4-Methyl-2-thiazolylmethyl- | H |
| 49 | p-HO-Phenyl | 5-Methyl-1,3,4-thiadiazol-2-yl | H |
| 50 | p-HO-Phenyl | 1,3,4-Triazol-2-ylmethyl- | H |
| 51 | p-HO-Phenyl | 5-Tetrazolylmethyl- | H |
| 52 | p-HO-Phenyl | 2-Hydroxy-5-pyrimidinyl | H |
| 53 | p-HO-Phenyl | 2,4-Dihydroxy-5-pyrimidinyl | H |
| 54 | p-HO-Phenyl | 4,6-Dihydroxy-5-pyrimidinyl | H |
| 55 | p-HO-Phenyl | 2,6-Dihydroxy-4-pyrimidinyl | H |

TABELLE 2: Cephalosporine

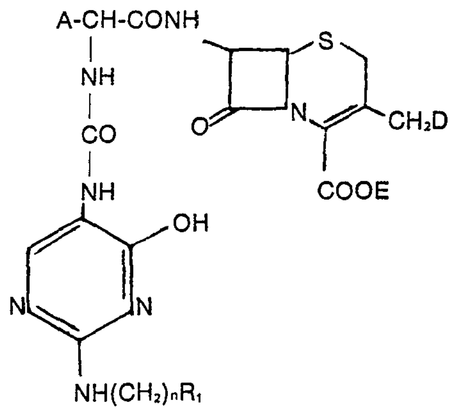

| | A | $(CH_2)_nR_1$ | D | E |
|---|---|---|---|---|
| 1 | p-HO-Phenyl | 3-Pyridyl | $OCOCH_3$ | H |
| 2 | p-HO-Phenyl | 3-Pyridyl | 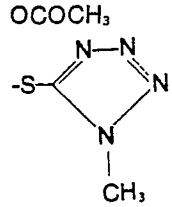 | H |
| 3 | p-HO-Phenyl | 6-Methylsulfinyl-3-pyridyl | 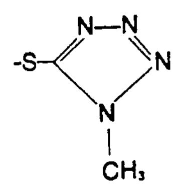 | H |
| 4 | p-HO-Phenyl | 6-Methylsulfinyl-3-pyridyl | $OCOCH_3$ | H |
| 5 | p-HO-Phenyl | 6-Methylsulfonyl-3-pyridyl | 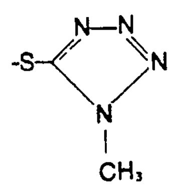 | H |
| 6 | p-HO-Phenyl | 6-Methylsulfonyl-3-pyridyl | $OCOCH_3$ | H |
| 7 | 2-Furyl | 6-Methylsulfinyl-3-pyridyl | 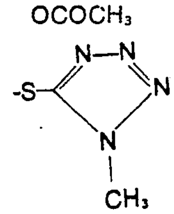 | H |
| 8 | 2-Thienyl | 6-Methylsulfinyl-3-pyridyl | 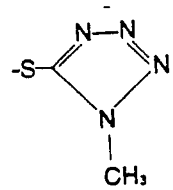 | H |
| 9 | p-HO-Phenyl | 6-Hydroxy-3-pyridyl | 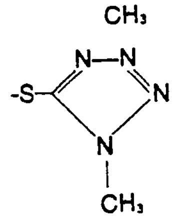 | H |

TABELLE 2 (Fortsetzung)

| | A | $(CH_2)_nR_1$ | D | E |
|---|---|---|---|---|
| 10 | Phenyl | 3-Pyridylmethyl | 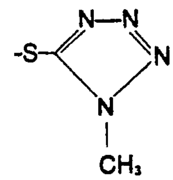 | H |
| 11 | p-HO-Phenyl | 3-Pyridylmethyl | H | H |
| 12 | p-HO-Phenyl | 3-Pyridylmethyl | $OCOCH_3$ | H |
| 13 | p-HO-Phenyl | 3-Pyridylmethyl | $OCONH_2$ | H |
| 14 | p-HO-Phenyl | 3-Pyridylmethyl | 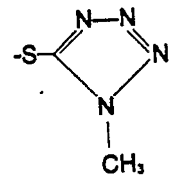 | H |
| 15 | p-HO-Phenyl | 3-Pyridylmethyl | 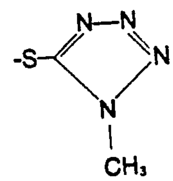 | $-CH_2OCOC(CH_3)_3$ |
| 16 | p-HO-Phenyl | 3-Pyridylmethyl | 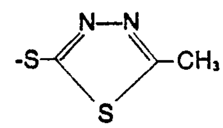 | H |
| 17 | 2-Furyl | 3-Pyridylmethyl | $OCOCH_3$ | H |
| 18 | 2-Furyl | 3-Pyridylmethyl | 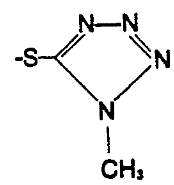 | H |
| 19 | 3-Furyl | 3-Pyridylmethyl | 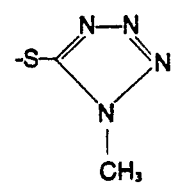 | H |
| 20 | 2-Thienyl | 3-Pyridylmethyl | 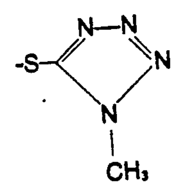 | H |
| 21 | p-HO-Phenyl | 2-Pyridylmethyl | 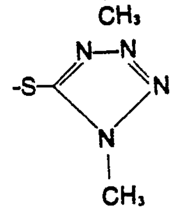 | H |

TABELLE 2 (Fortsetzung)

| | A | $(CH_2)_nR_1$ | D | E |
|---|---|---|---|---|
| 22 | p-HO-Phenyl | 4-Pyridylmethyl | 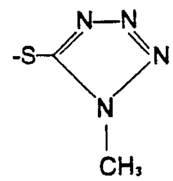 | H |
| 23 | p-HO-Phenyl | 5-Pyrimidinyl | 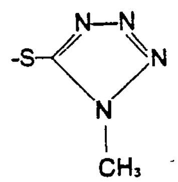 | H |
| 24 | p-HO-Phenyl | 2-Methyl-5-pyrimidinyl | H | H |
| 25 | p-HO-Phenyl | 2-Methyl-5-pyrimidinyl | $OCOCH_3$ | H |
| 26 | p-HO-Phenyl | 2-Methyl-5-pyrimidinyl | $OCONH_2$ | H |
| 27 | p-HO-Phenyl | 2-Methyl-5-pyrimidinyl | 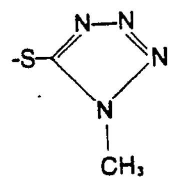 | H |
| 28 | p-HO-Phenyl | 2-Methyl-5-pyrimidinyl | 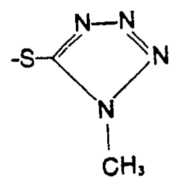 | $-CH_2OCOC(CH_3)_3$ |
| 29 | p-HO-Phenyl | 2-Methyl-5-pyrimidinyl | 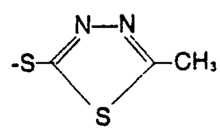 | H |
| 30 | 2-Furyl | 2-Methyl-5-pyrimidinyl | $-OCOCH_3$ | H |
| 31 | 2-Furyl | 2-Methyl-5-pyrimidinyl | 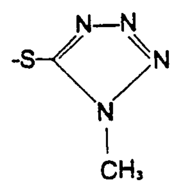 | H |
| 32 | 2-Thienyl | 2-Methyl-5-pyrimidinyl | 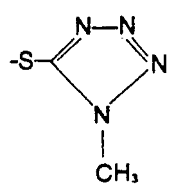 | H |
| 33 | p-HO-Phenyl | 5-Äthoxycarbonyl-2-thienyl- | $OCOCH_3$ | H |
| 34 | p-HO-Phenyl | 5-Äthoxycarbonyl-2-thienyl- | 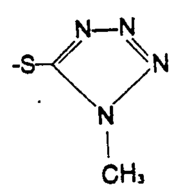 | H |

TABELLE 2 (Fortsetzung)

| | A | $(CH_2)_nR_1$ | D | E |
|---|---|---|---|---|
| 35 | p-HO-Phenyl | 5-Äthoxycarbonyl-2-furyl | 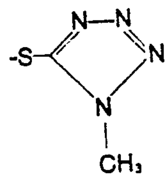 | H |
| 36 | p-HO-Phenyl | 5-Aminosulfonyl-2-thienylmethyl | 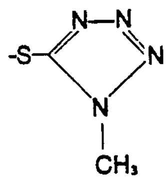 | H |
| 37 | p-HO-Phenyl | 5-Aminosulfonyl-2-thienylmethyl | 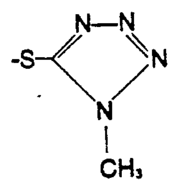 | $-CH_2OCOC(CH_3)_3$ |
| 38 | p-HO-Phenyl | 5-Aminosulfonyl-2-thienylmethyl | $OCOCH_3$ | H |
| 39 | p-HO-Phenyl | 5-Aminosulfonyl-2-thienylmethyl | $OCONH_2$ | H |
| 40 | p-HO-Phenyl | 5-Aminosulfonyl-2-thienylmethyl | 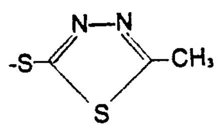 | H |
| 41 | p-HO-Phenyl | 2-Thienylmethyl- | H | H |
| 42 | p-HO-Phenyl | 2-Thienylmethyl- | $OCOCH_3$ | H |
| 43 | p-HO-Phenyl | 2-Thienylmethyl- | $OCONH_2$ | H |
| 44 | p-HO-Phenyl | 2-Thienylmethyl- | 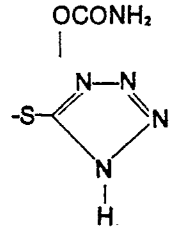 | H |
| 45 | p-HO-Phenyl | 2-Thienylmethyl- | 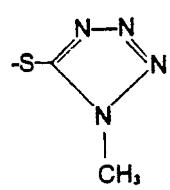 | H |
| 46 | p-HO-Phenyl | 2-Thienylmethyl- | 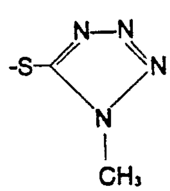 | $-CH_2OCOC(CH_3)_3$ |
| 47 | p-HO-Phenyl | 2-Thienylmethyl- | 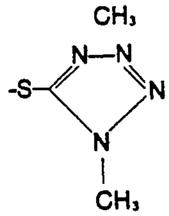 | $-CHOCOC_2H_5$<br>$CH_3$ |

TABELLE 2 (Fortsetzung)

| | A | $(CH_2)_nR_1$ | D | E |
|---|---|---|---|---|
| 48 | p-HO-Phenyl | 2-Thienylmethyl | 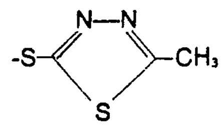 | H |
| 49 | p-HO-Phenyl | 2-Thienylmethyl | 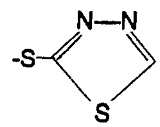 | H |
| 50 | p-HO-Phenyl | 2-Thienylmethyl | 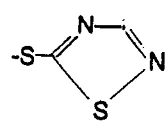 | H |
| 51 | p-HO-Phenyl | 2-Thienylmethyl | 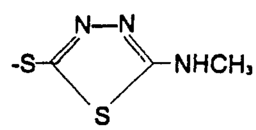 | H |
| 52 | p-HO-Phenyl | 2-Thienylmethyl | 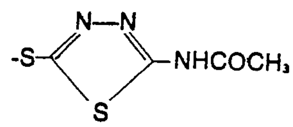 | H |
| 53 | p-HO-Phenyl | 2-Thienylmethyl | 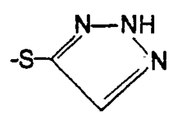 | |
| 54 | p-HO-Phenyl | 2-Thienylmethyl | 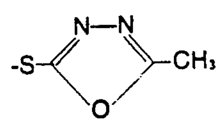 | H |
| 55 | m,p-Di-OH-Phenyl | 2-Thienylmethyl | 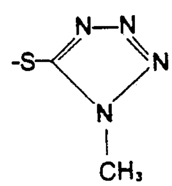 | H |
| 56 | 2-Furyl | 2-Thienylmethyl | $-OCOCH_3$ | H |
| 57 | 2-Furyl | 2-Thienylmethyl | 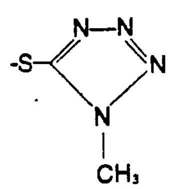 | H |
| 58 | 3-Furyl | 2-Thienylmethyl | 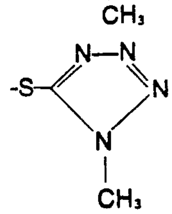 | H |
| 59 | 2-Thienyl | 5-Aminosulfonyl-2-thienylmethyl | 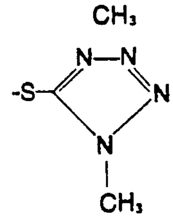 | H |

| | A | $(CH_2)_nR_1$ | D | E |
|---|---|---|---|---|
| 60 | 2-Thienyl | 2-Thienylmethyl | 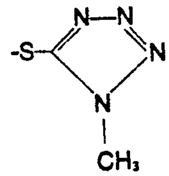 | H |
| 61 | p-HO-Phenyl | 3-Thienylmethyl | 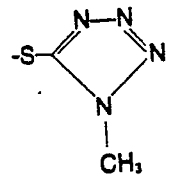 | H |
| 62 | Phenyl | 2-Furylmethyl | $-OCOCH_3$ | H |
| 63 | p-HO-Phenyl | 2-Furylmethyl | H | H |
| 64 | p-HO-Phenyl | 2-Furylmethyl | $-OCOCH_3$ | H |
| 65 | p-HO-Phenyl | 2-Furylmethyl | $-OCONH_2$ | H |
| 66 | p-HO-Phenyl | 2-Furylmethyl | 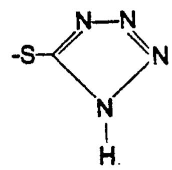 | H |
| 67 | p-HO-Phenyl | 2-Furylmethyl | 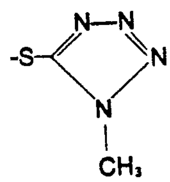 | H |
| 68 | p-HO-Phenyl | 2-Furylmethyl | 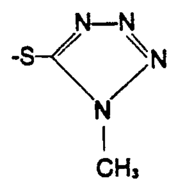 | $-CH_2OCOC(CH_3)_3$ |
| 69 | p-HO-Phenyl | 2-Furylmethyl | 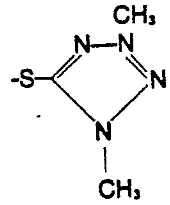 | $CHOCOC_2H_5$<br>\|<br>$CH_3$ |
| 70 | p-HO-Phenyl | 2-Furylmethyl | 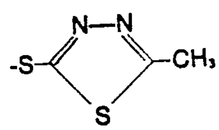 | H |
| 71 | p-HO-Phenyl | 2-Furylmethyl | 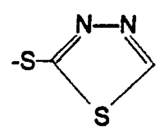 | H |
| 72 | p-HO-Phenyl | 2-Furylmethyl | 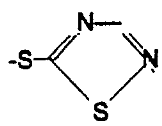 | H |
| 73 | p-HO-Phenyl | 2-Furylmethyl | 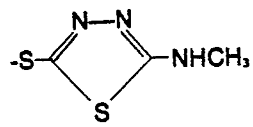 | H |

TABELLE 2 (Fortsetzung)

| | A | $(CH_2)_nR_1$ | D | E |
|---|---|---|---|---|
| 74 | p-HO-Phenyl | 2-Furylmethyl | 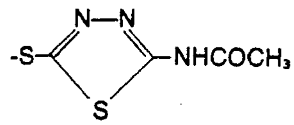 | H |
| 75 | p-HO-Phenyl | 2-Furylmethyl | 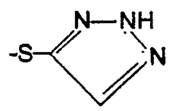 | H |
| 76 | p-HO-Phenyl | 2-Furylmethyl | 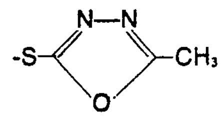 | H |
| 77 | p-HO-Phenyl | 2-Furylmethyl | 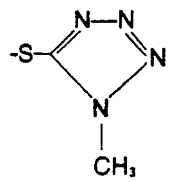 | H |
| 78 | p-HO-Phenyl | 2-Furylmethyl | 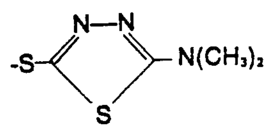 | H |
| 79 | m,p-Di-OH-Phenyl | 2-Furylmethyl | 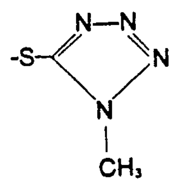 | H |
| 80 | 2-Furyl | 2-Furylmethyl | $-OCOCH_3$ | H |
| 81 | 2-Furyl | 2-Furylmethyl | 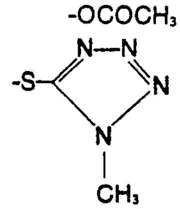 | H |
| 82 | 2-Thienyl | 2-Furylmethyl | $-OCOCH_3$ | H |
| 83 | 2-Thienyl | 2-Furylmethyl | 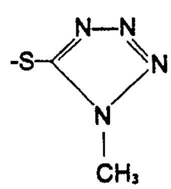 | H |
| 84 | 2-Thienyl | 2-Furylmethyl | 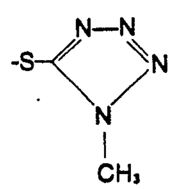 | $-CH_2OCOC(CH_3)_3$ |
| 85 | p-HO-Phenyl | 3-Furylmethyl | 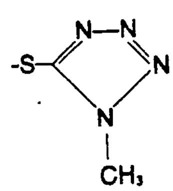 | H |

TABELLE 2 (Fortsetzung)

| | A | $(CH_2)_nR_1$ | D | E |
|---|---|---|---|---|
| 86 | p-HO-Phenyl | 3-Furylmethyl | $-OCOCH_3$ | H |
| 87 | p-HO-Phenyl | Tetrahydro-2-furylmethyl- | 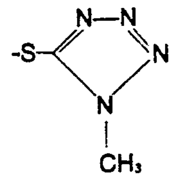 | H |
| 88 | p-HO-Phenyl | 6-Methylsulfinyl-3-pyridyl | $OCOCH_3$ | H |
| 89 | p-HO-Phenyl | 6-Methylsulfinyl-3-pyridyl | 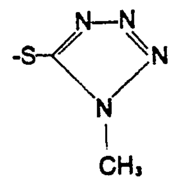 | H |
| 90 | p-HO-Phenyl | 6-Methylsulfinyl-3-pyridyl | 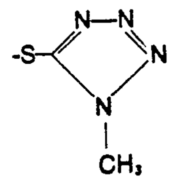 | $-CH_2OCOC(CH_3)_3$ |
| 91 | p-HO-Phenyl | 6-Methylsulfinyl-3-pyridyl | 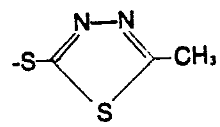 | H |
| 92 | 2-Thienyl | 6-Methylsulfinyl-3-pyridyl | 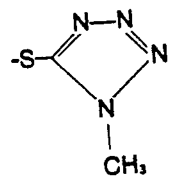 | H |
| 93 | p-HO-Phenyl | 6-Methylsulfonyl-3-pyridyl | $OCOCH_3$ | H |
| 94 | p-HO-Phenyl | 6-Methylsulfonyl-3-pyridyl | 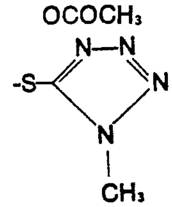 | H |
| 95 | p-HO-Phenyl | 6-Methylsulfonyl-3-pyridyl | 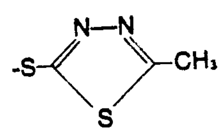 | H |
| 96 | 2-Thienyl | 6-Methylsulfonyl-3-pyridyl | 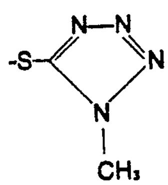 | H |
| 97 | p-HO-Phenyl | 5-Methyl-2-pyrrolylmethyl | $-OCOCH_3$ | H |
| 98 | p-HO-Phenyl | 5-Methyl-2-pyrrolylmethyl | 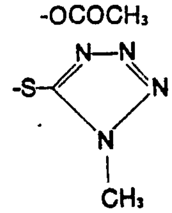 | H |

TABELLE 2 (Fortsetzung)

| | A | $(CH_2)_nR_1$ | D | E |
|---|---|---|---|---|
| 99 | p-HO-Phenyl | 4-Methyl-2-imidazolylmethyl | 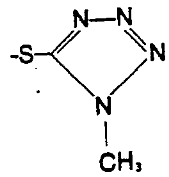 | H |
| 100 | p-HO-Phenyl | 4-Methyl-2-thiazolyl- | 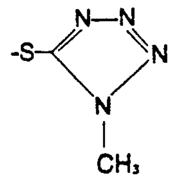 | H |
| 101 | p-HO-Phenyl | 4-Methyl-2-thiazolylmethyl- | 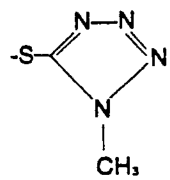 | H |
| 102 | p-HO-Phenyl | 5-Methyl-2-thiadiazolyl- | 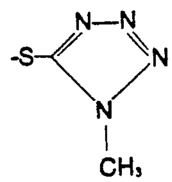 | H |
| 103 | p-HO-Phenyl | 2-Triazolylmethyl | 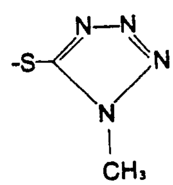 | H |
| 104 | p-HO-Phenyl | 2-Thienylmethyl | 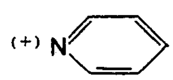 | — |
| 105 | p-HO-Phenyl | 2-Thienylmethyl | 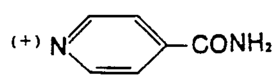 | — |
| 106 | p-HO-Phenyl | 2-Furylmethyl | 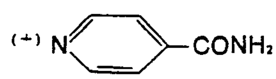 | — |
| 107 | p-HO-Phenyl | 3-Pyridylmethyl | 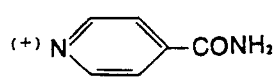 | — |
| 108 | p-HO-Phenyl | 6-Methylsulfonyl-3-pyridyl | 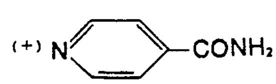 | — |
| 109 | p-HO-Phenyl | 5-Aminosulfonyl-2-thienylmethyl | 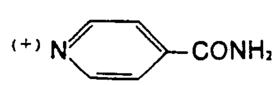 | — |

TABELLE 2 (Fortsetzung)

| | A | $(CH_2)_nR_1$ | D | E |
|---|---|---|---|---|
| 110 | p-HO-Phenyl | 6-Methylsulfinyl-3-pyridyl | 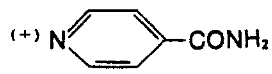 | — |
| 111 | p-HO-Phenyl | 2-Methyl-5-pyrimidinylmethyl | 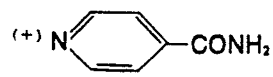 | — |
| 112 | p-HO-Phenyl | 5-Aminocarbonyl-2-thienyl | $OCOCH_3$ | H |
| 113 | p-HO-Phenyl | 5-Aminocarbonyl-2-thienyl | 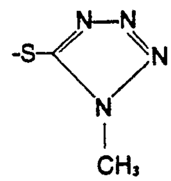 | H |
| 114 | 2-Thienyl | 5-Aminocarbonyl-2-thienyl | 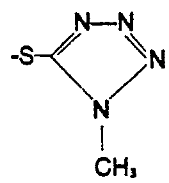 | H |
| 115 | p-HO-Phenyi | 6-Hydroxy-3-pyridyl | $OCOCH_3$ | H |
| 116 | p-HO-Phenyl | 6-Hydroxy-3-pyridyl | 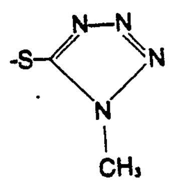 | H |
| 117 | p-HO-Phenyl | 6-Hydroxy-3-pyridyl | 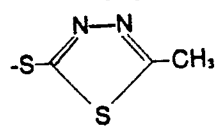 | H |
| 118 | p-HO-Phenyl | 2-Hydroxy-5-pyrimidinyl- | $OCOCH_3$ | H |
| 119 | p-HO-Phenyl | 2-Hydroxy-5-pyrimidinyl- | 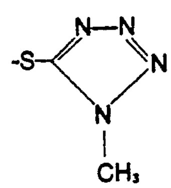 | H |
| 120 | p-HO-Phenyl | 2-Hydroxy-5-pyrimidinyl- | 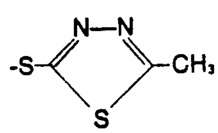 | H |
| 121 | 2-Thienyl | 2-Hydroxy-5-pyrimidinyl- | 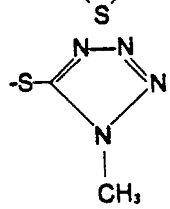 | H |
| 122 | 2-Thienyl | 6-Hydroxy-3-pyridyl- | 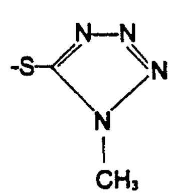 | H |

TABELLE 2 (Fortsetzung)

| | A | $(CH_2)_nR_1$ | D | E |
|---|---|---|---|---|
| 123 | p-HO-Phenyl | 2,4-Dihydroxy-5-pyrimidinyl | 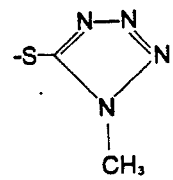 | H |
| 124 | p-HO-Phenyl | 2,6-Dihydroxy-4-pyrimidinyl | $OCOCH_3$ | H |
| 125 | p-HO-Phenyl | 2,6-Dihydroxy-4-pyrimidinyl | 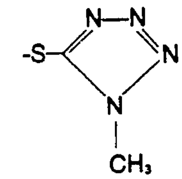 | H |
| 126 | p-HO-Phenyl | 4,6-Dihydroxy-2-pyrimidinyl | $OCOCH_3$ | H |
| 127 | p-HO-Phenyl | 4,6-Dihydroxy-2-pyrimidinyl | 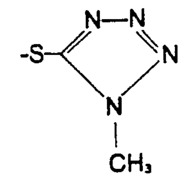 | H |

Die Wirksamkeit der erfindungsgemässen β-Lactam-Antibiotika lässt sich durch folgende Untersuchungen beispielhaft demonstrieren:

1. In vitro-Versuche

Für die Untersuchungen wurde die Methode des Reihenverdünnungstestes im Mikrotitersystem angewandt. Die Prüfung der Substanzen auf Bakteriostase erfolgte in flüssigem Medium. Es wurde die Bakteriostasewirkung bei folgenden Konzentrationen untersucht:

128; 64; 32; 16; 8; 4; 2; 1; 0,5; 0,25; 0,12; 0,06 µg/ml.

Es wurde ein Nährboden folgender Zusammensetzung benutzt: 10 g Pepton, 8 g Fleischextrakt-Oxoid, 3 g Natriumchlorid, 2 g sek. Natriumsphosphat werden mit dest. Wasser auf 100 ml aufgefüllt (pH 7,2-7,4). Lediglich bei der Testung gegen Streptokokken wurde 1% Glucose hinzugefügt. Das Alter der Primärkulturen betrug etwa 20 Stunden. Die Einstellung der Keimsuspension erfolgte am Photometer (nach «Eppendorf») (Reagenzglas-Durchmesser 14 mm, Filter 546 nm) anhand der Trübung einer Bariumsulfat-Vergleichssuspension, die durch eine Bariumsulfat-Aufschwemmung erzeugt wurde, welche durch die Zugabe von 3,0 ml 1%ige Barium-chloridlösung in 97 ml 1%ige Schwefelsäure entstand. Nach der Einstellung wurden Streptococcus Aronson im Verhältnis 1:15 und die übrigen Testkeime im Verhältnis 1:1500 mit einer Kochsalzlösung weiter verdünnt.

16 mg der jeweiligen Substanz wurden in 10 ml-Messkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension (0,01 ml) beschickt und 18 bis 20 Stunden bei 37°C bebrütet. Eine Lösungsmittelkontrolle wurde stets mitgeführt.

Die Ablesung wurde makroskopisch vorgenommen, wobei die jeweilige Grenzkonzentration (= niedrigste noch bakteriostatisch wirksame Konzentration) ermittelt wurde.

Als Testorganismen wurden benützt:

Staphylococcus aureus SG 511, Escherichia coli ATCC 11 775, Pseudomonas aeruginosa Hamburgensis und Pseudomonas aeruginosa Walter, Serratia marcescens ATCC 13 880, Klebsiella pneumoniae ATCC 10 031 und 272, Proteus mirabilis Hamburgensis, Proteus rettgeri, Enterbacter cloaceae ATCC 13 047, E. coli R+TEM (β-Lactamase-Träger).

In der folgenden Tabelle 1 sind die ermittelten minimalen Hemmkonzentrationen (MIC) für typische Vertreter der erfindungsgemässen Verbindungen aufgeführt:

a) Penicilline

Natriumsalze von Verbindungen der allgemeinen Formel I mit A=p-Hydroxyphenyl und folgender Bedeutung von R:

| R | Verbindung |
|---|---|
| 3-Pyridylmethyl | A |
| 5-Aminocarbonyl-2-thienyl | B |
| 2-Thienylmethyl | C |
| 2-Furylmethyl | D |
| 6-Methylsulfonyl-3-pyridyl | E |
| 2-Methyl-5-pyrimidinylmethyl | F |
| 6-Hydroxy-3-pyridyl | G |
| im Vergleich zu Azlocillin | H |

TABELLE 1

| Substanz | E. coli ATCC 9637 | E. coli ATCC 11775 | Pseud. aerug. Hbg. | Pseud. aerug. Walter | Serrat. marcesc. ATCC 13880 | Klebs. pneum. ATCC 10031 | Klebs. pneum. 272 | Prot. mira-bilis | Prot. rett-geri | Eb. cloa-ceae ATCC 13047 |
|---|---|---|---|---|---|---|---|---|---|---|
| A | 0,5 | 0,5 | 1 | 1 | 0,5 | 8 | 8 | 0,12 | 1 | 4 |
| B | 0,5 | 0,25 | 1 | 1 | 0,2 | 2 | 2 | 0,06 | 0,5 | 2 |
| C | 0,5 | 0,5 | 2 | 1 | 0,5 | 2 | 2 | 0,25 | 0,12 | 0,5 |
| D | 0,5 | 1 | 1 | 2 | 0,5 | 4 | 8 | 0,25 | 0,5 | 4 |
| E | 0,2 | 0,2 | 1 | 1 | 0,2 | 2 | 2 | 0,06 | 0,25 | 2 |
| F | 0,5 | 0,5 | 1 | 1 | 0,5 | 8 | 8 | 0,06 | 0,5 | 4 |
| G | 0,2 | 0,2 | 0,5 | 0,5 | 0,1 | 4 | 4 | 0,01 | 0,5 | 4 |
| H | 16 | 8 | 8 | 8 | 4 | 32 | 32 | 2 | 8 | 32 |

b) Cephalosporine

Natriumsalze von Verbindungen der allgemeinen Formel I mit folgender Bedeutung von A, R und D

| A | R | D | Verbindung |
|---|---|---|---|
| 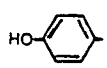 | 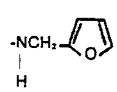 | 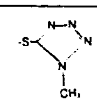 | J |
| 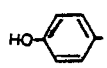 | 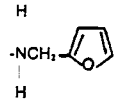 | $-OCOCH_3$ | K |
| 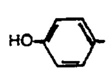 | 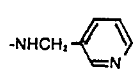 | 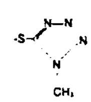 | L |
| 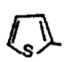 (Diastereomeres I) | 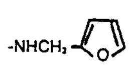 | 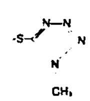 | M |
| 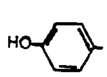 | 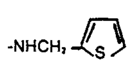 | 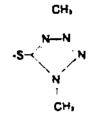 | N |
| 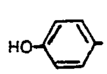 | 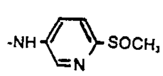 | 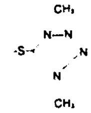 | O |
| 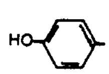 | 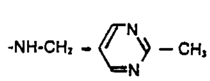 | 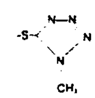 | P |

TABELLE 2

MHK-Werte verschiedener Cephalosporine (in µg/ml)

| Verbindung | S. aureus SG 511 | E. coli ATCC 11775 | Pseud. aerug. Hbg. | Pseud. aerug. Walter | Klebs. pneum. ATCC 10031 | Klebs. pneum. 272 | Prot. mir. Hbg. | Prot. rettg. | Eb. cloa-ceae | E. coli R+TEM | Serr. marcesc. ATCC 13880 |
|---|---|---|---|---|---|---|---|---|---|---|---|
| Cefuroxim | 1 | 8 | 128 | 128 | 2 | 4 | 0,5 | 2 | 32 | 4 | 8 |
| Cephazolin | 0,06 | 4 | 128 | 128 | 1 | 2 | 4 | 128 | 128 | 4 | 128 |
| J | 0,5 | 0,25 | 8 | 4 | 0,5 | 0,5 | 0,12 | 0,5 | 0,5 | 8 | 0,25 |
| K | 1 | 0,5 | 16 | 8 | 1 | 1 | 0,25 | 1 | 4 | 16 | 2 |
| L | 0,5 | 0,12 | 4 | 4 | 0,25 | 0,25 | 0,06 | 0,5 | 0,25 | 4 | 0,25 |
| M | 0,25 | 0,25 | 8 | 4 | 0,25 | 0,5 | 0,12 | 0,5 | 0,25 | 2 | 0,5 |
| N | 0,5 | 0,25 | 8 | 4 | 0,25 | 0,25 | 0,12 | 0,5 | 0,25 | 4 | 0,25 |
| O | 0,25 | 0,25 | 4 | 4 | 0,25 | 0,25 | 0,12 | 0,5 | 0,5 | 4 | 0,5 |
| P | 0,25 | 0,12 | 4 | 4 | 0,25 | 0,5 | 0,06 | 0,25 | 0,5 | 4 | 0,25 |

Wie aus den vorstehenden Tabellen ersichtlich wird, sind die genannten Verbindungen der Vergleichssubstanzen in ihrer Wirksamkeit gegenüber typischen gramnegativen Hospitalkeimen bei Erhalt der Wirkung gegen grampositive Keime deutlich überlegen. Hervorzuheben ist die gute Wirksamkeit gegen Pseudomonasstämme.

Die akute Toxizität wurde durch perorale und subcutane Applikation der Verbindungen der Tabelle 1 und 2 an weissen Laboratoriumsmäusen in steigenden Dosen bestimmt.

Die $LD_{50}$ ist die Dosis, nach deren Applikation 50% der Tiere innerhalb von 8 Tagen sterben. Sämtliche Substanzen zeigten bei oraler Gabe eine $LD_{50}$ von über 4 g/kg, bei subcutaner Gabe eine $LD_{50}$ von über 3 g/kg, d.h. bei 3 g/kg starben keine Tiere, sie sind damit für die Praxis untoxisch.

Eine Reihe der erfindungsgemässen Verbindungen wurde in vivo bei experimentellen Infektionen an Mäusen untersucht. Man verwendete als pathogene Bakterien E. coli ATCC 11775 und Pseudomonas aeruginosa Walter. Es wurde eine intraperitoneale Infektion mit 0,2 ml einer Bakteriensuspension (mit 5% Mucin) gesetzt. Dies entspricht etwa $1{,}4 \times 10^6$ Keime E. coli bzw. $1{,}3 \times 10^6$ Keime Pseudomonas/Maus. Weibliche Mäuse vom Stamm NMRI wurden in Gruppen von jeweils 10 Tieren aufgeteilt, zwei Gruppen blieben unbehandelt, die restlichen Gruppen wurden mit verschiedenen Dosen der jeweiligen erfindungsgemässen Cephalosporine subcutan zur Bestimmung der $ED_{50}$ (Dosis bei der 50% der Tiere überleben) behandelt. Bei der E. coli-Infektion wurde am ersten Tag dreimal therapiert (1,4 und 7 h post-Infektionem) und am 2. Tag 2 mal. Bei der Pseudomonas-Infektion wurde am ersten Tag 6x (1, 3, 5, 7, 9, 11 h post-Infektionem) und an den darauffolgenden 2 Tagen zweimal täglich behandelt.

Der Beobachtungszeitraum betrug in beiden Fällen 7 Tage. Die Ergebnisse dieser Teste mit repräsentativen Vertretern der erfindungsgemässen Penicilline und Cephalosporine sind in der Tabelle 3 dargestellt.

TABELLE 3

In vivo Aktivität bei Mäusen

a) E. coli-Infektion (s.c. Applikation):

| | Verbindung | $ED_{50}$ (mg*/kg) |
|---|---|---|
| | A | 0,7 |
| | B | 1,3 |
| | C | 0,8 |
| | D | 0,8 |
| Azlocillin | H | ~35 |
| | J | 0,3 |
| | L | 0,2 |
| | M | 0,8 |
| | N | 0,6 |
| Cefuroxim | | 37 |

* pro Dosis

b) Pseudomonas (s.c. Applikation):

| Verbindung | $ED_{50}$ (mg*/kg) |
|---|---|
| A | 1,2 |
| B | 4,8 |
| C | 2,5 |
| D | 1,5 |

| | | | |
|---|---|---|---|
| Azlocillin | H | | ~110 |
| | J | | 6,3 |
| | L | | 4,6 |
| Cefuroxim | | >200 d.h. bei | 200 mg/kg alle Tiere gestorben |

* pro Dosis

Es ist eine weitere Aufgabe der vorliegenden Erfindung, pharmazeutische Mittel zu schaffen, die bei der Behandlung infektiöser Krankheiten sowohl beim Menschen als auch beim Tier wertvoll sind.

Als bevorzugte pharmazeutische Zubereitungen seien Tabletten Dragées, Kapseln, Granulate, Suppositorien, Lösungen, Suspensionen, Emulsionen, Salben, Gele, Cremes, Puder und Sprays genannt. Vorteilhafterweise wird der Wirkstoff in der Human- oder Tiermedizin oder ein Gemisch der verschiedenen Wirkstoffe der allgemeinen Formel I in einer Dosierung zwischen 5 und 500, vorzugsweise 10-200 mg/kg Körpergewicht je 24 Stunden verabreicht, gegebenenfalls in Form mehrerer Einzelgaben. Eine Einzelgabe enthält den oder die erfindungsgemässen Wirkstoffe, vorzugsweise in Mengen von etwa 1 bis etwa 250, insbesondere 10 bis 60 mg/kg Körpergewicht. Es kann jedoch erforderlich sein, von den genannten Dosierungen abzuweichen, und zwar in Abhängigkeit von der Art und dem Körpergewicht das zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wächstums und eine Verbesserung der Verwertung des Futters erreicht werden.

Die folgenden Beispiele sollen die Erfindung näher erläutern:

I Darstellung der Ausgangsprodukte

Beispiel 1 Aminopyrimidine

1a) 5-Amino-4-hydroxy-2-(2'-thienylmethylamino)-pyrimidin

Eine Suspension von 10,5 g (0,05 Mol) 2-Äthylmercapto-4-hydroxy-5-nitro-pyrimidin und 5,65 g (0,05 Mol) 2-Thienylmethylamin in 250 ml n-Propanol wird 8 h auf 100°C erhitzt. Nach dem Abkühlen wird das ausgefallene Festprodukt abgesaugt, mit wenig eiskaltem Propanol und anschliessend mit Äther ausgewaschen. Ein Dünnschichtchromatogramm (Silicagel, Dichlormethan/Methanol 10:1) zeigt, dass das Ausgangsprodukt vollständig umgesetzt wurde.
Ausbeute: 11,90 g (94%).

2,72 (0,01 Mol) der so gewonnenen Nitroverbindung werden in 40 ml Wasser suspendiert, mit 10 ml konzentriertem Ammoniak und unter Rühren portionsweise mit 7,7 g (0,05 Mol) Natriumdithionit versetzt. Anschliessend wird 30 min auf dem Dampfbad erwärmt. Ein Dünnschichtchromatogramm der wässrigen Lösung (Silicagel, Dichlormethan/Methanol 10:1) zeigt an, dass kein Ausgangsprodukt mehr vorhanden ist. Es wird im Vakuum zur Trockne eingeengt und das zurückbleibende Festprodukt mehrmals mit insgesamt 300 ml Tetrahydrofuran extrahiert. Das Lösungsmittel wird im Vakuum entfernt. Man erhält so 1,65 g (74%) der gewünschten Verbindung.
Fp.: 66-68°C

| | | | |
|---|---|---|---|
| Ber.: | C 48,63 | H 4,53 | N 25,21 |
| Gef.: | C 48,47 | H 4,51 | N 24,99 |

1b) 5-Amino-4-hydroxy-2-(2'-thiazolylamino)-pyrimidin

Wie in Beispiel 1a werden 4,02 g (0,02 Mol) 2-Äthylmercapto-4-hydroxy-5-nitropyrimidin mit 2,0 g 2-Aminothiazol eingesetzt. Die Reaktionszeit beträgt 24 Stunden. Man erhält 3,75 g (79%) Nitroverbindung, die ohne weitere Reinigung in 150 ml Dimethylformamid gelöst und bei 50°C mit 2 g Palladium/Kohlenstoff (5%ig) bis zur Aufnahme der berechneten Wasserstoffmenge unter Normaldruck hydriert werden. Man entfernt das Lösemittel im Vakuum und rührt das zurückbleibende Fertigprodukt mit Äthanol aus. Nach dem Absaugen bleiben 2,75 g (84%) der gewünschten Verbindung zurück.
Fp.: >300°C

| | | | |
|---|---|---|---|
| Ber.: | C 40,18 | H 3,37 | N 33,47 |
| Gef.: | C 40,00 | H 3,37 | N 32,80 |

1c) 5-Amino-4-hydroxy-2-(2'-thienylmethylamino)-pyrimidin

Die unter 1a) genannte Verbindung kann auch auf folgende Art synthetisiert werden:

169,5 g Aminomethylthiophen (1,5 Mol) und 93 g Eisessig werden gemischt. Das entstehende Pulver wird in einem Mörser zerkleinert. Es werden 130,5 g (0,5 Mol) 2-Methylmercapto-4-hydroxy-5-benzylamino-pyrimidin hinzugefügt. Das entstehende Gemisch wird in einem 1l-Kolben 6 Stunden lang auf 180°C erhitzt, wobei gerührt wird.

Die entstandene Mischung wird zweimal mit 0,5 l warmem Äthylalkohol verrührt und das zurückbleibende Festprodukt abgenutscht und mit 150 ml Aceton gewaschen.
Ausbeute: 120 g.
Fp.: 275°C (aus Dimethylformamid umkristallisiert).

| | | | |
|---|---|---|---|
| Ber.: | C 58,88 | H 4,32 | N 17,17 |
| Gef.: | C 59,00 | H 4,39 | N 17,21 |

3,26 g (0,01 Mol) des so erhaltenen Produktes werden mit 4 g Natriumhydroxid, 0,2 g Natriumsulfit und 20 ml Wasser 3 Stunden lang zum Rückfluss erhitzt. Das Gemisch wird abgekühlt und mit konzentrierter Salzsäure auf pH 0,5 angesäuert, die ausgefallene Benzoesäure wird durch Ausschütteln mit Äther entfernt. Die wässrige Phase wird mit Natronlauge unter Kühlung auf pH 6,9 gestellt und das ausgefallene Produkt angesaugt.
Ausbeute: 1,9 g (85%).

1d) 2-Chlor-4-hydroxy-5-nitro-pyrimidin-natrium-monohydrat

Zur Lösung von 9,7 g (0,05 Mol) 2,4-Dichlor-5-nitropyrimidin wird eine Lösung von 8,2 g (0,1 Mol) Natriumacetat in 25 ml Wasser und 10 ml Eisessig bei +10°C unter Rühren zugetropft. Nach vollständigem Zutropfen der Pufferlösung wird noch 1 Stunde unter Eiskühlung gerührt. Der ausgefallene, dicke Niederschlag wird abgesaugt und im Vakuum über Calciumchlorid getrocknet. Das 2-Chlor-4-hydroxy-5-nitro-pyrimidin fällt in Form seines Natriumsalzes mit einem Molekül Kristallwasser an.
Ausbeute: 10,20 g (94,4%).
Fp.: kein Fp., ab 150°C Wasserabspaltung, ab 195°C Zersetzung.

| | | | | |
|---|---|---|---|---|
| Ber.: | C 22,29 | H 1,40 | Cl 16,45 | N 19,50 |
| Gef.: | C 22,42 | H 1,50 | Cl 16,46 | N 19,84 |

Wassergehalt (nach Karl Fischer):
Ber.: 8,36
Gef.: 8,4 %

1e) 4-Hydroxy-2-(2'-methyl-5'-pyrimidinylmethylamino)-5-nitro-pyrimidin

Eine Lösung von 0,75 g (0,0061 Mol) 2-Methyl-5-methylaminopyrimidin in 15 ml Dioxan wird zur Lösung von 1,4 g (0,0065 Mol) 2-Chlor-4-hydroxy-5-nitro-pyrimidin-natrium-monohydrat in 20 ml Wasser zugegeben, wobei eine homogene Lösung erhalten wird. Diese Lösung wird 2,5 Stunden unter Rückfluss gekocht. Das ausgefallene Umsetzungsprodukt wird abgesaugt, mit Wasser gewaschen und bei 50-100°C im Vakuum getrocknet.
Ausbeute: 1,05 g (65,6%)
Fp.: 244-246°C (Zers.)

| | | | |
|---|---|---|---|
| Ber.: | C 45,80 | H 3,84 | N 32,04 |
| Gef.: | C 45,33 | H 4,02 | N 32,03 |

1f) 5-Amino-4-hydroxy-2-(2'-methyl-5'-pyrimidinylmethylamino)-pyrimidin

1,0 g des unter 1e) genannten Nitropyrimidins werden zusammen mit 0,5 g Pd/C (10%ig), 80 ml Methanol, 10 ml Wasser und 5 ml konzentrierte Salzsäure in einem Hydriergefäss bei Normaldruck und Raumtemperatur hydriert. Nach Ende der Wasserstoffaufnahme wird vom Katalysator abfiltriert, das Methanol im Vakuum abgezogen und etwas Wasser hinzugefügt. Mit Natronlauge wird der pH-Wert auf 4,5 gestellt und das ausgefallene Produkt abgesaugt.
Ausbeute: 720 mg.
Zersetzung >250°C

Nach einer der genannten Methoden wurden folgende neue Pyrimidine synthetisiert:

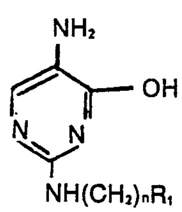

| | $(CH_2)_nR_1$ | Ausbeute in % | Fp. |
|---|---|---|---|
| g) | 3'-Pyridyl | 62 | >300°C |
| h) | 6'-Methoxy-3'- | 44 | >300°C |

| | $(CH_2)_nR_1$ | Ausbeute in % | Fp. |
|---|---|---|---|
| i) | pyridyl | | |
| k) | 2'-Pyridylmethyl- | 57 | sintert ab 100°C |
| l) | 3'-Pyridylmethyl | 84 | 150°C |
| m) | 5'-Pyrimidinyl- | 34 | 166-170°C |
| n) | 2'-Amino-5'-pyrimidinyl | 71 | >200°C (Zers.) |
| o) | 2'-Propylamino-5'-pyrimidinyl | 36 | Zers. >210°C |
| p) | 5'-Äthoxycarbonyl-2'-thienyl- | 64 | 175°C |
| q) | 5'-Methyl-2'-thienyl | 52,5 | 120-123°C |
| r) | 3'-Thienylmethyl- | 64 | 90-94°C |
| s) | 5'-Chlor-2'-thienylmethyl- | 43 | 86°C |
| t) | 2'-Furylmethyl- | 61 | sinter ab ~100°C |
| u) | 3'-Furylmethyl- | 48 | Zers. >120°C |
| v) | 5'-Methyl-2'-furylmethyl- | 52 | Zers. >130°C |
| w) | 2'-Tetrahydrofurylmethyl- | 46 | sintert ab ~80°C |
| x) | 2'-Pyrrolylmethyl- | 53 | |
| y) | 2'-Imidazolylmethyl- | 48 | |
| t) | 4'-Methyl-2'-thiazolylmethyl- | 66 | sintert ab ~90°C |
| aa) | 4'-Oxazolylmethyl- | 60 | 110-120°C |
| ab) | 5'-Methyl-2'-thiadiazolyl- | 57,5 | Zers. >250°C |
| ac) | 5'-Methyl-2'-triazolylmethyl- | 41 | Zers. >110°C |
| ad) | 5'-Methyl-2'-triazolyl- | 49 | Zers. >250°C |
| ae) | 4'-Pyridylmethyl- | 52 | ~175°C (Zers.) |
| af) | 4'-Pyrimidinylmethyl- | 35 | 213°C |
| ag) | 5'-Methyl-2'-thienylmethyl | 66 | ~80°C |
| ah) | 2'-Pyridyl | 42 | 245-248°C |
| ai) | 6'-Hydroxy-3'-pyridyl | 46 | >300°C |
| ak) | 6'-Hydroxy-2'-pyridyl | 27 | >300°C |
| al) | 2'-Hydroxy-5'-pyrimidinyl | 45 | >300°C |
| am) | 2'-Cyclopropyl-4'-hydroxy-5'--pyrimidinyl | 41 | Zers. >220°C |
| an) | 2'-Methyl-5'-pyrimidinyl | 38 | 244°C |
| ao) | 5'-Aminocarbonyl-2'-thienyl | 22 | Zers. >110°C |
| ap) | 5'-Tetrazolylmethyl | 41,5 | Zers. >270°C |
| aq) | 2',4'-Dihydroxy-5'-pyrimidinyl | 58 | Zers. 300°C |
| ar) | 4',6'-Dihydroxy-2'-pyrimidinyl | 47 | Zers. >300°C |
| as) | 2',6'-Dihydroxy-4'-pyrimidinyl | 44,5 | Zers. >300°C |

at) 4-Hydroxy-2-(6'-methylsulfinyl-3'-pyridylamino)-5-nitropyrimidin

4,68 g (0,03 Mol) 2-Mercapto-5-nitropyridin werden in einer durch Lösen von 1,32 g (0,033 Mol) Natriumhydroxid in 60 ml Wasser hergestellten Natronlauge unter schwachem Erwärmen gelöst. Zu dieser Lösung werden 4,17 g (0,033 Mol) Dimethylsulfat zugesetzt und das Gemisch gut durchgeschüttelt. Der entstehende Niederschlag wird abgesaugt und das noch feuchte Produkt aus Äthanol umkristallisiert. Man erhält 2-Methylthio-5-nitropyridin.
Ausbeute: 4,5 g (88,1%).
Fp.: 111-112°C

4,5 g (0,0264 Mol) 2-Methylthio-5-nitropyridin werden bei 50°C und 5 bar in Äthanol mit Raney-Nickel als Katalysator hydriert. Nach dem Abfiltrieren des Katalysators und Eindampfen im Vakuum erhält man ein Öl. Zur Lösung dieses Öls in Wasser wird eine Lösung von 6,84 g (0,032 Mol) 2-Chlor-4-hydroxy-5-nitropyrimidin-natrium-monohydrat in 150 ml Wasser zugesetzt. Die wässrige Lösung wird 30 Minuten auf dem Dampfbad erwärmt, der entstehende Niederschlag abgesaugt, mit Wasser gewaschen und getrocknet. Man erhält 4-Hydroxy-2-(6'-methylmercapto-3-pyridyl)amino-5-nitropyrimidin.
Ausbeute: 7 g (94,8%).
Fp.: 295°C (Zers.)
Für die Analyse wird die Verbindung in Dimethylsulfoxid gelöst und mit Methanol gefällt.

| | | | |
|---|---|---|---|
| Ber.: | C 43,00 | H 3,25 | N 25,08 |
| Gef.: | C 43,09 | H 3,36 | N 24,90 |

5,59 g (0,02 Mol) 4-Hydroxy-2-(6'-methylmercapto-3-pyridylamino)-5-nitropyrimidin werden in 20 ml Ameisensäure gelöst und 1,87 g (0,022 Mol) 50%iges Perhydrol zugesetzt. Nach 3 Stunden Stehen bei Zimmertemperatur entsteht ein Niederschlag, der durch Zusatz von Aceton vervollständigt wird.
Ausbeute: 5,4 g (91%).
Fp.: >300°C

| | | | |
|---|---|---|---|
| Ber.: | C 40,67 | H 3,07 | N 23,72 |
| Gef.: | C 40,36 | H 3,06 | N 23,50 |

au) 5-Amino-4-hydroxy-2-(6'-methylsulfinyl-3'-pyridylamino)-pyrimidin

Die Reaktion erfolgt mit Dithionit, entsprechend der in Beispiel 1a( angegebenen Vorgehensweise.
Ausbeute: 56%
IR-Spektrum: 1660, 1020 $cm^{-1}$,
NMR-Spektrum ($CDCl_3/CD_3OD$) Signal bei ppm: 2,9 (s, 3H), 7,3 (s, 1H), 7,85 u. 8,4 (m, 2H), 8,8 (d, 1H).

av) 4-Hydroxy-2-(6-methylsulfonyl-3-pyridylamino)-5-nitropyrimidin

Dieses 4-Hydroxy-5-nitropyrimidin wird analog zu Beispiel 1au) dargestellt. Dabei wird ein Überschuss an Perhydrol eingesetzt und die Reaktionsdauer auf 5 Tage verlängert.
Ausbeute: 72,6%
Fp.: 300°C

Das Präparat enthält noch geringe Mengen der Methylsulfinylverbindung. Diese Verunreinigung kann nach der Reduktion der Nitrogruppe durch Säulenchromatographie abgetrennt werden.

aw) 5-Amino-4-hydroxy-2-(6'-methylsulfonyl-3'-pyridylamino)-pyrimidin

Die Reduktion erfolgt mit Dithionit entsprechend Beispiel 1a).
Ausbeute: 33%
IR-Spektrum: 1670, 1150, 1355 $cm^{-1}$,
NMR-Spektrum ($DMSO/CD_3OD$) Signale bei ppm: 3,1 (s, 3H), 7,15 (s, 1H), 7,9 u. 8,4 (m, 2H), 8,8 (d, 1H).

ax) 5-Amino-4-hydroxy-2-(5'-sulfonamido-2'-thienylmethylamino)-pyrimidin

4 g (0,0123 Mol) 5-Benzoylamino-4-hydroxy-2-(2'-thienylmethylamino)-pyrimidin werden unter Kühlung portionsweise in 14,32 g (0,12 Mol) Chlorsulfonsäure eingetragen. Die Lösung wird 1,5 Stunden bei Raumtemperatur gerührt und anschliessend die überschüssige Chlorsulfonsäure durch Eingiessen in Eiswasser zersetzt. Das ausgefallene 5-Benzoylamino-4-hydroxy-2-(5'-chlorsulfonyl-2'-thienylmethylamino)-pyrimidin wird abgesaugt und im Vakuum über Phosphorpentoxid getrocknet.
Ausbeute: 4,6 g (88,5%)
IR-Spektrum: 1170, 1370 $cm^{-1}$,
NMR-Spektrum ($DMSO/CD_3OD$) Signale bei ppm: 4,8 (s, 2H), 7,1 (dd, 2H), 7,55 (m, 3H), 7,9 (m, 2H), 8,4 (s, 1H).

4,6 g (0,0108 Mol) 5-Benzoylamino-4-hydroxy-2-(5'-chlorsulfonyl-2'-thienylmethylamino)pyrimidin werden in 100 ml Aceton aufgeschlämmt und 10 ml konzentrierte wässrige Ammoniaklösung zugesetzt. Es wird 10 Minuten auf dem Dampfbad erwärmt, wobei alles in Lösung geht. Die Lösung wird im Vakuum zur Trockne eingedampft und der Rückstand mit Wasser verrieben und abgesaugt. Der Rückstand wird in 30 ml Wasser aufgeschlämmt, 2 g Natriumhydroxid zugesetzt und 4 Stunden unter Rückfluss gekocht. Die Lösung wird mit Wasser verdünnt, mit Aktivkohle behandelt, filtriert, mit 2n-Salzsäure auf pH 7 eingestellt und über Nacht stehengelassen. Der ausgefallene Niederschlag wird abgesaugt und aus Wasser umkristallisiert.
Ausbeute: 0,85 g (26,1%).
IR-Spektrum: 1150, 1340 $cm^{-1}$,
NMR-Spektrum ($DMSO/CD_3OD$) Signale bei ppm: 4,55 (s, 2H), 6,95 (d, 2H), 7,35 (d, 1H).

Beispiel 2

Darstellung der Ureidocarbonsäuren

a) D-α-(2'-Furylmethylamino)-4-hydroxy-5-pyrimidinyl)-ureido-phenylessigsäure

2,47 g 5-Amino-2-(2'-furylmethylamino-4-hydroxy-pyrimidin (0,012 Mol) werden in 80 ml absolutem Tetrahydrofuran gelöst und mit 1,65 ml Triäthylamin versetzt. Diese Lösung wird bei 0°C zu einer Lösung von 1,20 g Phosgen in 25 ml Tetrahydrofuran getropft. Man rührt Eiskühlung etwa 10 Minuten nach. Anschliessend wird Stickstoff durch die Lösung geblasen, um nicht umgesetztes Phosgen zu entfernen.

Eine Suspension von 1,8 g (0,012 Mol) D-α-Amino-phenylessigsäure in 50 ml Tetrahydrofuran und 20 ml Wasser wird durch Zugabe von 12 ml 1n Natronlauge unter Kühlen und Rühren in Lösung gebracht. Zu dieser Lösung wird unter Eiskühlung die oben hergestellte Suspension getropft, wobei der pH-Wert mit n-Natronlauge auf 8,0-8,5 gehalten wird. Es wird eine Stunde bei 5°C und 2 Stunden bei Raumtemperatur nachgerührt. Dann wird das Tetrahydrofuran im Vakuum entfernt und die zurückbleibende wässrige Lösung zweimal mit Essigsäureäthylester bei pH 8,0-8,5 ausgeschüttelt. Die wässrige Phase wird dann unter Kühlen und Rühren mit verdünnter Salzsäure auf pH 2,9 eingestellt. Das ausgefallene Festprodukt wird abgesaugt, mit wenig eiskaltem Wasser gewaschen und getrocknet.

Ausbeute: 2,8 g (70%).

IR-Spektrum: 3320 (breit), 1650, 1550 $cm^{-1}$;

NMR-Spektrum ($CDCl_3+D_2O$) Signale bei ppm: 4,4 (s, 2H), 5,15 (s, 1H), 6,3 (m, 2H), 7,5 (m, 6H), 8,1 (s, 1H).

a) D-α-[(2-(2'-Methyl-5'-pyrimidinylmethylamino)-4-hydroxy-5-pyrimidinyl)-ureido]-p-hydroxy-phenylessigsäure

2,32 g 5-Amino-2-(2'-methyl-5'-pyrimidinylmethylamino)-4-hydroxy-pyrimidin (0,01 Mol) werden in 100 ml trockenem Tetrahydrofuran aufgeschlämmt und bis zur vollständigen Lösung mit 4 ml Trimethylsilyldiäthylamin am Rückfluss erhitzt. Es wird im Vakuum zur Trockene eingeengt. Das zurückbleibende Festprodukt wird in 50 ml trockenem Tetrahydrofuran gelöst und unter Eiskühlung zu einer Lösung von 1,05 g Phosgen in 40 ml Tetrahydrofuran getropft.

Nach Entfernung von überschüssigem Phosgen setzt man analog zu Beispiel 1 mit 1,65 g p-Hydroxyphenylessigsäure weiter um.

Ausbeute: 2,78 g (66%).

IR-Spektrum: 3330 (breit), 1650, 1560 $cm^{-1}$,

NMR-Spektrum ($DMSO+CD_3OD$) Signale bei ppm: 4,4 (s, 2H), 5,05 (s, 1H), 6,7 (d, 2H), 7,20 (d, 2H), 8,05 (s, 1H), 8,6 (s, 2H).

Nach diesen Methoden wurden folgende Ureidocarbonsäuren synthetisiert:

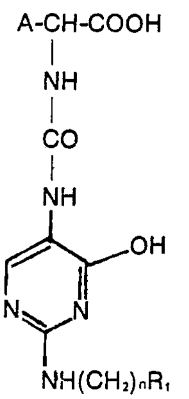

| | A | $(CH_2)_nR_1$ | Ausbeute % | NMR-Spektrum (DMSO/$CD_3OD$) |
|---|---|---|---|---|
| b) | p-HO-Phenyl | 3-Pyridyl | 71 | 5,15 (s, 1H), 6,8 (d, 2H), 7,25 (d, 2H), 7,4 (m, 1H), 8,3 (m, 3H), 8,8 (breites s, 1H) |
| c) | 2-Furyl | 3-Pyridyl | 66 | 5,5 (s, 1H), 6,3 (m, 2H), 7,4 (m, 1H), 7,6 (s, 1H), 8,3 (m, 3H), 8,75 (1H) |
| d) | 2-Thienyl | 3-Pyridyl | 64 | 5,50 (s, 1H), 7,0 (m, 2H), 7,4 (m, 2H), 8,25 (m, 3H), 8,8 (breites s, 1H) |
| e) | p-HO-Phenyl | 3-Pyridyl-methyl | 80,5 | 4,5 (breites s, 2H), 5,15 (s, 1H), 6,8 (d, 2H), 7,3 (m, 3H), 7,7 (m, 1H), 8,1 (s, 1H), 8,5 (m, 2H) |

| | A | $(CH_2)_nR_1$ | Ausbeute % | NMR-Spektrum (DMSO/$CD_3OD$) |
|---|---|---|---|---|
| f) | p-HO-Phenyl | 5-Äthoxycarbonyl-2-thienyl | 49 | 1,3 (t, 3H), 4,3 (q, 2H), 5,2 (s, 1H), 6,8 (d, 2H), 7,3 (d, 2H), 7,9 (m, 2H), 8,3 (s, 1H) |
| g) | p-HO-Phenyl | 2-Thienylmethyl | 54 | 4,5 (2H), 5,15 (s, 1H), 6,6-7,4 (m, 7H), 8,1 (s, 1H) |
| h) | p-HO-Phenyl | 5-Methyl-2-thienylmethyl | 70,5 | 2,4 (s, 3H), 4,5 (s, 2H), 5,15 (s, 1H), 6,7 (d, 2H), 6,8 (breites s, 2H), 7,20 (d, 2H), 8,1 (s, 1H) |
| i) | p-HO-Phenyl | 5-Chlor-2-thienylmethyl | 65 | 4,4 (s, 2H), 5,15 (s, 1H), 6,7-7,0 (m, 4H), 7,25 (d, 2H), 8,15 (s, 1H) |
| j) | p-HO-Phenyl | 2-Furylmethyl | 58 | 4,4 (2H), 5,1 (1H), 6,3 (m, 2H), 6,7 (d, 2H), 7,2 (d, 2H), 7,5 (s, 1H), 8,05 (s, 1H) |
| k) | m,p-Di-OH-Phenyl | 2-Thienylmethyl- | 62 | 4,5 (s, 2H), 5,0 (s, 1H), 6,7 (d, 1H), 7,0 (m, 4H), 7,3 (m, 1H), 8,1 (s, 1H) |
| l) | 2-Furyl | 2-Thienylmethyl- | 53 | 4,5 (s, 2H), 5,45 (s, 1H), 6,4 (m, 2H), 7,0 (m, 2H), 7,3 (m, 1H), 7,6 (s, 1H), 8,1 (s, 1H) |
| m) | 2-Thienyl | 2-Thienylmethyl- | 57 | 4,5 (s, 2H), 5,50 (m, 1H), 7,0 (m, 4H), 7,25 (m, 2H), 8,1 (s, 1H) |
| n) | 2-Furyl | 2-Furylmethyl- | 74 | 4,4 (s, 2H), 5,5 (s, 1H), 6,3 (m, 4H), 7,5 (m, 2H), 8,1 (s, 1H) |
| o) | 2-Thienyl | 2-Furylmethyl- | 56 | 4,4 (s, 2H), 5,45 (s, 1H), 6,3 (m, 2H), 7,0 (m, 2H), 7,35 (m, 1H), 7,5 (s, 1H), 8,1 (s, 1H) |
| p) | p-HO-Phenyl | 5-Methyl-2-furylmethyl | 77 | 2,5 (s, 3H), 4,5 (s, 2H), 5,15 (s, 1H), 6,3 (m, 2H), 6,8 (d, 2H), 7,3 (d, 2H), 8,1 (s, 1H) |
| q) | p-HO-Phenyl | Tetrahydro-2-furylmethyl | 70 | 1.9 (m, 4H), 3,5-4,0 (m, 5H), 5,1 (s, 1H), 6,7 (d, 2H), 7,2 (d, 2H), 8,0 (s, 1H) |
| r) | p-HO-Phenyl | 2-Pyrrolylmethyl- | 48,5 | 4,3 (breites s, 2H), 5,15 (s, 1H), 6,1 (s, 2H), 6,7 (m, 1H+d, 2H), 7,3 (d, 2H), 8,1 (s, 1H) |
| | | | | 2,1 (s, 3H) |
| s) | p-HO-Phenyl | 4-Methyl-2-imidazolyl- | 51 | 4,4 (s, 2H), 5,15 (s, 1H), 6,8 (d, 2H), 7,2 (m, 4H), 8,05 (s, 1H) |
| t) | p-HO-Phenyl | 4-Methyl-2-thiazolyl- | 36,5 | 2,1 (s, 3H), 5,15 (s, 1H), 6,45 (s, 1H), 6,85 (d, 2H), 7,25 (d, 2H), 8,05 (s, 1H) |
| u) | p-HO-Phenyl | 5'-Methyl-2'-thienyl- | 44 | 2,5 (s, 3H), 5,15 (s, 1H), 6,5 (m, 2H), 6,8 (d, 2H), 7,25 (d, 2H), 8,1 (s, 1H) |
| v) | p-HO-Phenyl | 6-Methylsulfinyl-3-pyridyl | 52 | 2,7 (s, 3H), 5,15 (s, 1H), 6,70 (d, 2H), 7,2 (d, 2H), 7,7 (d, 1H), 8,25 (s, 1H), 8,5 (m, 1H), 8,9 (s, 1H) |
| w) | 2-Furyl | 6-Methylsulfinyl-3-pyridyl | 60 | 2,73 (s, 3H), 5,45 (s, 1H), 6,4 (m, 2H), 7,4 (m, 1H), 7,7 (d, 1H), 8,25 (s, 1H), 8,5 (m, 1H), 8,9 (s, 1H) |
| x) | 2-Thienyl | 6-Methylsulfinyl- | 64 | 2,75 (s, 3H), 5,50 (s, 1H), 7,0 (m, 2H), 7,4 (m, 1H), 7,7 (d, 1H), 8,25 (s, 1H), 8,5 (m, 1H), 8,85 (s, 1H) |

| | A | $(CH_2)_nR_1$ | Ausbeute % | NMR-Spektrum ($DMSO/CD_3OD$) |
|---|---|---|---|---|
| y) | p-HO-Phenyl | 6-Methylsulfonyl-3-pyridyl | 62 | 3,1 (s, 3H), 5,1 (s, 1H), 6,75 (d. 2H), 7,2 (d, 2H), 7,9 (d, 1H), 8,25 (s, 1H), 8,6 (d, 1H), 8,9 (s, 1H) |
| x) | p-HO-Phenyl | 6-Hydroxy-3-pyridyl | 51 | 5,05 (s, 1H), 6,45 (d, 1H), 6,75 (d, 2H), 7,25 (d, 2H), 7,6 (d, 1H), 7,9 (s, 1H), 8,15 (s, 1H) |
| aa) | 2-Furyl | 3-Pyridylmethyl | 56,5 | 4,5 (breites s, 2H), 5,5 (s, 1H), 6,3 (m, 2H), 7,3 (m, 1H), 7,4 (m, 1H), 7,7 (m, 1H), 8,1 (s, 1H), 8,5 (m, 2H) |
| ab) | 2-Thienyl | 3-Pyridylmethyl | 62 | 4,45 (breites s, 2H), 5,50 (s, 1H), 7,0 (m, 2H), 7,3 (m, 1H), 7,4 (m, 1H), 7,65 (m, 1H), 8,05 (s, 1H), 8,45 (m, 2H) |
| ac) | p-HO-Phenyl | 2-Methyl-5-pyrimidinyl | 59 | 2.5 (s, 3H), 5,15 (s, 1H), 6,75 (d, 2H), 7,25 (d, 2H), 8,3 (s, 1H), 9,1 (s, 2H) |
| ad) | p-HO-Phenyl | 5-Aminosulfonyl-2-thienylmethyl | 53,5 | 4.6 (s, 2H), 5,10 (s, 1H), 6,7 (d, 2H), 6,9 (d, 1H), 7,2 (d, 2H), 7,4 (d, 1H), 8,05 (s, 1H) |
| ae) | 2-Thienyl | 5-Aminosulfonyl-2-thienylmethyl | 61,5 | 4,55 (s, 2H), 5.50 (s, 1H), 7,0 (m, 3H), 7,4 (m, 2H), 8,05 (s, 1H) |
| af) | p-HO-Phenyl | 5-Aminocarbonyl-2-thienyl | 44 | 5.15 (s, 1H), 6,6 (d, 1H), 6,75 (d, 2H), 7.25 (d, 2H), 7,45 (d, 1H), 8,40 (s, 1H) |
| ag) | 2-Thienyl | 5-Aminocarbonyl-2-thienyl | 49,5 | 5,45 (s, 1H), 6.6 (d, 1H), 7,0 (m, 2H), 7,4 (m, 1+1H), 8,35 (s, 1H) |
| ah) | p-HO-Phenyl | 2-Hydroxy-5-pyrimidinyl | 64 | 5,15 (s, 1H), 6,80 (d, 2H), 7,25 (d, 2H), 8,35 (1H), 8.75 (s, 2H) |
| ai) | p-HO-Phenyl | 2,4-Dihydroxy-5-pyrimidinyl | 46,5 | 5,15 (s, 1H). 6,75 (d, 2H), 7,25 (d, 2H), 8,35 (s, 1H), 8,55 (s, 1H) |
| ak) | p-HO-Phenyl | 2,6-Dihydroxy-4-pyrimidinyl | 58 | 5,10 (s, 1H), 6,75 (d, 2H), 7,25 (d, 2H), 8.35 (s, 1H), 8,45 (s, 1H) |
| al) | p-HO-Phenyl | 4,6-Dihydroxy-2-pyrimidinyl | 53,5 | 5.15 (s, 1H), 6,75 (d, 2H), 7,30 (d, 2H), 8,30 (s, 1H), 8,80 (s, 1H) |
| am) | p-HO-Phenyl | 2'-Methyl-5'-pyrimidinylmethyl-amino | 62 | 2,45 (s, 3H), 4,45 (s. 2H), 5,15 (s, 1H), 6.65 (d, 2H), 7,2 (d, 2H), 8,05 (s, 1H), 8,6 (s, 2H) |

II Darstellung der Penicilline gemäss Formel I bzw. I'

Beispiel 1

D-α-[3-(4-Hydroxy-2-{3'-pyridylamino}-5-pyrimidinyl)-ureido]-p-hydroxybenzylpenicillin-Natrium

1,02 mg 5-Amino-4-hydroxy-2-(3'-pyridylamino)-pyrimidin (0,005 Mol) werden in absolutem Tetrahydrofuran gelöst und mit 500 mg Triäthylamin versetzt. Diese Lösung wird bei 0°C zu einer Lösung von 500 mg Phosgen in 30 ml absolutem Tetrahydrofuran getropft. Man rührt unter Eiskühlung etwa 30 Minuten nach. Anschiessend wird Stickstoff durch die Lösung geblasen, um nicht umgesetztes Phosgen zu entfernen.

Man suspendiert 2,1 g Amoxicillin-trihydrat (0,005 Mol) in 80 ml wässrigem 80%igem Tetrahydrofuran und kühlt auf 0°C ab. Dann wird soviel Triäthylamin zugegeben, dass eine Lösung entsteht. Innerhalb von 5 Minuten wird die oben hergestellte Suspension zugetropft, wobei der pH-Wert mit Triäthylamin auf 7,5 gehalten wird. Man setzt weitere 30 ml Wasser zu und hält das Reaktionsgemisch eine Stunde bei 0-2°C. Die Kühlung wird entfernt und es wird eine Stunde bei Raumtemperatur nachgerührt.

Man setzt dann 40 ml Wasser zu und entfernt das Tetrahydrofuran im Vakuum. Die zurückbleibende Wasserphase wird zweimal mit 50 ml Essigsäureäthylester gewaschen. Unter Rühren wird dann zu der wässrigen Lösung langsam 2 n Salzsäure zugetropft bis pH 2,9, wobei die Temperatur unter 5°C gehalten wird. Das ausgefallene Produkt wird abgesaugt und im Exsiccator getrocknet. Zu dem Festprodukt wird eine Lösung von 700 mg Natriumhexonanoat in 25 ml Methanol gegeben und die entstehende Lösung mit Äther versetzt. Das ausgefallene Natriumsalz wird abgesaugt und im Vakuum getrocknet.
Ausbeute: 2,19 g Natriumsalz (71%).
IR-Spektrum: 1770, 1660, 1610, 1545 $cm^{-1}$.
NMR-Spektrum ($DMSO+CD_3OD$) Signale bei ppm: 1,55 (d, 6H), 4,05 (1H), 5,45 (q, 2H + s, 1H), 6,8 (d, 2H), 7,25 (d, 2H), 7,4 (m, 1H), 8,2 (m, 3H), 8,8 (s, 1H).

Beispiel 2

D-α-[3-(4-Hydroxy-2-{3'-pyridylamino}-5-pyrimidinyl)-ureido]-benzylpenicillin-Natrium

Dieses Penicillin wurde analog Beispiel II/1 hergestellt. Man geht aus von 2,25 g Ampicillin-Natriumsalz (0,006 Mol) sowie dem Umsetzungsprodukt von 1,22 g 5-Amino-4-hydroxy-2-(3'-pyridylamino)-pyrimidin mit 600 mg Phosgen und 0,82 ml Triäthylamin.
Ausbeute: 2,13 g Natriumsalz (57,5%);
IR-Spektrum: 1765, 1650, 1615, 1545 $cm^{-1}$;
NMR-Spektrum ($DMSO+CD_3OD$) Signale bei ppm: 1,55 (d, 6H), 4,05 (s, 1H), 5,50 (g, 2H + s, 1H), 7,4 (m, 6H), 8,2 (m, 3H), 8,8 (s, 1H).

Beispiel 3

D-α-[3-(4-Hydroxy-2-{6'-methoxy-3'-pyridylamino}-5-pyrimidinyl)-ureido]-p-hydroxy-benzylpenicillin-Natrium

Dieses Penicillin wurde analog Beispiel II/1 hergestellt. Man geht aus von 840 mg Amoxicillin-trihydrat (0,002 Mol) sowie dem Umsetzungsprodukt von 470 mg (0,002 Mol) 5-Amino-4-hydroxy-2-(6-methoxy-3'-pyridylamino)-pyrimidin mit 200 mg Phosgen und 0,27 ml Triäthylamin.
Ausbeute: 630 mg Natriumsalz (48%);
IR-Spektrum: 1770, 1650, 1615, 1550 $cm^{-1}$;
NMR-Spektrum ($CD_3OD$) Signale bei ppm: 1,6 (d, 6H), 3,9 (s, 3H), 4,2 (s, 1H), 5,4 (m, 3H), 6,8 (m, 3H), 7,2 (m, 2H), 7,7-8,3 (m, 3H).

Beispiel 4

D-α-[3-(4-Hydroxy-2-{2'-pyridylmethylamino}-5-pyrimidinyl)-ureido]-p-hydroxy-benzylpenicillin-Natrium

Dieses Penicillin wurde analog Beispiel II/1 hergestellt. Man geht aus von 4,2 g Amoxicillin-trihydrat (0,01 Mol) sowie dem Umsetzungsprodukt von 2,17 g 5-Amino-4-hydroxy-2-(2'-pyridylmethylamino)-pyrimidin (0,01 Mol) mit 1,05 g Phosgen und 1,35 ml Triäthylamin.
Ausbeute: 4,06 g Natriumsalz (64%);
IR-Spektrum: 1765, 1650, 1615, 1545 $cm^{-1}$;
NMR-Spektrum ($DMSO+SD_3OD$) Signale bei ppm: 1,55 (d, 6H), 3,9 (s, 1H), 4,6 (s, 2H), 5,45 (q, 2H + s, 1H), 6,85 (d, 2H), 7,35 (m, 4H), 7,85 (m, 1H), 8,2 (s, 1H), 8,7 (m, 1H).

Beispiel 5

D-α-[3-(4-Hydroxy-2-{3'-pyridylmethylamino}-5-pyrimidinyl)-ureido]-p-hydroxy-benzylpenicillin-Natrium

Dieses Penicillin wurde analog Beispiel II/1 hergestellt. Man geht aus von 2,1 g Amoxicillin-trihydrat (0,005 Mol) sowie dem Umsetzungsprodukt von 1,19 g (0,005 Mol) 5-Amino-4-hydroxy-2-(3'-pyridylmethylamino)-pyrimidin mit 500 mg Phosgen und 0,67 ml Triäthylamin.
Ausbeute: 2,45 g Natriumsalz (79%);
IR-Spektrum: 1765, 1640, 1610, 1560 $cm^{-1}$;
NMR-Spektrum ($DMSO+CD_3OD$) Signale bei ppm: 1,5 (d, 6H), 3,9 (s, 1H), 4,55 (s, 2H), 5,35 (m, 3H), 6,75 (d, 2H), 7,2 (m, 3H), 7,6 (m, 1H), 8,0 (s, 1H), 8,3 (m, 2H).

Beispiel 6

D-α-[3-(4-Hydroxy-2-{4'-pyridylmethylamino}-5-pyrimidinyl)-ureido]-p-hydroxy-benzylpenicillin-Natrium

Dieses Penicillin wurde analog Beispiel II/1 hergestellt. Man geht aus von 420 mg Amoxicillin-trihydrat (0,001 Mol) sowie dem Umsetzungsprodukt von 220 mg 5-Amino-4-hydroxy-2-(4'-pyridylmethylamino)-pyrimidin mit 100 mg Phosgen und 0,14 ml Triäthylamin.
Ausbeute: 310 mg Natriumsalz (50%);
IR-Spektrum: 1770, 1650, 1615, 1545 $cm^{-1}$;
NMR-Spektrum ($DMSO+CD_3OD$) Signale bei ppm: 1,5 (d, 6H), 3,9 (s, 1H), 4,5 (breites s, 2H), 5,4 (q, 2H + s, 1H), 6,7 (d, 2H), 7,2 (m, 4H), 8,0 (s, 1H), 8,4 (m, 2H).

Beispiel 7

D-α-[3-(4-Hydroxy-2-{5'-pyrimidinylamino}-5-pyrimidinyl)-ureido]-p-hydroxy-benzylpenicillin-Natrium

410 mg (0,002 Mol) 5-Amino-4-hydroxy-2-(5'-pyrimidinylamino)-pyrimidin werden mit 5 ml Trimethylsilyldiathylamin auf 80°C erwärmt. Es entsteht ein homogenes Gemisch, das im Hochvakuum zur Trockne eingeengt wird. Man löst in 40 ml trockenem Tetrahydrofuran und tropft diese Lösung unter Eiskühlung zu 200 mg Phosgen, das in 30 ml Tetrahydrofuran gelöst war. Die weitere Umsetzung erfolgt analog Beispiel II/1.
Ausbeute: 735 mg Natriumsalz (64%);
IR-Spektrum: 1765, 1655, 1615, 1550 $cm^{-1}$;

NMR-Spektrum ($DMSO+CD_3OD$) Signale bei ppm: 1,55 (d, 6H), 3,95 (s, 1H), 5,40 (q, 2H + s, 1H), 6,8 (d, 2H), 7,3 (d, 2H), 8,3 (s, 1H), 8,6 (breites s, 2H), 8,9 (s, 1H).

Analog wurden synthetisiert:

D-α-[3-(4-Hydroxy-2-{5'-pyrimidinylamino}-5-pyrimidyl)-ureido]-benzylpenicillin-Natrium

D-α-[3-(4-Hydroxy-2-{1',2',3',4'-tetrahydro-1',3'-dimethyl-2',4'-dioxo-5'-pyrimidinyl}-5-pyrimidinyl)-ureido]-p-hydroxybenzylpenicillin-Natrium

Beispiel 8

D-α-[3-(4-Hydroxy-2-{2'-amino-5'-pyrimidinylamino}-5-pyrimidinyl)-ureido]-p-hydroxy-benzylpenicillin-Natrium

660 mg (0,003 Mol) 5-Amino-4-hydroxy-2-(2'-amino-5'-pyrimidinylamino)-pyrimidin werden in 50 ml absolutem Tetrahydrofuran aufgeschlämmt und bei Raumtemperatur 2 Stunden lang mit 2 ml N,O-Bis-trimethylsilylacetamid gerührt. Die entstandene Lösung wird im Wasserstrahlvakuum und anschliessend im Hochvakuum bei 80°C sorgfältig getrocknet. Dann nimmt man erneut in 50 ml absolutem Tetrahydrofuran auf und tropft diese Lösung unter Eiskühlung zu einer Lösung von 300 mg Phosgen in 20 ml absolutem Tetrahydrofuran.

Die weitere Umsetzung mit 1,26 g Amoxicillin (0,003 Mol) erfolgt wie in den vorhergegangenen Beispielen angegeben.

Ausbeute: 645 mg Natriumsalz (33%);
IR-Spektrum: 1770, 1665, 1615, 1550 $cm^{-1}$;
NMR-Spektrum ($DMSO+CD_3OD$) Signale bei ppm: 1,55 (6H), 4,0 (1H), 5,40 (q, 2H + s, 1H), 6,75 (d. 2H), 7,3 (d, 2H), 8,25 (s, 1H), 8,50 (breites s, 2H).

Beispiel 9

D-α-[3-(4-Hydroxy-2-{5'-äthoxycarbonyl-2'-thienylamino}-5-pyrimidinyl)-ureido]-p-hydroxy-benzylpenicillin-Natrium

Dieses Penicillin wurde analog Beispiel II/1 hergestellt. Man geht aus von 4,2 g Amoxicillin-trihydrat (0,01 Mol) sowie dem Umsetzungsprodukt von 2,8 g 5-Amino-4-hydroxy-2-(5'-äthoxycarbonyl-2'-thienylamino)-pyrimidin mit 500 mg Phosgen und 0,68 ml Triäthylamin.

Ausbeute: 4,05 g Natriumsalz (59,5%);
IR-Spektrum: 1770, 1650, 1620, 1545 $cm^{-1}$;
NMR-Spektrum ($DMSO+CD_3OD$) Signale bei ppm: 1,3 (t, 3H), 1,55 (d, 6H), 4,0 (s, 1H), 4,3 (q, 2H), 5,4 (q, 2H + s, 1H), 6,8 (s, 2H), 7,3 (d, 2H), 7,9 (m, 2H), 8,3 (s, 1H).

Beispiel 10

D-α-[3-(4-Hydroxy-2-{2'-thienylmethylamino}-5-pyrimidinyl)-ureido]-p-hydroxy-benzylpenicillin-Natrium

Dieses Penicillin wurde analog Beispiel II/1 hergestellt. Man geht aus von 8,4 g Amoxicillin-trihydrat (0,02 Mol) sowie dem Umsetzungsprodukt von 4,48 g (0,02 Mol) 5-Amino-4-hydroxy-2-(2'-thienylmethylamino)-pyrimidin mit 2,0 g Phosgen und 2,75 ml Triäthylamin.

Ausbeute: 9,75 g Natriumsalz (76%);
IR-Spektrum: 1770, 1660, 1620, 1560 $cm^{-1}$;
NMR-Spektrum ($DMSO+CD_3OD$) Signale bei ppm: 1,55 (d, 6H), 4,0 (s, 1H), 4,5 (s, 2H), 5,35 (q, 2 + s, 1H), 6,7 (d, 2H), 6,85 (m, 2H), 7,25 (d, 2H), 7,35 (m, 1H), 8,1 (s, 1H).

Beispiel 11

D-α-[3-(4-Hydroxy-2-{2'-thienylmethylamino}-5-pyrimidinyl)-ureido]-benzylpenicillin-Natrium

Dieses Penicillin wurde analog Beispiel II/1 hergestellt. Man geht aus von 1,86 g Ampicillin-Natriumsalz (0,005 Mol) sowie dem Umsetzungsprodukt von 1,12 g 5-Amino-4-hydroxy-2-(2'-thienylmethylamino)-pyrimidin mit 500 mg Phosgen und 0,68 ml Triäthylamin.

Ausbeute: 2,20 g Natriumsalz (70,5%);
IR-Spektrum: 1765, 1655, 1610, 1545 $cm^{-1}$;
NMR-Spektrum ($DMSO+CD_3OD$) Signale bei ppm: 1,55 (6H), 4,05 (s, 1H), 4,5 (s, 2H), 5,4 (q, 2 + s, 1H), 6,85 (m, 2H), 7,4 (m, 6H), 8,05 (s, 1H).

Analog wurden synthetisiert:

D-α-[3-(4-Hydroxy-2-{2'-thienylmethylamino}-5-pyrimidinyl)-ureido]-m,p-dihydroxy-benzylpenicillin-Natrium

D-α-[3-(4-Hydroxy-2-{2'-thienylmethylamino}-5-pyrimidinyl)-ureido]-cyclohexa-1,4-dien-1-yl-methylpenicillin-Natrium

D-α-[3-(4-Hydroxy-2-{2'-thienylmethylamino}-5-pyrimidinyl)-ureido]-2-thienylmethylpenicillin-Natrium

D-α-[3-(4-Hydroxy-2-{2'-thienylmethylamino}-5-pyrimidinyl)-ureido]-2-furylmethylpenicillin-Natrium

Beispiel 12

D-α-[3-(4-Hydroxy-2-{3'-thienylmethylamino}-5-pyrimidinyl)-ureido]-p-hydroxy-benzylpenicillin-Natrium

Dieses Penicillin wurde analog Beispiel II/1 hergestellt. Man geht aus von 840 g Amoxicillin-trihydrat (0,002 Mol) sowie dem Umsetzungsprodukt von 460 mg 5-Amino-4-hydroxy-2-(3'-thienylmethylamino)-pyrimidin mit 200 mg Phosgen und 0,27 ml Triäthylamin.

Ausbeute: 860 mg Natriumsalz (66%);
IR-Spektrum: 1765, 1650, 1610, 1545 $cm^{-1}$.

Beispiel 13

D-α-[3-(4-Hydroxy-2-{5'-methyl-2'-thienylmethylamino}-5-pyrimidinyl)-ureido]-p-hydroxy-benzylpenicillin-Natrium

Dieses Penicillin wurde analog Beispiel II/1 hergestellt. Man geht aus von 1,26 g Amoxicillin--trihydrat (0,003 Mol) sowie dem Umsetzungsprodukt von 700 mg (0,003 Mol) 5-Amino-4-hydroxy-2-(5'-methyl-2'-thienylmethylamino)-pyrimidin mit 300 mg Phosgen und 0,41 ml Triäthylamin.
Ausbeute: 380 mg Natriumsalz (54,5%);
IR-Spektrum: 1770, 1645, 1610, 1550 $cm^{-1}$;
NMR-Spektrum ($DMSO+CD_3OD$) Signale bei ppm: 1,55 (6H), 2,45 (s, 3H), 4,0 (s, 1H), 4,5 (s, 2H), 5,4 (m, 3H), 6,7 (d, 2H), 6,85 (2H), 7,20 (d, 2H), 8,05 (s, 1H).

Analog wurden synthetisiert:
D-α-[3-(4-Hydroxy-2-{5'-methyl-2'-thienylmethylamino}-5-pyrimidinyl)-ureido]-benzylpenicillin-Natrium
D-α-[3-(4-Hydroxy-2-{5'-methyl-2'-thienylmethylamino}-5-pyrimidinyl)-ureido]-cycohexa-1,4-dien--1-yl-methylpenicillin-Natrium
D-α-[3-(4-Hydroxy-2-{5'-methyl-2'-thienylmethylamino}-5-pyrimidinyl)-ureido]-2-furylmethyl-penicillin-Natrium.

Beispiel 14

D-α-[3-(4-Hydroxy-2-{5'-chlor-2'-thienylmethylamino}-5-pyrimidinyl)-ureido]-p-hydroxy-benzylpenicillin-Natrium

Dieses Penicillin wurde analog Beispiel II/1 hergestellt. Man geht aus von 1,0 g Amoxicillin--trihydrat (0,0024 Mol) sowie dem Umsetzungsprodukt von 700 mg 5-Amino-4-hydroxy-2-(5'--chlor-2'-thienylmethylamino)-pyrimidin mit 250 mg Phosgen und 0,33 ml Triäthylamin.
Ausbeute: 1,1 g Natriumsalz (67%);
IR-Spektrum: 1765, 1655, 1615, 1550 $cm^{-1}$;
NMR-Spektrum ($DMSO+CD_3OD$) Signale bei ppm: 1,55 (6H), 4.0 (1H), 4,45 (s, 2H), 5,35 (q, 2H + s, 1H), 6,7-7,0 (m, 4H), 7,25 (d, 2H), 8,10 (s, 1H).

Beispiel 15

D-α-[3-(4-Hydroxy-2-{2'-furylmethylamino}-5--pyrimidinyl)-ureido]-p-hydroxy-benzylpenicillin-Natrium

Dieses Penicillin wurde analog Beispiel II/1 hergestellt. Man geht aus von 6,3 g Amoxicillin--trihydrat (0,015 Mol) sowie dem Umsetzungsprodukt von 3,1 g (0,015 Mol) 5-Amino-4-hydroxy--2-(2'-furylmethylamino)-pyrimidin mit 1,5 g Phosgen und 2,0 ml Triäthylamin.
Ausbeute: 6,45 g Natriumsalz (69%);
IR-Spektrum: 1770, 1660, 1620, 1550 $cm^{-1}$;
NMR-Spektrum ($DMSO+CD_3OD$) Signale bei ppm: 1,5 (d. 6H), 3,9 (s, 1H), 4,4 (breites s, 2H), 5,4 (q. 2H + s, 1H), 6,3 (m, 2H), 6,7 (d, 2H), 7,2 (d, 2H), 7,5 (s, 1H), 8,0 (s, 1H).

Beispiel 16

D-α-[3-(4-Hvdroxy-2-{2'-furylmethylamino}-5--pyrimidinyl)-ureido]-benzylpenicillin-Natrium

Dieses Penicillin wurde analog Beispiel II/1 hergestellt. Man geht aus von 1,86 g Ampicillin-Natriumsalz (0,005 Mol) sowie dem Umsetzungsprodukt von 1,06 g 5-Amino-4-hydroxy-2-(2'-furylmethylamino)-pyrimidin mit 500 mg Phosgen und 0,68 ml Triäthylamin.
Ausbeute: 1,91 g Natriumsalz (62%);
NMR-Spektrum ($DMSO+CD_3OD$) Signale bei ppm: 1,55 (d, 6H), 4,0 (s, 1H), 4,4 (breites s, 2H), 5,4 (q, 2H + s, 1H), 6,3 (m, 2H), 7,45 (m, 6H), 8,05 (s, 1H).

Analog wurden hergestellt:
D-α-[3-(4-Hydroxy-2-{2'-furylmethylamino}-5-pyrimidinyl)-ureido]-m,p-dihydroxy-benzylpenicillin-Natrium
D-α-[3-(4-Hydroxy-2-{2'-furylmethylamino}-5-pyrimidinyl)-ureido]-cyclohexa-1,4-dien-1-yl-methylpenicillin-Natrium
D-α-[3-(4-Hydroxy-2-{2'-furylmethylamino}-5-pyrimidinyl)-ureido]-2-furylmethyl-penicillin-Natrium
D-α-[3-(4-Hydroxy-2-{2'-furylmethylamino}-5-pyrimidinyl)-ureido]-3-furylmethyl-penicillin-Natrium
D-α-[3-4-Hydroxy-2-{2'-furylmethylamino}-5-pyrimidinyl)-ureido]-2-thienylmethyl-penicillin-Natrium
D-α-[3-(4-Hydroxy-2-{2'-furylmethylamino}-5-pyrimidinyl)-ureido]-3-thienylmethyl-penicillin-Natrium

Beispiel 17

D-α-[3-(4-Hydroxy-2-{5'-methyl-2'-thienylamino}--5-pyrimidinyl)-ureido]-p-hydroxy-benzylpenicillin-Natrium

Dieses Penicillin wurde analog Beispiel II/1 hergestellt. Man geht aus von 500 mg Amoxicillin-trihydrat (0,0012 Mol) sowie dem Umsetzungsprodukt von 270 mg 5-Amino-4-hydroxy-2-(5'-methyl-2'-thienylamino)-pyrimidin mit 120 mg Phosgen und 0,17 ml Triäthylamin.
Ausbeute: 350 mg Natriumsalz (47%);
IR-Spektrum: 1765, 1655, 1610, 1550 $cm^{-1}$;
NMR-Spektrum ($DMSO+CD_3OD$) Signale bei ppm: 1,55 (6H), 2,5 (s, 3H), 4,0 (s, 1H), 5,35 (q, 2H + s, 1H), 6,5 (m, 2H), 6,75 (d, 2H), 7,2 (d, 2H), 8,1 (s, 1H).

Beispiel 18

D-α-[3-(4-Hydroxy-2-{5'-methyl-2'-furylmethylamino}-5-pyrimidinyl)-ureido]-p-hydroxy-benzylpenicillin-Natrium

Dieses Penicillin wurde analog Beispiel II/1 hergestellt. Man geht aus von 840 mg Amoxicillin-trihydrat (0,002 Mol) sowie dem Umsetzungsprodukt von 440 mg (0,002 Mol) 5-Amino-4-hydroxy-2-(5'-methyl-2'-furylmethylamino-pyrimidin

mit 200 mg Phosgen und 0,27 ml Triäthylamin.
Ausbeute: 620 mg Natriumsalz (48%);
IR-Spektrum: 1770, 1655, 1620, 1550 $cm^{-1}$;
NMR-Spektrum ($DMSO+CD_3OD$) Signale bei ppm: 1,55 (d, 6H), 2,45 (s, 3H), 4,0 (s, 1H), 5,40 (q, 2H + s, 1H), 6,3 (m, 2H), 6,75 (d, 2H), 7,2 (d, 2H), 8,05 (s, 1H).

Beispiel 19

D-α-[3-(4-Hydroxy-2-{tetrahydro-2'-furylmethylamino}-5-pyrimidinyl)-ureido]-p-hydroxy-benzylpenicillin-Natrium

Dieses Penicillin wurde analog Beispiel II/1 hergestellt. Man geht aus von 4,2 g Amoxicillin-trihydrat (0,01 Mol) sowie dem Umsetzungsprodukt von 2,1 g 5-Amino-4-hydroxy-2-(tetrahydro-2'-furylmethylamino)-pyrimidin mit 1,0 g Phosgen und 1,35 ml Triäthylamin.
Ausbeute: 3,62 g Natriumsalz (58%);
IR-Spektrum: 1765, 1650, 1615, 1545 $cm^{-1}$;
NMR-Spektrum ($DMSO+CD_3OD$) Signale bei ppm: 1,55 (d, 6H), 1,9 (m, 4H), 3,5-4,0 (m, 6H), 5,4 (q, 2H + s, 1H), 6,7 (d, 2H), 7,2 (d, 2H), 8,0 (s, 1H).

Beispiel 20

D-α-[3-(4-Hydroxy-2-{2'-pyrrolylmethylamino}-5-pyrimidinyl)-ureido]-p-hydroxy-benzylpenicillin-Natrium

Dieses Penicillin wurde analog Beispiel II/1 hergestellt. Man geht aus von 1,0 g Amoxicillin-trihydrat (0,0024 Mol) sowie dem Umsetzungsprodukt von 490 mg (0,0024 Mol) 5-Amino-4-hydroxy-2-(2'-pyrrolylmethylamino)-pyrimidin mit 250 mg Phosgen und 0,33 ml Triäthylamin.
Ausbeute: 635 mg Natriumsalz (42,5%);
IR-Spektrum: 1770, 1650, 1615, 1550 $cm^{-1}$;
NMR-Spektrum ($DMSO+CD_3OD$) Signale bei ppm: 1,55 (d, 6H), 3,95 (s, 1H), 4,3 (s, 2H), 5,4 (m, 3H), 6,1 (m, 2H), 6,7 (m, 3H), 7,3 (d, 2H), 8,05 (s, 1H).

Beispiel 21

D-α-[3-(4-Hydroxy-2-{2'-thiazolylamino}-5-pyrimidinyl)-ureido]-p-hydroxy-benzylpenicillin-Natrium

Dieses Penicillin wurde analog Beispiel II/7 hergestellt. Man geht aus von 2,1 g Amoxicillin-trihydrat (0,005 Mol) sowie von 1,05 g 5-Amino-4-hydroxy-2-(2'-thiazolylamino)-pyrimidin das nach Silylierung mit 500 mg Phosgen und 0,68 ml Triäthylamin umgesetzt wurde.
Ausbeute: 1,12 g Natriumsalz (36%);
IR-Spektrum: 1765, 1650, 1610, 1540 $cm^{-1}$;
NMR-Spektrum ($DMSO+CD_3OD$) Signale bei ppm: 1,55 (6H), 3,95 (s, 1H), 5,40 (q, 2H + s, 1H), 6,7 (m, 4H), 7,25 (d, 2H), 8,0 (s, 1H).

Beispiel 22

D-α-[3-(4-Hydroxy-2-{4'-methyl-2'-thiazolylmethylamino}-5-pyrimidinyl)-ureido]-p-hydroxy-benzylpenicillin-Natrium

Dieses Penicillin wurde analog Beispiel II/1 hergestellt. Man geht aus von 2,0 g Amoxicillin-trihydrat (0,0048 Mol) sowie dem Umsetzungsprodukt von 1,75 g 5-Amino-4-hydroxy-2-(4'-methyl-2'-thiazolylmethylamino)-pyrimidin mit 500 mg Phosgen und 0,65 ml Triäthylamin.
Ausbeute: 1,30 g Natriumsalz (41%);
IR-Spektrum: 1765, 1650, 1615, 1550 $cm^{-1}$;
NMR-Spektrum ($DMSO+CD_3OD$) Signale bei ppm: 1,55 (d, 6H), 2,25 (s, 3H), 4,0 (s, 1H), 4,4 (breites s, 2H), 6,6 (s, 1H), 6,75 (d, 2H), 7,2 (d, 2H), 8,0 (s, 1H).

Beispiel 23

D-α-[3-(4-Hydroxy-2-{4'-methyl-2'-imidazolylmethylamino}-5-pyrimidinyl)-ureido]-p-hydroxy-benzylpenicillin-Natrium

Dieses Penicillin wurde analog Beispiel II/7 hergestellt. Man geht aus von 1,5 g Amoxicillin-trihydrat (0,0035 Mol) sowie dem Umsetzungsprodukt von 730 mg 5-Amino-4-hydroxy-2-(2'-imidazolylmethylamino)-pyrimidin mit Trimethylsilyldiäthylamin und 350 mg Phosgen.
Ausbeute: 1,18 g Natriumsalz (54%);
IR-Spektrum: 1765, 1655, 1615, 1545 $cm^{-1}$;
NMR-Spektrum ($DMSO+CD_3OD$) Signale bei ppm: 1,55 (d, 6H), 2,1 (s, 3H), 4,0 (s, 1H), 4,4 (s, 2H), 5,40 (m, 3H), 6,8 (d, 2H), 7,2 (m, 4H), 8,05 (s, 1H).

Analog lässt sich synthetisieren:
D-α-[3-(4-Hydroxy-2-{2'-oxazolylmethylamino}-5-pyrimidinyl)-ureido]-p-hydroxy-benzylpenicillin-Natrium

Beispiel 24

D-α-[3-(4-Hydroxy-2-{5'-methyl-1',3',4'-triazol-2'-ylmethylamino}-5-pyrimidinyl)-ureido]-p-hydroxy-benzylpenicillin-Natrium

Dieses Penicillin wurde analog Beispiel II/7 hergestellt. Man geht aus von 840 mg Amoxicillin-trihydrat (0,002 Mol) sowie dem Umsetzungsprodukt von 440 mg (0,002 Mol) 5-Amino-4-hydroxy-2-(5'-methyl-1',3',4'-triazol-2'-ylmethylamino-pyrimidin mit 200 mg Phosgen.
Ausbeute: 615 mg Natriumsalz (47,5%);
IR-Spektrum: 1770, 1660, 1620, 1550 $cm^{-1}$;
NMR-Spektrum ($DMSO+CD_3OD$) Signale bei ppm: 1,55 (d, 6H), 2,15 (s, 3H), 3,95 (s, 1H), 4,4 (breites s, 2H), 5,45 (m, 3H), 6,8 (d, 2H), 7,25 (d, 2H), 8,1 (s, 1H).

Beispiel 25

D-α-[3-(4-Hydroxy-2-{5'-methyl-1',3',4'-thiadiazol--2'-ylamino}-5-pyrimidinyl)-ureido]-p-hydroxy--benzylpenicillin-Natrium

Dieses Penicillin wurde analog Beispiel II/7 hergestellt. Man geht aus von 4,2 g Amoxicillin--trihydrat (0,01 Mol) sowie von 2,24 g 5-Amino--4-hydroxy-2-(5'-methyl-1',3',4'-thiadiazol-2'-yl--amino)-pyrimidin das nach Silylierung mit 1,0 g Phosgen umgesetzt wurde.
Ausbeute: 2,4 g Natriumsalz (33,5%);
IR-Spektrum: 1765, 1655, 1615, 1545 $cm^{-1}$;
NMR-Spektrum (DMSO+$CD_3OD$) Signale bei ppm: 1,55 (d, 6H), 2,4 (s, 3H), 4,0 (s, 1H), 5,4 (q, 2H + s, 1H), 6,75 (d, 2H), 7,25 (d, 2H), 8,1 (s, 1H).
Analog wurde synthetisiert:
D-α-[3-(4-Hydroxy-2-{5'-methyl-1',3',4'-triazol-2'--ylamino}-ureido]-p-hydroxy-benzylpenicillin-Natrium

Beispiel 26

D-α-[3-(4-Hydroxy-2-{4'-pyrimidinylmethylamino}-5-pyrimidinyl)-ureido]-p-hydroxy-benzylpenicillin-Natrium

Dieses Penicillin wurde analog Beispiel II/7 hergestellt. Man geht aus von 840 mg Amoxycillin-trihydrat (0,002 Mol) sowie von 420 mg 5-Amino-4-hydroxy-2-(4'-pyrimidinylmethylamino)-pyrimidin.
Ausbeute: 780 mg (62%);
IR-Spektrum: 1770, 1650, 1610, 1555 $cm^{-1}$;
NMR-Spektrum (DMSO+$CD_3OD$) Signale bei ppm: 1,5 (d, 6H), 3,9 (s, 1H), 4,5 (breites s, 2H), 5,35 (q, 2H + d, 1H), 6,7 (d, 2H), 7,2 (d, 2H), 7,4 (d, 1H), 8,0 (s, 1H), 8,7 (d, 2H), 9,0 (s, 1H).

Beispiele 27

D-α-[3-(4-Hydroxy-2-{5'-aminosulfonyl-2'-thienylmethylamino}-5-pyrimidinyl)-ureido]-p-hydroxy--benzylpenicillin-Natrium

Dieses Penicillin wurde analog Beispiel II/7 hergestellt. Man geht aus von 766 mg Amoxicillin--trihydrat (0,00183 Mol) sowie dem Umsetzungsprodukt von 500 mg (0,00166 Mol) 5-Amino-4--hydroxy-2-(2'-thienylmethylamino)-pyrimidin mit 5 ml N,N-Diäthyl-trimethylsilylamin und 164 mg (0,00166 Mol) Phosgen.
Ausbeute: 185 mg Natriumsalz (16%);
IR-Spektrum: 1600, 1660, 1765 $cm^{-1}$;
NMR-Spektrum ($CD_3OD$) Signale bei ppm: 1,5 (d, 6H), 4,1 (s, 1H), 4,6 (breit, s, 2H), 5,4 (d, 3H), 6,7 (d, 2H), 6,9 (d, 1H), 7,2 (d, 2H), 7,4 (d, 1H), 8,05 (s, 1H).

Beispiel 28

D-α-[3-(4-Hydroxy-2-{2'-methyl-5'-pyrimidinylmethylamino}-5-pyrimidinyl)-ureido]-p-hydroxy--benzylpenicillin-Natrium

Dieses Penicillin wurde analog Beispiel II/7 hergestellt. Man geht aus von 990 mg (0,00235 Mol) Amoxicillin-trihydrat sowie dem Umsetzungsprodukt von 500 mg (0,00215 Mol) 5-Amino-4-hydroxy-2-(2'-methyl-5'-pyrimidinylmethylamino)-pyrimidin mit 5 ml N,N-Diäthyl-trimethylsilylamin und 213 mg Phosgen.
Ausbeute: 265 mg Natriumsalz (19%);
IR-Spektrum: 1765, 1660 $cm^{-1}$;
NMR-Spektrum (DMSO/$CD_3OD$) Signale bei ppm: 1,5 (d, 6H), 2,5 (s, 3H), 3,95 (s, 1H), 4,4 (s, 2H), 5,4 (m, 3H), 6,7 (d, 2H), 7,15 (d, 2H), 8,05 (s, 1H), 8,6 (s, 2H).

Beispiel 29

D-α-[3-(4-Hydroxy-2-{2'-pyridylamino}-5-pyrimidinyl)-ureido]-p-hydroxy-benzylpenicillin-Natrium

Dieses Penicillin wurde analog Beispiel II/7 hergestellt. Man geht aus von 1,85 g (0,0044 Mol) Amoxicillin-trihydrat sowie dem Umsetzungsprodukt von 0,81 g (0,004 Mol) 5-Amino-4-hydroxy--2-(2'-pyridylamino)-pyrimidin mit 10 ml N,N-Diäthyltrimethylsilylamin, 396 mg Phosgen und 0,56 ml Triäthylamin.
Ausbeute: 0,9 g Natriumsalz (37%);
IR-Spektrum: 1765, 1660, 1600 $cm^{-1}$;
NMR-Spektrum (DMSO/$CD_3OD$) Signale bei ppm: 1,5 (d, 6H), 4,0 (s, 1H), 5,4 (m, 3H), 6,8 (d, 2H), 7,2 (m, 4H), 7,7 (m, 2H), 8,3 (m, 2H).

Beispiel 30

D-α-[3-(4-Hydroxy-2-{6'-hydroxy-3'-pyridylamino}-5-pyrimidinyl)-ureido]-p-hydroxy-benzylpenicillin-Natrium

Dieses Penicillin wurde analog Beispiel II/7 hergestellt. Man geht aus von 0,879 g (0,0021 Mol) Amoxicillin-trihydrat sowie dem Umsetzungsprodukt von 438 mg (0,002 Mol) 5-Amino-4--hydroxy-2-(6'-hydroxy-3'-pyridylamino)-pyrimidin mit 12 ml N,N-Diäthyltrimethylsilylamin und 216 mg Phosgen.
Ausbeute: 0,311 g Natriumsalz (25%);
IR-Spektrum: 1765, 1665, 1610 $cm^{-1}$;
NMR-Spektrum (DMSO/$CD_3OD$) Signale bei ppm: 1,5 (d, 6H), 4,1 (s, 1H), 5,4 (breit, m, 3H), 6,4 (d, 1H), (d, 2H), 7,25 (d, 2H), 7,6 (d, 1H), 7,9 (s, 1H), 8,15 (s, 1H).

Beispiel 31

D-α-[3-(4-Hydroxy-2-{6'-hydroxy-2'-pyridylamino}-5-pyrimidinyl)-ureido]-p-hydroxy-benzylpenicillin-Natrium

Dieses Penicillin wurde analog Beispiel II/7 hergestellt. Man geht aus von 420 mg Amoxicillin-trihydrat (0,001 Mol) sowie dem Umsetzungsprodukt von 220 mg (0,001 Mol) 5-Amino-4-hydroxy-2-(6'-hydroxy-2'-pyridylamino)-pyrimidin mit 2 ml N,N-Diäthyltrimethylsilylamin und 100 mg Phosgen.

Ausbeute: 264 mg Natriumsalz (42%); IR-Spektrum: 1765, 1660, 1610, 1020 $cm^{-1}$; NMR-Spektrum (DMSO/$CD_3OD$) Signale bei ppm: 1,5 (d, 6H), 4,05 (s, 1H), 5,35 (q, 2H), 5,45 (s, 1H), 6,45 (d, 1H), 6,75 (d, 2H), 7,25 (d, 2H), 7,6 (d, 1H), 7,9 (s, 1H), 8,20 (s, 1H).

Beispiel 32

D-α-[3-(4-Hydroxy-2-{2'-hydroxy-5'-pyrimidinyl-amino}-5-pyrimidinyl)-ureido]-p-hydroxy-benzylpenicillin-Natrium

Dieses Penicillin wurde analog Beispiel II/7 hergestellt. Man geht aus von 800 mg Amoxicillin-trihydrat (0,0019 Mol) sowie dem Umsetzungsprodukt von 420 mg (0,0019 Mol) 5-Amino-4-hydroxy-2-(2'-hydroxy-5'-pyrimidinylamino)-pyrimidin mit 4 ml N,N-Diäthyl-trimethylsilylamin und 190 mg Phosgen.
Ausbeute: 550 mg Natriumsalz (46%); IR-Spektrum: 1770, 1655, 1610 $cm^{-1}$; NMR-Spektrum (DMSO/$CD_3OD$) Signale bei ppm: 1,55 (d, 6H), 4,1 (s, 1H), 5,45 (m, 3H), 6,75 (d, 2H), 7,25 (d, 2H), 8,35 (s, 1H), 8,75 (s, 2H).

Beispiel 33

D-α-[3-(4-Hydroxy-2-{6'-methylsulfinyl-3'-pyridyl-amino}-5-pyrimidinyl)-ureido]-p-hydroxy-benzylpenicillin-Natrium

Dieses Penicillin wurde analog Beispiel II/7 hergestellt. Man geht aus von 1 g (0,0024 Mol) Amoxicillin-trihydrat sowie dem Umsetzungsprodukt von 580 mg (0,00218 Mol) 5-Amino-4-hydroxy-2-(6'-methylsulfinyl-3'-pyridylamino)-pyrimidin mit 10 ml N,N-Diäthyltrimethylsilylamin und 216 mg Phosgen.
Ausbeute: 0,39 g Natriumsalz (27%); IR-Spektrum: 1770, 1650, 1020 $cm^{-1}$; NMR-Spektrum (DMSO/$CD_3OD$) Signale bei ppm: 1,5 (d, 6H), 2,7 (s, 3H), 4,0 (s, 1H), 5,4 (m, 3H), 6,65 (d, 2H), 7,2 (d, 2H), 7,7 (d, 1H), 8,25 (s, 1H), 8,5 (m, 1H), 8,9 (s, 1H).

Beispiel 34

D-α-[3-(4-Hydroxy-2-{6'-methylsulfonyl-3'-pyridylamino}-5-pyrimidinyl)-ureido]-p-hydroxy-benzylpenicillin-Natrium

Dieses Penicillin wurde analog Beispiel II/7 hergestellt. Man geht aus von 570 mg (0,00136 Mol) Amoxicillin-trihydrat sowie dem Umsetzungsprodukt von 350 mg (0,00124 Mol) 5-Amino-4-hydroxy-2-(6'-methylsulfonyl-3'-pyridylamino)-pyrimidin mit 5 ml N,N-Diäthyltrimethylsilylamin und 123 mg Phosgen.
Ausbeute: 300 mg Natriumsalz (35%); IR-Spektrum: 1765, 1660, 1380, 1155 $cm^{-1}$; NMR-Spektrum ($CD_3OD$) Signale bei ppm: 1,5 (d, 6H), 3,1 (s, 3H), 4,0 (s, 1H), 5,4 (m, 3H), 6,65 (d, 2H), 7,2 (d, 2H), 7,9 (d, 1H), 8,3 (s, 1H), 8,6 (d, 1H), 8,9 (s, 1H).

Beispiel 35

D-α-[3-(2-{5'-Aminocarbonyl-2'-thienylamino}-4-hydroxy-5-pyrimidinyl)-ureido]-p-hydroxy-benzylpenicillin-Natrium

Herstellung analog Beispiel II/7, ausgehend von 840 mg Amoxycillin-trihydrat (0,001 Mol) und dem Unmsetzungsprodukt von 250 mg des Pyrimidins des Beispiels I, 1ao (0,001 Mol).
Ausbeute: 340 mg Natriumsalz (51%); IR-Spektrum: 1765, 1655, 1610, 1140 $cm^{-1}$; NMR-Spektrum (DMSO+$CD_3OD$) Signale bei ppm: 1,55 (d, 6H), 4,05 (s, 1H), 5,45 (m, 3H), 6,6 (d, 1H), 6,75 (d, 2H), 7,25 (d, 2H), 7,45 (d, 1H), 8,35 (s, 1H).

Beispiel 36

D-α-[3-(4-Hydroxy-2-{5'-tetrazolylmethylamino}-5-pyrimidinyl)-ureido]-p-hydroxy-benzylpenicillin-Natrium

Herstellung analog Beispiel II/7 mit dem Pyrimidin des Beispiels I, 1ap.
Ausbeute: 46%; IR-Spektrum: 1765, 1650, 1605 $cm^{-1}$; NMR-Spektrum (DMSO+$CD_3OD$) Signale bei ppm: 1,55 (d, 6H), 4,05 (s, 1H), 4,45 (s, 2H), 5,40 (q, 2H), 5,50 (s, 1H), 6,75 (d, 2H), 7,25 (d, 2H), 8,10 (s, 1H).

Beispiel 37

D-α-[3-(4-Hydroxy-2-{2',6'-dihydroxy-4'-pyrimidinylamino}-5-pyrimidinyl)-ureido]-p-hydroxy-benzylpenicillin-Natrium

Synthese analog Beispiel II/7 mit dem Pyrimidin des Beispiels I, 1as und Amoxycillin.
Ausbeute: 54,5%; IR-Spektrum: 1765, 1660, 1605 $cm^{-1}$; NMR-Spektrum (DMSO+$CD_3OD$) Signale bei ppm: 1,55 (d, 6H), 4,0 (s, 1H), 5,45 (q, 2H + s, 1H), 6,75 (d, 2H), 7,3 (d, 2H), 8,35 (s, 1H), 8,55 (s, 1H).

Beispiel 38

D-α-[3-(4-Hydroxy-2-{2',4'-dihydroxy-5'-pyrimidinylamino}-5-pyrimidinyl)-ureido]-p-hydroxy-benzylpenicillin-Natrium

Synthese analog Beispiel II/7 mit dem Pyrimidin des Beispiels I, 1aq und Amoxycillin.
Ausbeute: 39% Natriumsalz; IR-Spektrum: 1765, 1655, 1600 $cm^{-1}$; NMR-Spektrum (DMSO+$CD_3OD$) Signale bei ppm: 1,55 (d, 6H), 4,10 (s, 1H), 5,40 (q, 2H), 5,50 (s, 1H), 6,70 (d, 2H), 7,25 (d, 2H), 8,35 (s, 1H), 8,70 (s, 1H).

Beispiel 39

D-α-[3-(4-Hydroxy-2-{4',6'-dihydroxy-2'-pyrimidi-

nylamino}-5-pyrimidinyl)-ureido]-p-hydroxy-benzylpenicillin-Natrium

Synthese analog Beispiel II/7, ausgehend von Amoxycillin und dem Aminopyrimidin des Beispiels I, 1ar.
Ausbeute: 42,5%;
IR-Spektrum: 1765, 1660, 1610 $cm^{-1}$;
NMR-Spektrum (DMSO+$CD_3OD$) Signale bei ppm: 1,55 (d, 6H), 4,05 (s, 1H), 5,45 (m, 3H), 6,70 (d, 2H), 7,25 (d, 2H), 8,30 (s, 1H), 8,85 (s, 1H).

Beispiel 40

6-{D-α-[3-(4-Hydroxy-2-(2'-thienylmethylamino)-5-pyrimidinyl)-ureido]-p-hydroxy-phenylacetamido}-penicillansäure-pivaloyloxymethylester

1,28 g (0,002 Mol) der Verbindung des Beispiels II/10 werden in 10 ml Aceton suspendiert. Dazu gibt man eine Lösung von 340 mg Pivaloyloxymethylchlorid (0,0022 Mol) in 10 ml Aceton, sowie 0,2 ml einer 25%igen Natriumjodidlösung in Wasser. Man erhitzt 5 Stunden zum Rückfluss, kühlt ab und versetzt mit 15 ml Eiswasser. Es wird abdekantiert und das ausgefallene etwas schmierige Produkt erneut mit 10 ml Eiswasser versetzt und 5 Min. gerührt. Danach wird abgesaugt, das farblose Produkt in Essigester gelöst, mit Wasser, Natriumhydrogencarbonat-Lösung und wieder mit Wasser gewaschen und anschliessend im Vakuum getrocknet.
Ausbeute: 950 mg (66%);
IR-Spektrum: 1770, 1720, 1650 $cm^{-1}$;
NMR-Spektrum ($CDCl_3$+$CD_3OD$) Signale bei ppm: 1,2 (s, 9H), 1,55 (d, 6H), 4,2 (s, 1H), 4,4 (s, 2H), 5,45 (q, 2H), 5,5 (s, 1H), 5,65 (q, 2H), 6,7 (d, 2H), 6,85 (m, 2H), 7,30 (d, 2H), 7,40 (m, 1H), 8,1 (s, 1H).

Analog wurde synthetisiert:
6-{D-α-[3-(4-Hydroxy-2-(2'-thienylmethylamino)-5-pyrimidinyl)-ureido]-p-hydroxy-phenylacetamido}-penicillansäure-1'-propionyloxy-äthylester
6-{D-α-[3-(4-Hydroxy-2-(2'-thienylmethylamino)-5-pyrimidinyl)-ureido]-p-hydroxy-phenylacetamido}-penicillansäure-1'-acetoxyäthylester
6-{D-α-[3-(4-Hydroxy-2-(5'-chlor-2'-thienylmethylamino)-5-pyrimidinyl)-ureido]-p-hydroxy-phenylacetamido}-penicillansäurepivaloyloxymethylester

Beispiel 41

6-{D-α-[3-(4-Hydroxy-2-(2'-furylmethylamino)-5-pyrimidinyl)-ureido]-p-hydroxy-phenylacetamido}-penicillansäure-pivaloyloxymethylester

Synthese analog Beispiel II/39, ausgehend von 625 mg (0,001 Mol) der Verbindung des Beispiels II/15 und 170 mg Pivaloyloxymethylchlorid.
Ausbeute: 420 mg (69%);
IR-Spektrum: 1770, 1715, 1650 $cm^{-1}$;
NMR-Spektrum ($CDCl_3$+$CD_3OD$) Signale bei ppm: 1,2 (s, 9H), 1,55 (d, 6H), 4,3 (s, 1H), 4,5 (breites s, 2H), 5,4 (q, 2H + s, 1H), 5,65 (q, 2H), 6,3 (m, 2H), 6,7 (d, 2H), 7,2 (d, 2H), 7,45 (s, 1H), 8,1 (s, 1H).

Beispiel 42

6-{D-α-[3-‹4-Hydroxy-2-(5'-aminosulfonyl-2'-thienylmethylamino)-5-pyrimidinyl›-ureido]-p-hydroxy-phenylacetamido}-penicillansäure-pivaloyloxymethylester

Synthese analog Beispiel II, 39, ausgehend von dem Natriumsalz des Beispiels II, 27 und Pivaloyloxymethylchlorid.
Ausbeute: 74%;
IR-Spektrum: 1770, 1700, 1660 $cm^{-1}$;
NMR-Spektrum ($CDCl_3$+$CD_3OD$) Signale bei ppm: 1,2 (s, 9H), 1,55 (d, 6H), 4,25 (s, 1H), 4,6 (s, 2H), 5,4 (m, 3H), 5,70 (g, 2H), 6,7 (d, 2H), 6,9 (d, 1H), 7,2 (d, 2H), 7,45 (d, 1H), 8,05 (s, 1H).

Beispiel 43

6-{D-α-[3-(4-Hydroxy-2-(3'-pyridylmethylamino)-5-pyrimidinyl)-ureido]-p-hydroxy-phenylacetamido}-penicillansäure-pivaloyloxymethylester

Herstellung analog Beispiel II, 39. Man geht aus von dem Natriumsalz des Beispiels II, 5 und Pivaloyloxymethylchlorid.
Ausbeute: 58%;
IR-Spektrum: 1770, 1710, 1650, 1600 $cm^{-1}$;
NMR-Spektrum ($CD_3OD$) Signale bei ppm: 1,15 (s, 9H), 1,60 (d, 2H), 4,20 (s, 1H), (s, 2H), 5,45 (m, 3H), 5,70 (q, 2H), 6,70 (d, 2H), 7,20 (m, 3H), 7,6 (m, 1H), 8,05 (s, 1H), 8,35 (m, 2H).

III Darstellung der Cephalosporine gemäss Formel I bzw. I'

Beispiel 1

Natrium-7-{D-α-[3-(4-Hydroxy-2-(3'-pyridylamino)-5-pyrimidinyl)-ureido]-p-hydroxy-phenylacetamido}-3-acetoxymethyl-ceph-3-em-4-carboxylat

1,96 g (0,005 Mol) der nach Beispiel I/2b) erhaltenen Ureidocarbonsäure werden zusammen mit 2,1 g 7-Amino-3-acetoxymethyl-ceph-3-em-4-carbonsäure-benzhydrylester (0,005 Mol) in einem Gemisch aus 50 ml Methylenchlorid und 10 ml Dimethylformamid gelöst. Zu der Lösung wird unter Eiskühlung 1,15 g (0,0055 Mol) Dicyclohexylcarbodiimid gegeben und die Lösung 8 Stunden bei 5°C gerührt. Dann wird vom entstandenen Harnstoff abfiltriert und im Vakuum zur Trockne eingeengt. Zur Entfernung geringfügiger Verunreinigungen wird rasch über eine Kieselgelsäule chromatographiert (Eluens Methylenchlorid-Methanol 12:1).
Ausbeute an Benzhydrylester 3,4 g (82%).

Das so erhaltene Produkt wird in wenig Methylenchlorid gelöst und unter Eiskühlung 45 Minuten mit 2 ml Anisol und 10 ml Trifluoressigsäure gerührt. Anschliessend werden zweimal 50

ml Toluol hinzugefügt und im Vakuum jeweils zur Trockne eingeengt. Das entstandene Produkt wird mit Äther versetzt und durch Absaugen isoliert. Durch Zugabe der berechneten Menge Natriumäthylhexanoat in Methanol und Zufügen von Äther wird das Natriumsalz ausgefällt, abgesaugt und im Vakuum getrocknet.
Ausbeute an Natriumsalz: 2,50 g (91%);
IR-Spektrum: 1765, 1660, 1615, 1550 $cm^{-1}$;
NMR-Spektrum ($DMSO+CD_3OD$) Signale bei ppm: 2,1 (s, 3H), 3,45 (q, 2H), 4,85 (q, 2H + d, 1H), 5,55 (s, 1H), 5,60 (d, 1H), 6,75 (d, 2H), 7,25 (d, 2H), 7,4 (m, 1H), 8,3 (m, 3H), 8,7 (s, 1H).

Beispiel 2

Natrium-7-{D-α-[3-‹4-Hydroxy-2-(3'-pyridylamino)-5-pyrimidinyl›-ureido]-p-hydroxy-phenylacetamido}-3-[(1-methyl-tetrazol-5-yl)-thiomethyl]-ceph-3-em-4-carboxylat

3,94 g (0.01 Mol) der Ureidocarbonsäure des Beispiels I/2 b werden auf analoge Weise, wie in Beispiel III/1 beschrieben, mit 4,94 g (0,01 Mol) 7-Amino-3-[(1-methyl-tetrazol-5-yl)-thiomethyl]-ceph-3-em-4-carbonsäurebenzhydrylester umgesetzt. Nach Abspaltung der Schutzgruppe erhält man 4,85 g (65%) Natriumsalz.
IR-Spektrum: 1765, 1660, 1610, 1540 $cm^{-1}$;
NMR-Spektrum ($DMSO+CD_3OD$) Signale bei ppm: 3,50 (q, 2H), 3,90 (s, 3H), 4,30 (q, 2H, teilweise verdeckt durch LM), 4,80 (d, 1H), 5,50 (s, 1H), 5.70 (d, 1H), 6,75 (d, 2H), 7,35 (d, 2H), 7,4 (m, 1H), 8.3 (m, 3H), 8,75 (s, 1H).

Beispiel 3

Natrium-7-{D-α-[3-‹4-Hydroxy-2-(3'-pyridylamino)-5-pyrimidinyl›-ureido]-p-hydroxy-phenylacetamido}-3-[(2-methyl-thiadiazol-5-yl)-thiomethyl]-ceph-3-em-4-carboxylat

Analog Beispiel III/1 ausgehend von 790 mg (0.002 Mol) der Ureidocarbonsäure des Beispiels I/2 b und 1,02 g (0.002 Mol) 7-Amino-3-[(2-methyl-thiadiazol-5-yl)-thiomethyl]-ceph-3-em-4-carbonsäure-benzhydrylester. Nach Abspaltung der Schutzgruppe wurden 540 mg (39%) Natriumsalz erhalten.
IR-Spektrum: 1760, 1655, 1615, 1540 $cm^{-1}$;
NMR-Spektrum ($DMSO+CD_3OD$) Signale bei ppm: 2,7 (s, 3H), 3,50 (q, 2H), 4,45 (q, 2H), 4,90 (d. 1H), 5,50 (s, 1H). 5,65 (d, 1H), 6,75 (d, 2H), 7,3 (d, 2H), 7,4 (m, 1H), 8,3 (m, 3H), 8,75 (s, 1H).

Beispiel 4

Natrium-7-{D,L-α-[3-‹4-Hydroxy-2-(3'-pyridylamino)-5-pyrimidinyl›-ureido]-2-thienylacetamido}-3-[(1-methyl-tetrazol-5-yl)-thiomethyl]-ceph-3-em-4-carboxylat

Dieses Cephalosporin wird erhalten, wenn man von 1,11 g (0,0029 Mol) der Ureidocarbonsäure des Beispiels I/2 d sowie 1,5 g (0,003 Mol) des in Beispiel III/2 eingesetzten Benzhydrylesters ausgeht und die Umsetzung analog Beispiel III/1 führt. Nach Abspalten der Schutzgruppe werden 920 mg (48%) Natriumsalz erhalten.
IR-Spektrum: 1760, 1660, 1610, 1540 $cm^{-1}$;
NMR-Spektrum ($DMSO+CD_3OD$) Signale bei ppm: 3,55 (q, 2H), 3,90 (s, 3H), 4,35 (q, 2H), 4,90 (dd, 1H), 5,5 (dd, 1H), 5,75 (breites s, 1H), 6,9 (m, 2H), 7,35 (m, 2H), 8,25 (m, 3H), 8,75 (s, 1H).

Analog wurde hergestellt:
Natrium-7-{D-α-[3-‹4-hydroxy-2-(6'-methoxy-3'-pyridyl)-5-pyrimidinyl›-ureido-p-hydroxy-phenylacetamido}-3-[(1-methyltetrazol-5-yl)-thiomethyl]-ceph-3-em-4-carboxylat.

Beispiel 5

Natrium-7-{D-α-[3-‹4-hydroxy-2-(2'-thienylmethyl)-5-pyrimidinyl›-ureido]-p-hydroxy-phenylacetamido}-3-[(1,2,4-thiadiazol-5-yl)-thiomethyl]-ceph-3-em-4-carboxylat

Dieses Cephalosporin erhält man aus 415 mg (0,001 Mol) der Ureidocarbonsäure des Beispiels I/2 g sowie 500 mg (0,001 Mol) 7-Amino-3-[(1,2,4-thiadiazol-5-yl)-thiomethyl]-ceph-3-em-4-carbonsäurebenzhydrylester analog Beispiel III/1.

Nach Abspalten der Schutzgruppe erhält man 410 mg Natriumsalz (35%);
IR-Spektrum: 1760, 1660, 1615, 1540 $cm^{-1}$;
NMR-Spektrum ($DMSO+CD_3OD$) Signale bei ppm: 3,50 (q, 2H), 4,50 (m, 4H), 4,95 (d, 1H), 5,50 (s, 1H), 5,60 (d, 1H), 6,75 (d, 2H), 6,85 (m, 2H), 7,25 (d, 2H), 7,4 (m, 1H), 8,1 (s, 1H).

Beispiel 6

Natrium-7-{D-α-[3-‹4-hydroxy-2-(5'-äthoxycarbonyl-2-thienylamino)-5-pyrimidinyl›-ureido]-p-hydroxy-phenylacetamido}-3-acetoxymethyl]-ceph-3-em-4-carboxylat

Dieses Cephalosporin wird analog Beispiel III/1 erhalten, indem man von 4,70 g (0,01 Mol) der Ureidocarbonsäure des Beispiels I/2 f sowie 4,20 g (0,01 Mol) des in Beispiel III/1 eingesetzten Cephalosporinderivats ausgeht.
Ausbeute nach Abspalten der Schutzgruppe: 3,95 g Natriumsalz (51%);
IR-Spektrum: 1760, 1655, 1610, 1545 $cm^{-1}$;
NMR-Spektrum ($DMSO+CD_3OD$) Signale bei ppm: 1,3 (t, 3H), 2,05 (s, 2H), 3,55 (q, 2H), 4,30 (q, 2H), 4,85 (m, 2+1H), 5,4 (s, 1H), 5,6 (d, 1H), 6,8 (d, 2H), 7,35 (d, 2H), 7,9 (m, 2H), 8,05 (s, 1H).

Beispiel 7

Natrium-7-{D-α-[3-‹4-hydroxy-2-(2'-thienylmethylamino)-5-pyrimidinyl›-ureido]-p-hydroxy-phenylacetamido}-3-[(2-methyl-1,3,4-thiadiazol-5-yl)-thiomethyl]-ceph-3-em-4-carboxylat

Analog Beispiel III/1, ausgehend von 580 mg (0,0014 Mol) der Ureidocarbonsäure des Beispiels I/2 g und 720 mg (0,0014 Mol) des Cephalosporins, das im Beispiel III/3 eingesetzt wurde.
Ausbeute nach Abspalten der Benzhydrylgruppe 500 mg (46%) Natriumsalz.
IR-Spektrum: 1760, 1660, 1610, 1550 $cm^{-1}$;
NMR-Spektrum ($DMSO+CD_3OD$) Signale bei ppm: 2,75 (s, 3H), 3,55 (q, 2H), 4,25 (q, 2H, teilweise durch LM verdeckt), 4,5 (breites s, 2H), 5,0 (d, 1H), 5,45 (s, 1H), 5,65 (d, 1H), 6,6-7,4 (m, 7H), 8,1 (s, 1H).

Beispiel 8

Natrium-7-{D-α-[3-‹4-hydroxy-2-(3'-pyridylamino)-5-pyrimidinyl›-ureido]-2-furylacetamido}-3--[(1-methyl-tetrazol-5-yl)-thiomethyl]-ceph-3-em--4-carboxylat

Analog Beispiel III/1 werden 775 mg der Ureidocarbonsäure des Beispiels I/2c (0,002 Mol) mit 1,0 g (0,0021 Mol) des Cephalosporin-benzhydrylesters, der auch in Beispiel III/2 verwendet wurde, umgesetzt.

Nach Abspaltung der Benzhydrylschutzgruppe erhält man 730 mg (50%) Natriumsalz.
IR-Spektrum: 1770, 1615, 1545 $cm^{-1}$;
NMR-Spektrum ($DMSO+CD_3OD$) Signale bei ppm: 3,55 (q, 2H), 3,90 (s, 3H), 4,35 (q, 2H), 4,95 (d, 1H), 5,45 (d, 1H), 5,75 (s, 1H), 6,4 (m, 2H), 7,4 (m, 1H), 7,6 (breites s, 1H), 8,25 (m, 3H), 8,8 (breites s, 1H).

Beispiel 9

Natrium-7-{D-α-[3-‹4-hydroxy-2-(5'-äthoxycarbonyl-2'-thienyl)-5-pyrimidinyl›-ureido]-p-hydroxy--phenylacetamido}-3-[(1-methyltetrazol-5-yl)--thiomethyl]-ceph-3-em-4-carboxylat

Analog Beispiel III/1 ausgehend von 470 mg (0,001 Mol) der Ureidocarbonsäure des Beispiels I/2f sowie 500 mg (0,001 Mol) des Benzhydrylesters, der auch in Beispiel III/2 eingesetzt wurde.

Nach Abspaltung der Benzhydrylschutzgruppe erhält man 440 mg Natriumsalz (53,5%);
IR-Spektrum: 1760, 1655, 1615, 1540 $cm^{-1}$;
NMR-Spektrum ($DMSO+CD_3OD$) Signale bei ppm: 1,3 (t, 3H), 3,4 (q, 2H), 3,9 (s, 3H), 4,35 (m, 4H), 4,85 (d, 1H), 5,40 (s, 1H), 5,55 (d, 1H), 6,7 (d, 2H), 7,35 (d, 2H), 7,85 (m, 2H), 8,25 (s, 1H).

Beispiel 10

Natrium-7-{D-α-[3-‹4-hydroxy-2-(2'-furylmethylamino)-5-pyrimidinyl›-ureido]-p-hydroxy-phenylacetamido}-3-[(2-methylthiadiazol-5-yl)-thiomethyl]-ceph-3-em-4-carboxylat

Synthese analog Beispiel III/1, ausgehend von 1,12 g der Ureidocarbonsäure des Beispiels I/2j) (0,0028 Mol) sowie 1,53 g (0,003 Mol) 7-Amino-3-[(2-methyl-thiadiazol-5-yl)-thiomethyl]-ceph--3-em-4-carbonsäure-benzhydrylester.
Ausbeute an Natriumsalz 760 mg (36%);
IR-Spektrum: 1770, 1660, 1610, 1545 $cm^{-1}$;
NMR-Spektrum ($DMSO+CD_3OD$) Signale bei ppm: 2,75 (s, 3H), 3,45 (q, 2H), 4,45 (m, 4H), 4,95 (d, 1H), 5,40 (s, 1H), 5,60 (d, 1H), 6,3 (m, 2H), 6,85 (d, 2H), 7,35 (d, 2H), 7,5 (s, 1H), 8,05 (s, 1H).

Analog wurden hergestellt:
Natrium-7-{D-α-[3-‹4-hydroxy-2-(2'-pyridylmethylamino)-5-pyrimidinyl›-ureido]-p-hydroxy-phenylacetamido}-3-[(1-methyltetrazol-5-yl-thiomethyl]-ceph-3-em-4-carboxylat
Natrium-7-{D-α-[3-‹4-hydroxy-2-(4'-pyridylmethylamino)-5-pyrimidinyl›-ureido]-p-hydroxy-phenylacetamido}-3-[(1-methyltetrazol-5-yl)-thiomethyl]-ceph-3-em-4-carboxylat.

Beispiel 11

Natrium-7-{D-α-[(4-hydroxy-2-(2'-furylmethylamino-5-pyrimidinyl)-ureido]-p-hydroxy-phenylacetamido}-3-acetoxymethyl-ceph-3-em-4-carboxylat

Zu einer Lösung von 3,99 g der Ureidocarbonsäure des Beispiels I/2j (0,01 Mol) in 30 ml wasserfreiem Methylenchlorid und 30 ml Dimethylformamid gibt man 1,21 g (0,01 Mol) N,N-Dimethylanilin. Die Lösung wird auf −15°C abgekühlt und es wird bei dieser Temperatur eine Lösung von 1,1 g (0,01 Mol) Chlorameisensäureäthylester in 5 ml Methylenchlorid zugetropft. Die erhaltene Mischung wird 45 Minuten bei dieser Temperatur gehalten. Andererseits gibt man zu einer Suspension von 2,72 g (0,01 Mol) 7-Aminocephalosporansäure in 80 ml wasserfreiem Acetonitril 3 g N,O-Bis-trimethylsilylacetamid, wobei eine Lösung erhalten wird. Die Lösung wird auf −20°C abgekühlt und zu der oberen Lösung zugetropft. Danach wird die Mischung bei −10°C 60 Minuten und bei +10°C ebenfalls 60 Minuten lang umgesetzt. Nach dieser Zeit fügt man 5 ml Methanol zu und filtriert von unlöslichem Material ab. Danach wird das Lösungsmittel im Vakuum entfernt. Der Rückstand wird in 100 ml Wasser aufgenommen und die Lösung auf pH 7,5 gestellt. Bei diesem pH-Wert wird zweimal mit Essigsäureäthylester ausgeschüttelt und die organische Phase verworfen. Die wässrige Phase wird unter Eiskühlung mit verdünnter Salzsäure auf pH 2,9 gestellt, das ausgefallene Produkt abgesaugt, mit wenig Wasser gewaschen und im Vakuum getrocknet. Die wässrige Lösung wird noch zweimal mit Essigester ausgeschüttelt, die Essigesterphase getrocknet und das Lösungsmittel im Vakuum abdestilliert. Man erhält eine zweite Charge, die laut DC mit dem zunächst ausgefallenen Produkt identisch ist.

Beide Festprodukte werden vereinigt und in 80 ml trockenem Methanol mit der berechneten Menge Natriumäthylhexanoat gelöst. Es wird von etwas unlöslichem Produkt abfiltriert und bis zur vollständigen Fällung Äther hinzugefügt. Das ausgefallene Festprodukt wird abgesaugt und getrocknet.
Ausbeute: 3,60 g Natriumsalz (53%);

IR-Spektrum: 1765, 1655, 1615, 1645 $cm^{-1}$; NMR-Spektrum ($DMSO+CD_3OD$) Signale bei ppm: 2,05 (s, 3H), 3,45 (q, 2H), 4,5 (s, 2H), 4,80 (q, 2H + d, 1H), 5,50 (s, 1H), 5,65 (d, 1H), 6,35 (m, 2H), 6,75 (d, 2H), 7,30 (d, 2H), 7,45 (s, 1H), 8,05 (s, 1H).

Beispiel 12

Natrium-7-{D-α-[3-(4-hydroxy-2-(2'-furylmethylamino)-5-pyrimidinyl)-ureido]-p-hydroxy-phenylacetamido}-3-carbamoyloxymethyl-ceph-3-em-4-carboxylat

Herstellung analog Beispiel III/11, ausgehend von 1,32 g (0,005 Mol) 7-Amino-3-carbamoyloxymethyl-ceph-3-em-4-carbonsäure sowie 2,00 g der Ureidocarbonsäure des Beispiels I/2j (0,005 Mol).
Ausbeute an Natriumsalz: 1,47 g (41%);
IR-Spektrum: 1765, 1660, 1610, 1540 $cm^{-1}$;
NMR-Spektrum ($DMSO+CD_3OD$) Signale bei ppm: 3,55 (g, 2H), 4,45 (breites s, 2H), 4,8 (breit, 2H + 1H), 5,45 (s, 1H), 5,65 (d, 1H), 6,25 (m, 2H), 6,85 (d, 2H), 7,35 (d, 2H), 7,45 (s, 1H), 8,0 (s, 1H).

Beispiel 13

Natrium-7-{D-α-[(4-hydroxy-2-(2'-furylmethylamino-5-pyrimidinyl)-ureido]-p-hydroxy-phenylacetamido}-3-[(1-methyl-tetrazol-5-yl)-thiomethyl]-ceph-3-em-4-carboxylat

Herstellung analog Beispiel III/11, ausgehend von 400 mg (0,001 Mol) der Ureidocarbonsäure des Beispiels I/2j) mit 334 mg (0,001 Mol) 7-Amino-3-[(1-methyl-tetrazol-5-yl)-thiomethyl]-ceph-3-em-4-carbonsäure.
Ausbeute 430 mg Natriumsalz (58%);
IR-Spektrum: 1765, 1655, 1610, 1540 $cm^{-1}$;
NMR-Spektrum ($DMSO+CD_3OD$) Signale bei ppm: 3,50 (q, 2H), 3,95 (s, 3H), 4,35 (q, 2H), 4,50 (s, 2H), 4,95 (d, 1H), 5,45 (s, 1H), 5,60 (d, 1H), 6,35 (m, 2H), 6,7 (d, 2H), 7,2 (d, 2H), 7,5 (s, 1H), 8,05 (s, 1H).

Beispiel 14

Natrium-7-{D-α-[3-(4-hydroxy-2-(2'-furylmethylamino-5-pyrimidinyl)-ureido]-p-hydroxyphenylacetamido}-3-[(2-methylaminothiadiazol-5-yl)-thiomethyl]-ceph-3-em-4-carboxylat

Herstellung analog Beispiel III/11, ausgehend von 800 mg (0,002 Mol) der Ureidocarbonsäure des Beispiels I/2j) sowie 720 mg (0,002 Mol) 7-Amino-3-[(2-methylamino-thiadiazol-5-yl)-thiomethyl]-ceph-3-em-4-carbonsäure.
Ausbeute: 960 mg Natriumsalz (62%);
IR-Spektrum: 1770, 1650, 1615, 1545 $cm^{-1}$;
NMR-Spektrum ($DMSO+CD_3OD$) Signale bei ppm: 3,0 (s, 3H), 3,65 (q, 2H), 4,15 (m, teilweise verdeckt durch LM = 2H), 4,4 (s, 2H), 5,0 (d, 1H), 5,5 (s, 1H), 5,70 (d, 1H), 6,3 (m, 2H), 6,85 (d, 2H), 7,35 (d, 2H), 7,5 (s, 1H), 8,05 (s, 1H).

Beispiel 15

Natrium-7-{D-α-[3-(4-hydroxy-2-(3'-pyridylmethylamino)-5-pyrimidinyl)-ureido]-p-hydroxy-phenylacetamido}-3-carbamoyl-oxymethyl-ceph-3-em-4-carboxylat

Herstellung analog Beispiel III/11, ausgehend von 2,64 g (0,01 Mol) 7-Amino-3-carbamoyloxymethyl-ceph-3-em-4-carbonsäure sowie 4,10 g der Ureidocarbonsäure des Beispiels I/2e (0,01 Mol).
Ansbeute: 60%;
IR-Spektrum: 1765, 1660, 1610, 1540 $cm^{-1}$;
NMR-Spektrum ($DMSO+CD_3OD$) Signale bei ppm: 3,55 (q, 2H), 4,5 (s, 2H), 4,8 (breit, 2H+1H), 5,45 (s, 1H), (d, 1H), 6,85 (d, 2H), 7,35 (m, 3H), 7,7 (m, 1H), 8,1 (s, 1H), (m, 2H).

Beispiel 16

Natrium-7-{D-α-[3-‹4-hydroxy-2-(3'-pyridylmethylamino)-5-pyrimidinyl›-ureido]-p-hydroxy-phenylacetamido}-3-[(1-methyltetrazol-5-yl)-thiomethyl]-ceph-3-em-4-carboxylat

Herstellung analog Beispiel III/11, ausgehend von 1,23 g der Ureidocarbonsäure des Beispiels I/2e) (0,003 Mol) sowie 985 mg (0,003 Mol) der 7-Amino-3-[(1-methyl-tetrazol-5-yl)-thiomethyl]-ceph-3-em-4-carbonsäure.
Ausbeute an Natriumsalz: 1,58 g (71%);
IR-Spektrum: 1765, 1655, 1610, 1550 $cm^{-1}$;
NMR-Spektrum ($DMSO+CD_3OD$) Signale bei ppm: 3.5 (q, 2H), 3.9 (s, 3H), 4,40 (q, 2H), 4,50 (s, 2H), 4,85 (d, 1H), 5,45 (s, 1H), 5,65 (d, 1H), 6,85 (d, 2H), 7,35 (m, 3H), 7,75 (m, 1H), 8,1 (s, 1H), 8,5 (m, 2H).

Beispiel 17

Natrium-7-{D-α-[3-‹4-hydroxy-2-(2'-thienylmethylamino)-5-pyrimidinyl›-ureido]-p-hydroxy-phenylacetamido}-3-(carbamoyloxymethyl)-ceph-3-em-4-carboxylat

Herstellung analog Beispiel III/11, indem man von 1,0 g (0,0038 Mol) des Cephalosporinderivats des Beispiels III/15 sowie von 1,6 g (0,00385 Mol) der Ureidocarbonsäure des Beispiels I/2 g ausgeht. Die Ausbeute an Natriumsalz beträgt 1,33 g (50,5%);
IR-Spektrum: 1765, 1655, 1605, 1545 $cm^{-1}$;
NMR-Spektrum ($DMSO+CD_3OD$) Signale bei ppm: 3,55 (q, 2H), 4,55 (berites s, 2H), 4,85 (m, 2 + 1H), 5,45 (s, 1H), 5,65 (d, 1H), 6,65 (d, 2H), 6,80 (m, 2H), 7,30 (d, 2H), 7,45 (m, 1H), 8,05 (s, 1H).

Beispiel 18

Natrium-7-{D-α-[3-‹4-hydroxy-2-(2'-thienylmethylamino)-5-pyrimidinyl›-ureido]-p-hydroxy-phenylacetamido}-3-[(tetrazol-5-yl)-thiomethyl]-ceph-3-em-4-carboxylat

Analog Beispiel III/11 ausgehend von 415 mg (0,001 Mol) der Ureidocarbonsäure des Beispiels I/2 g, sowie 315 mg (0,001 Mol) 7-Amino-3-(tetrazol-5-yl)-thiomethyl-ceph-3-em-4-carboxylat.
Man erhält 385 mg Natriumsalz (52%);
IR-Spektrum: 1765, 1655, 1615, 1540 $cm^{-1}$;
NMR-Spektrum ($DMSO+CD_3OD$) Signale bei ppm: 3,4 (q, 2H), 4,35 (q, 2H), 4,5 (s, 2H), 4,85 (d, 1H), 5,40 (s, 1H), 5,55 (d, 1H), 6,75 (d, 2H), 6,85 (m, 2H), 7,30 (d, 2H), 7,45 (m, 1H), 8,05 (s, 1H).

Beispiel 19

Natrium-7-{D-α-[3-‹4-hydroxy-2-(2'-thienylmethylamino)-5-pyrimidinyl›-ureido]-p-hydroxy-phenylacetamido}-3-[(1-methyltetrazol-5-yl)-thiomethyl]-ceph-3-em-4-carboxylat

Herstellung analog Beispiel III/11, ausgehend von 830 mg der Ureidocarbonsäure des Beispiels I/2g) (0,002 Mol) sowie 656 mg (0,002 Mol) der 7-Amino[(1-methyl-tetrazol-5-yl)-thiomethyl]-ceph-3-em-4-carbonsäure.
Ausbeute an Natriumsalz: 940 mg (63%);
IR-Spektrum: 1765, 1655, 1610, 1550 $cm^{-1}$;
NMR-Spektrum ($DMSO+CD_3OD$) Signale bei ppm: 3,5 (q, 2H), 3,9 (s, 3H), 4,40 (q, 2H), 4,50 (s, 2H), 4,85 (d, 1H), 5,45 (s, 1H), 5,65 (d, 1H), 6,85 (d, 2H), 6,95-7,4 (m, 5H), 8,05 (s, 1H).

Beispiel 20

Natrium-7-{D-α-[3-‹4-hydroxy-2-(2'-thienylmethylamino-5-pyrimidinyl›-ureido]-p-hydroxy-phenylacetamido}-3-[(2-methyloxadiazol-5-yl)-thiomethyl]-ceph-3-em-4-carboxylat

Dieses Cephalosporin wird analog Beispiel III/11 hergestellt. Man geht aus von 2,08 g (0,005 Mol) der Ureidocarbonsäure des Beispiels I/2g sowie von 1,65 g (0,005 Mol) 7-Amino-3-[(2-methyl-1,3,4-oxadiazol)-5-thiomethyl]-ceph-3-em-4-carboxylat.
Ausbeute an Natriumsalz: 2,22 g (59%);
IR-Spektrum: 1765, 1650, 1610, 1540 $cm^{-1}$;
NMR-Spektrum ($DMSO+CD_3OD$) Signale bei ppm: 2,45 (s, 3H), 3,6 (q, 2H), 4,2 (q, 2H), 4,45 (s, 2H), 4,95 (d, 1H), 5,45 (s, 1H), 5,60 (d, 1H), 6,75 (d, 2H), 6,85-7,4 (m, 5H), 8,05 (s, 1H).

Beispiel 21

Natrium-7-{D-α-[3-‹4-hydroxy-2-(2'-thienylmethylamino)-5-pyrimidinyl›-ureido]-m,p-dihydroxy-phenylacetamido}-3-[1-methyl-tetrazol-5-yl-thiomethyl]-ceph-3-em-4-carboxylat

Herstellung analog Beispiel III/11, ausgehend von 2,15 g der Ureidocarbonsäure des Beispiels I/2k (0,005 Mol) und 1,64 g (0,005 Mol) des Cephalosporinderivates, das in Beispiel III/19 eingesetzt wurde.
Ausbeute an Natriumsalz: 2,49 g (65%);
IR-Spektrum: 1765, 1660, 1605, 1545 $cm^{-1}$;
NMR-Spektrum ($DMSO+CD_3OD$) Signale bei ppm: 3,50 (q, 2H), 3,90 (s, 3H), 4,30 (q, 2H), teilweise durch LM verdeckt, 4,50 (s, 2H), 4,80 (d, 1H), 5,50 (s, 1H), 5,70 (d, 1H), 6,7 (d, 1H), 7,0 (m, 4H), 7,35 (m, 1H), 8,1 (s, 1H).

Beispiel 22

Natrium-7-{D-α-[3-‹4-hydroxy-2-(2'-thienylmethylamino)-5-pyrimidinyl›-ureido]-2-furyl-acetamido}-3-acetoxymethyl-ceph-3-em-4-carboxylat

Herstellung analog Beispiel III/11, ausgehend von 390 mg der Ureidocarbonsäure des Beispiels I/21 (0,001 Mol) und 272 mg 7-Aminocephalosporinsäure (0,001 Mol).
Ausbeute an Natriumsalz: 390 mg (54%);
IR-Spektrum: 1765, 1660, 1610, 1540 $cm^{-1}$;
NMR-Spektrum ($DMSO+CD_3OD$) Signale bei ppm: 2,1 (s, 3H), 3,45 (q, 2H), 4,5 (s, 2H), 4,85 (d, 1H + q, 2H), 5,5 (d, 1H), 5,85 (s, 1H), 6,3 (m, 2H), 7,0 (m, 2H), 7,3 (m, 1H), 7,5 (s, 1H), 8,1 (s, 1H).

Beispiel 23

Natrium-7-{D-α-[3-‹4-hydroxy-2-(2'-thienylmethylamino)-5-pyrimidinyl›-ureido]-2-furyl-acetamido}-3-[(1-methyl-tetrazol-5-yl)-thiomethyl]-ceph-3-em-4-carboxylat

Herstellung analog Beispiel III/11, ausgehend von 780 mg (0,002 Mol) der Ureidocarbonsäure des Beispiels I/21) sowie 655 mg (0,002 Mol) des Cephalosporinderivates, das auch in Beispiel III/19 eingesetzt wurde.
Ausbeute 650 mg Natriumsalz (46%);
IR-Spektrum: 1770, 1665, 1620, 1550 $cm^{-1}$;
NMR-Spektrum ($DMSO+CD_3OD$) Signale bei ppm: 3,45 (q, 2H), 3,90 (s, 3H), 4,35 (q, 2H), 4,50 (s, 2H), 4,9 (d, 1H), 5,5 (d, 1H), 5,75 (s, 1H), 6,3 (m, 2H), 7,0 (m, 2H), 7,3 (m, 1H), 7,5 (s, 1H), 8,1 (s, 1H).

Analog wurde hergestellt:
Natrium-7-{D,L-α-[3-‹4-hydroxy-2-(2'-thienylmethylamino)-5-pyrimidinyl›-ureido]-3-furyl-acetamido}-3-[(1-methyl-tetrazol-5-yl)-thiomethyl]-ceph-3-em-4-carboxylat.

Beispiel 24

Natrium-7-{-α-[‹4-hydroxy-2-(2'-thienylmethylamino)-5-pyrimidinyl›-ureido]-2-thienylacetamido}-3-acetoxymethyl-ceph-3-em-4-carboxylat

Herstellung analog Beispiel III/11 ausgehend vom Umsetzungsprodukt von 1,21 g (0,003 Mol) der Ureidocarbonsäure des Beispiels I/2m sowie 815 mg (0,003 Mol) 7-Aminocephalosporansäure.
Ausbeute 1,44 g (61%) Natriumsalz;
IR-Spektrum: 1765, 1660, 1615, 1535 $cm^{-1}$;
NMR-Spektrum ($DMSO+CD_3OD$) Signale bei ppm: 2,05 (s, 3H), 3,55 (q, 2H), 4,50 (s, 2H), 4,80 (m, 2 + 1H), 5,45 (d, 1H), 5,75 (s, 1H), 7,0 (m, 4H), 7,25 (m, 2H), 8,1 (s, 1H).

Beispiel 25

Natrium-7-{D-α-[3-‹4-hydroxy-2-(2'-thienylmethylamino)-5-pyrimidinyl›-ureido]-2-thienylacetamido}-3-[(1-methyl-tetrazol-5-yl)-thiomethyl]-ceph-3-em-4-carboxylat

Dieses Cephalosporin wird erhalten, wenn man von 1,20 g (0,0029 Mol) der Ureidocarbonsäure des Beispiels I/2 m, sowie 980 mg (0,003 Mol) des in Beispiel III/19 eingesetzten Cephalosporinderivats ausgeht und die Umsetzung analog Beispiel III/11 führt.

Es werden 1,28 g (57,5%) Natriumsalz erhalten.
IR-Spektrum: 1770, 1660, 1610, 1540 $cm^{-1}$;
NMR-Spektrum ($DMSO+CD_3OD$) Signale bei ppm: 3,55 (q, 2H), 3,90 (s, 3H), 4,35 (q, 2H), 4,50 (s, 2H), 4,90 (d, 1H), 5,5 (d, 1H), 5,75 (s, 1H), 7,0 (m, 4H), 7,25 (m, 2H), 8,10 (s, 1H).

Analog wurde hergestellt:
Natrium-7-{D-α-[3-‹4-hydroxy-2-(3'-thienylmethylamino)-5-pyrimidinyl›-ureido]-p-hydroxy-phenylacetamido}-3-[(1-methyltetrazol-5-yl)-thiomethyl]-ceph-3-em-4-carboxylat.

Beispiel 26

Natrium-7-{D-α-[3-‹4-hydroxy-2-(5'-methyl-2'-thienylmethylamino)-5-pyrimidinyl›-ureido]-p-hydroxy-phenylacetamido}-3-acetoxymethyl-ceph-3-em-4-carboxylat

Herstellung analog Beispiel III/11, wenn man von 1,72 g (0,004 Mol) der Ureidocarbonsäure des Beispiels I/2h sowie von 1,09 g (0,004 Mol) 7-Aminocephalosporansäure ausgeht.
Ausbeute an Natriumsalz: 1,55 g (46%);
IR-Spektrum: 1765, 1655, 1605, 1545 $cm^{-1}$;
NMR-Spektrum ($DMSO+CD_3OD$) Signale bei ppm: 2,1 (s, 3H), 2,4 (s, 3H), 3,55 (q, 2H), 4,50 (s, 2H), 4,80 (m, 2 + 1H), 5,45 (s, 1H), 5,65 (d, 1H), 6,7 (d, 2H), 6,8 (breites s, 2H), 7,20 (d, 2H), 8,1 (s, 1H).

Analog wurde hergestellt:
Natrium-7-{D-α-[3-‹4-hydroxy-2-(5'-chlor-2'-thienylmethylamino)-5-pyrimidin-3-yl›-ureido]-p-hydroxy-phenylacetamido}-3-acetoxymethyl-ceph-3-em-4-carboxylat.

Beispiel 27

Natrium-7-{D-α-[3-‹4-hydroxy-2-(5'-methyl-2'-thienylmethylamino-5-pyrimidinyl›-ureido]-p-hydroxy-phenylacetamido}-3-[(1-methyl-tetrazol-5-yl)-thiomethyl]-ceph-3-em-4-carboxylat

Herstellung analog Beispiel III/11, ausgehend von 430 mg der Ureidocarbonsäure des Beispiels I/2h) (0,001 Mol) sowie 328 mg (0,001 Mol) der 7-Amino-[(1-methyl-tetrazol-5-yl)-thiomethyl]-ceph-3-em-4-carbonsäure.
Ausbeute an Natriumsalz: 410 mg (53,5%);
IR-Spektrum: 1765, 1655, 1610, 1550 $cm^{-1}$;
NMR-Spektrum ($DMSO+CD_3OD$) Signale bei ppm: 2,4 (s, 3H), 3,5 (q, 2H), 3,9 (s, 3H), 4,40 (q, 2H), 4,50 (s, 2H), 4,85 (d, 1H), 5,45 (s, 1H), 5,65 (d, 1H), 6,7 (d, 2H), 6,8 (breites s, 2H), 7,25 (d, 2H), 8,1 (s, 1H).

Beispiel 28

Natrium-7-{D-α-[3-‹4-hydroxy-2-(5'-chlor-2'-thienylmethylamino-5-pyrimidinyl›-ureido]-p-hydroxy-phenylacetamido}-3-[(1-methyl-tetrazol-5-yl)-thiomethyl]-ceph-3-em-4-carboxylat

Herstellung analog Beispiel III/11, ausgehend von 450 mg der Ureidocarbonsäure des Beispiels I/2 i (0,001 Mol) sowie 328 mg (0,001 Mol) der 7-Amino-[(1-methyl-tetrazol-5-yl)-thiomethyl]-ceph-3-em-4-carbonsäure.
Ausbeute an Natriumsalz: 540 mg (61,5%);
IR-Spektrum: 1765, 1655, 1610, 1550 $cm^{-1}$;
NMR-Spektrum ($DMSO+CD_3OD$) Signale bei ppm: 3,5 (q, 2H), 3,9 (s, 3H), 4,40 (q, 2H), 4,50 (s, 2H), 4,85 (d, 1H), 5,45 (s, 1H), 5,65 (d, 1H), 6,7-7,0 (m, 4H), 7,25 (d, 2H), 8,15 (s, 1H).

Beispiel 29

Natrium-7-{D-α-[3-‹4-hydroxy-2-(2'furylmethylamino-5-pyrimidinyl›-ureido]-p-hydroxy-phenylacetamido}-3-[(1,2,3-triazol-4-yl)-thiomethyl]-ceph-3-em-4-carboxylat

3,99 g (0,01 Mol) der Ureidocarbonsäure des Beispiels I/2 j werden auf analoge Weise, wie in Beispiel III/11 beschrieben, mit 3,12 g (0,01 Mol) 7-Amino-3-[(1,2,3-triazol-4-yl)-thiomethyl]-ceph-3-em-4-carbonsäure umgesetzt.
Man erhält 4,05 g (57%) Natriumsalz.
IR-Spektrum: 1770, 1660, 1610, 1540 $cm^{-1}$;
NMR-Spektrum ($DMSO+CD_3OD$) Signale bei ppm: 3,55 (q, 2H), 4,30 (q, 2H), 4,4 (s, 2H, teilweise verdeckt durch LM), 4,85 (d, 1H), 5,50 (s, 1H), 5,70 (d, 1H), 6,3 (m, 2H), 6,75 (d, 2H), 2,75 (d, 2H), 7,05 (s, 1H), 7,75 (s, 1H), 8,05 (s, 1H).

Beispiel 30

Natrium-7-{D-α-[3-‹4-hydroxy-2-(2'-furylmethylamino-5-pyrimidinyl›-ureido]-p-hydroxy-phenylacetamido}-3-[(1,3,4-thiadiazol-2-yl)-thiomethyl]-ceph-3-em-4-carboxylat

ausgehend von 800 mg (0,002 Mol) der Ureidocarbonsäure des Beispiels I/2 j und 660 mg (0,002 Mol) 7-Amino-3-[1,3,4-thiadiazol-2-yl)-thiomethyl]-ceph-3-em-4-carbonsäure. Es werden 810 mg (55%) Natriumsalz erhalten.
IR-Spektrum: 1770, 1655, 1615, 1540 $cm^{-1}$;
NMR-Spektrum ($DMSO+CD_3OD$) Signale bei ppm: 3,50 (q, 2H), 4,45 (m, 4H), 4,90 (d, 1H), 5,50 (s, 1H), 5,65 (d, 1H), 6,35 (m, 2H), 6,70 (d, 2H), 7,2 (d, 2H), 7,5 (s, 1H), 8,05 (s, 1H), 8,55 (s, 1H).

Beispiel 31

Natrium-7-{D-α-[3-‹4-hydroxy-2-(2'furylmethyl-

amino)-5-pyrimidinyl›-ureido]-2-furylacetamido}--3-acetoxymethyl-ceph-3-em-4-carboxylat

1,86 g (0,005 Mol) der Ureidocarbonsäure des Beispiels I/2n werden wie in Beispiel III/11 beschrieben mit 1,36 g 7-Aminocephalosporansäure umgesetzt. Man erhält 1,76 g (54,5%) Natriumsalz.
IR-Spektrum: 1765, 1660, 1605, 1545 $cm^{-1}$;
NMR-Spektrum DMSO+$CD_3OD$) Signale bei ppm: 2,05 (s, 3H), 3,55 (q, 2H), 4,4 (s, 2H), 4,85 (m, 2 + 1H), 5,45 (d, 1H), 5,75 (s, 1H), 6,3 (m, 4H), 7,5 (m, 2H), 8,1 (s, 1H).

Beispiel 32

Natrium-7-{D-α-[3-‹4-hydroxy-2-(2'furylmethylamino)-5-pyrimidinyl›-ureido]-2-furylacetamido}--3-[(1-methyl-tetrazol-5-yl)-thiomethyl]-ceph-3--em-4-carboxylat

Herstellung analog Beispiel III/11, ausgehend von 373 mg der Ureidocarbonsäure des Beispiels I/2n) (0,001 Mol) sowie 328 mg (0,001 Mol) der 7-Amino[(1-methyl-tetrazol-5-yl)-thiomethyl]--ceph-3-em-4-carbonsäure.
Ausbeute an Natriumsalz: 420 mg (60%);
IR-Spektrum: 1765, 1655, 1610, 1550 $cm^{-1}$;
NMR-Spektrum (DMSO+$CD_3OD$) Signale bei ppm: 3,5 (q, 2H), 3,9 (s, 3H), 4,40 (m, 4H), 4,95 (d, 1H), 5,45 (d, 1H), 5,70 (s, 1H), 6,35 (m, 4H), 7,45 (m, 2H), 8,1 (s, 1H).

Beispiel 33

Natrium-7-{D-α-[3-‹4-hydroxy-2-(2'-furylmethylamino)-5-pyrimidinyl›-ureido]-2-thienylacetamido}-3-acetoxymethyl-ceph-3-em-4-carboxylat

1,15 g (0,003 Mol) der Ureidocarbonsäure des Beispiels I/20 werden wie in Beispiel III/11 beschrieben mit 815 mg 7-Aminocephalosporansäure umgesetzt. Man erhält 890 mg Natriumsalz (45%);
IR-Spektrum: 1765, 1655, 1610, 1550 $cm^{-1}$;
NMR-Spektrum (DMSO+$CD_3OD$) Signale bei ppm: 2,05 (s, 3H), 3,50 (q, 2H), 4,50 (s, 2H), 4,85 (m, 3H), 5,5 (d, 1H), 5,7 (s, 1H), 6,3 (m, 2H), 7,0 (m, 2H), 7,35 (m, 1H), 7,5 (m, 1H), 8,1 (s, 1H).

Beispiel 34

Natrium-7-{D-α-‹4-hydroxy-2-(2'-furylamino)-5--pyrimidinyl›-ureido]-2-thienylacetamido}-3-[(1--methyl-tetrazol-5-yl)-thiomethyl]-ceph-3-em-4--carboxylat

Herstellung analog Beispiel III/11, ausgehend von 385 mg der Ureidocarbonsäure des Beispiels I/20 (0,001 Mol) sowie 328 mg (0,001 Mol) der 7-Amino-[(1-methyl-tetrazol-5-yl)-thiomethyl]--ceph-3-em-4-carbonsäure.
Ausbeute an Natriumsalz: 450 mg (57%);
IR-Spektrum: 1765, 1655, 1610, 1550 $cm^{-1}$;
NMR-Spektrum (DMSO+$CD_3OD$) Signale bei ppm: 3,5 (q, 2H), 3,9 (s, 3H), 4,40 (m, 4H), 4,95 (d, 1H), 5,45 (d, 1H), 5,70 (s, 1H), 6,3 (m, 2H), 7,0 (m, 2H), 7,4 (m, 2H), 8,1 (s, 1H).

Beispiel 35
Natrium-7-{D-α-[3-‹4-hydroxy-2-(5'-methyl-2'-furylmethylamino)-5-pyrimidinyl›-ureido]-p-hydroxy-phenylacetamido}-3-[(1-methyltetrazol-5--yl)-thiomethyl]-ceph-3-em-4-carboxylat

Herstellung analog Beispiel III/11, ausgehend von 1,65 g der Ureidocarbonsäure des Beispiels I/2p) (0,004 Mol) sowie 1,32 g (0,004 Mol) der 7-Amino-[(1-methyl-tetrazol-5-yl)-thiomethyl]--ceph-3-em-4-carbonsäure.
Ausbeute an Natriumsalz: 1,82 g (60,5%);
IR-Spektrum: 1765, 1650, 1610, 1550 $cm^{-1}$;
NMR-Spektrum (DMSO+$CD_3OD$) Signale bei ppm: 2,5 (s, 3H), 3,5 (q, 2H), 3,9 (s, 3H), 4,40 (m, 4H), 4,85 (d, 1H), 5,45 (s, 1H), 5,65 (d, 1H), 6,3 (m, 2H), 6,80 (d, 2H), 7,30 (d, 2H), 8,1 (s, 1H).
Analog wurden hergestellt:
Natrium-7-{D-α-[3-‹4-hydroxy-2-(5'-methyl-2'-furylmethylamino)-5-pyrimidinyl›-ureido]-2-thienylacetamido}-3-[(1-methyl-tetrazol-5-yl)-thiomethyl]-ceph-3-em-4-carboxylat
Natrium-7-{D-α-[3-‹4-hydroxy-2-(5'-methyl-2'-furylmethylamino)-5-pyrimidinyl›-ureido]-2-furylacetamido}-3-[(1-methyl-tetrazol-5-yl)-thiomethyl]-ceph-3-em-4-carboxylat.

Beispiel 36

Natrium-7-{D-α-[3-‹4-hydroxy-2-(2'-tetrahydro--furylmethylamino)-5-pyrimidinyl›-ureido]-p-hydroxy-phenylacetamido}-3-[(1-methyl-tetrazol-5--yl)-thiomethyl]-ceph-3-em-4-carboxylat

Herstellung analog Beispiel III/11, ausgehend von 403 mg der Ureidocarbonsäure des Beispiels I/2q) (0,001 Mol) sowie 328 mg (0,001 Mol) der 7-Amino-[(1-methyl-tetrazol-5-yl)-thiomethyl]-ceph-3-em-4-carbonsäure.
Ausbeute an Natriumsalz: 462 mg (58%);
IR-Spektrum: 1770, 1655, 1610, 1550 $cm^{-1}$;
NMR-Spektrum (DMSO+$CD_3OD$) Signale bei ppm: 1,9 (m, 4H), 3,5-4,0 (m, 7H), 3,9 (s, 3H), 4,40 (q, 2H), 4,85 (d, 1H), 5,45 (s, 1H), 5,65 (d, 1H), 6,70 (d, 2H), 7,20 (d, 2H), 8,0 (s, 1H).

Beispiel 37

Natrium-7-{D-α-[3-‹4-hydroxy-2-(2'-pyrrolylmethylamino)-5-pyrimidinyl›-ureido]-p-hydroxy-phenylacetamido}-3-[(1-methyl-tetrazol-5-yl)-thiomethyl]-ceph-3-em-4-carboxylat

Herstellung analog Beispiel III/11, ausgehend von 800 mg der Ureidocarbonsäure des Beispiels I/2r) (0,002 Mol) sowie 656 mg (0,002 Mol) der 7-Amino-[(1-methyl-tetrazol-5-yl)-thiomethyl]--ceph-3-em-4-carbonsäure.
Ausbeute an Natriumsalz: 650 mg (44,5%);
IR-Spektrum: 1765, 1655, 1610, 1550 $cm^{-1}$;

NMR-Spektrum (DMSO+$CD_3OD$) Signale bei ppm: 3,5 (q, 2H), 3,9 (s, 3H), 4,40 (m, 4H), 4,85 (d, 1H), 5,45 (s, 1H), 5,65 (d, 1H), 6,1 (m, 2H), 6,75 (d, 2H), 7,25 (d, 2H), 8,1 (s, 1H).

Analog wurde hergestellt:
Natrium-7-{D-α-[3-‹4-hydroxy-2-(2'-pyrrolylmethylamino)-5-pyrimidinyl›-ureido]-p-hydroxy-phenylacetamido}-3-acetoxy-methyl-ceph-3-em-4-carboxylat

Beispiel 38

Natrium-7-{D-α-[3-‹4-hydroxy-2-(4'-methyl-2'-imidazolyl-methylamino)-5-pyrimidinyl)-ureido]-p-hydroxy-phenylacetamido}-3-[(1-methyl-tetrazol-5-yl)-thiomethyl]-ceph-3-em-4-carboxylat

Herstellung analog Beispiel III/11, ausgehend von 1,6 g der Ureidocarbonsäure des Beispiels I/2s) (0,004 Mol) sowie 1,32 g (0,004 Mol) der 7-Amino-[(1-methyl-tetrazol-5-yl)-thiomethyl]-ceph-3-em-4-carbonsäure.
Ausbeute an Natriumsalz: 1,55 g (51%);
IR-Spektrum: 1770, 1655, 1610, 1555 $cm^{-1}$;
NMR-Spektrum (DMSO+$CD_3OD$) Signale bei ppm: 2,1 (s, 3H), 3,5 (q, 2H), 3,9 (s, 3H), 4,40 (m, 4H), 4,90 (d, 1H), 5,45 (s, 1H), 5,70 (d, 1H), 6,85 (d, 2H), 7,35 (m, 4H), 8,05 (s, 1H).

Analog wurde hergestellt:
Natrium-7-{D-α-[3-‹4-hydroxy-2-(2'-oxazolylmethylamino)-5-pyrimidinyl›-ureido]-p-hydroxy-phenylacetamido}-3-[(1-methyl-tetrazol-5-yl)-thiomethyl]-ceph-3-em-4-carboxylat
Natrium-7-{D-α-[3-‹4-hydroxy-2-(5'-methyl-2'-triazolylmethylamino)-5-pyrimidinyl)-ureido]-p-hydroxy-phenylacetamido}-3-[(1-methyl-tetrazol-5-yl)-thiomethyl]-ceph-3-em-4-carboxylat.

Beispiel 39

Natrium-7-{D-α-[3-‹4-hydroxy-2-(5'-methyl-2'-thienylamino)-5-pyrimidinyl›-ureido]-p-hydroxy-phenylacetamido}-3-acetoxy-methyl-ceph-3-em-4-carboxylat

Herstellung analog Beispiel III/11, man geht aus von 2,72 g 7-Aminocephalosporansäure des Beispiels I/2u).
Ausbeute an Natriumsalz: 3,73 (53,5%);
NMR-Spektrum (DMSO+$CD_3OD$) Signale bei ppm: 2,05 (s, 3H), 2,5 (s, 3H), 3,45 (q, 2H), 4,8 (m, 3H), 5,4 (s, 1H), 5,65 (d, 1H), 6,5 (m, 2H), 6,8 (d, 2H), 7,25 (d, 2H), 8,15 (s, 1H).

Beispiel 40

Natrium-7-{D-α-[3-‹4-hydroxy-2-(5'-methyl-2'-thienylamino)-5-pyrimidinyl›-ureido]-p-hydroxy-phenylacetamido}-3-[(1-methyl-tetrazol-5-yl)-thiomethyl]-ceph-3-em-4-carboxylat

Herstellung analog Beispiel III/11, ausgehend von 2,07 g der Ureidocarbonsäure des Beispiels I/2u) (0,005 Mol) sowie 1,64 g (0,005 Mol) der 7-Amino[(1-methyl-tetrazol-5-yl)-thiomethyl]-ceph-3-em-4-carbonsäure.
Ausbeute an Natriumsalz 2,2 g (59%);
IR-Spektrum: 1765, 1655, 1615, 1555 $cm^{-1}$;
NMR-Spektrum (DMSO+$CD_3OD$) Signale bei ppm: 2,45 (s, 3H), 3,45 (q, 2H), 3,9 (s, 3H), 4,40 (q, 2H), 4,85 (d, 1H), 5,45 (s, 1H), 5,65 (d, 1H), 6,55 (m, 2H), 6,85 (d, 2H), 7,35 (d, 2H), 8,1 (s, 1H).

Beispiel 41

Natrium-7-{D-α-[3-‹4-hydroxy-2-(4'-methyl-2'-thiazolylamino)-5-pyrimidinyl›-ureido]-p-hydroxy-phenylacetamido}-3-[(1-methyl-tetrazol-5-yl)-thiomethyl]-ceph-3-em-4-carboxylat

Herstellung analog Beispiel III/11, ausgehend von 1,0 g der Ureidocarbonsäure des Beispiels I/2u) (0,0024 Mol) sowie 800 mg (0,0025 Mol) der 7-Amino-[(1-methyl-tetrazol-5-yl)-thiomethyl]-ceph-3-em-4-carbonsäure.
Ausbeute an Natriumsalz: 630 mg (35,5%);
IR-Spektrum: 1765, 1655, 1610, 1550 $cm^{-1}$;
NMR-Spektrum (DMSO+$CD_3OD$) Signale bei ppm: 2,5 (s, 3H), 3,5 (q, 2H), 3,9 (s, 3H), 4,40 (q, 2H), 4,85 (d, 1H), 5,45 (s, 1H), 5,65 (d, 1H), 6,45 (s, 1H), 6,80 (d, 2H), 7,25 (d, 2H), 8,05 (s, 1H).

Analog wurde hergestellt:
Natrium-7-{D-α-[3-‹4-hydroxy-2-(2'-thiazolylamino)-5-pyrimidinyl)-ureido]-p-hydroxy-phenylacetamido}-3-[(1-methyl-tetrazol-5-yl)-thiomethyl]-ceph-3-em-4-carboxylat.

Beispiel 42

Natrium-7-{D-α-[3-‹4-hydroxy-2-(6'-methylsulfinyl-3'-pyridylamino)-5-pyrimidinyl›-ureido]-p-hydroxy-phenylacetamido}-3-acetoxymethyl-ceph-3-em-4-carboxylat

Die Synthese erfolgt analog Beispiel III, 11, indem man von 7-Aminocephalosporansäure und der Ureidocarbonsäure des Beispiels I, 2v ausgeht.
Ausbeute an Natriumsalz 44%;
IR-Spektrum: 1760, 1655, 1605, 1550 $cm^{-1}$;
NMR-Spektrum (DMSO+$CD_3OD$) Signale bei ppm: 2,05 (s, 3H), 2,7 (s, 3H), 3,40 (q, 2H), 4,65 (m, 2H), 4,85 (d, 1H), 5,45 (s, 1H), 5,60 (d, 1H), 6,70 (d, 2H), 7,2 (d, 2H), 7,7 (d, 1H), 8,25 (s, 1H), 8,45 (m, 1H), 8,85 (s, 1H).

Nach der Methode des Beispiels III, 11 und mit dem entsprechenden Cephalosporindevat wurden die Cephalosporine der folgenden Tabelle synthetisiert (Natriumsalze).

| Beispiel | A | $(CH_2)_nR_1$ | D | mit der Ureido-carbonsäure des Beispiels I | IR-Spektrum ($cm^{-1}$) | NMR-Spektrum ($DMSO/CD_3OD$) Signale bei ppm |
|---|---|---|---|---|---|---|
| 43 | p-HO-Phenyl | 6-Methylsulfinyl-3--pyridyl | 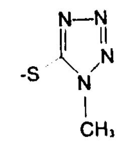 | 2v | 1760, 1655, 1605 | 2,75 (s, 3H), 3,50 (q, 2H), 3,95 (s, 3H), 4,30 (m, 2H), 4,80 (d, 1H), 5,50 (s, 1H), 5,65 (d, 1H), 6,75 (d, 2H), 7,25 (d, 2H), 7,7 (d, 1H), 8,40 (m, 1H), 8,85 (s, 1H). |
| 44 | p-HO-Phenyl | 6-Methylsulfinyl-3--pyridyl | 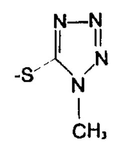 | 2y | 1765, 1660, 1610 | 3,05 (s, 3H), 3,45 (q, 2H), 3,90 (s, 3H), 4,35 (q, 2H), 4,85 (d, 1H), 5,45 (s, 1H), 5,65 (d, 1H), 6,80 (d, 2H), 7,30 (d, 2H), 7,85 (d, 1H), 8,25 (s, 1H), 8,6 (d, 1H), 8,90 (s, 1H). |
| 45 | p-HO-Phenyl | 6-Methylsulfinyl-3--pyridyl | $OCOCH_3$ | 2y | 1765, 1660, 1615 | 2,05 (s, 3H), 3,10 (s, 3H), 3,4 (q, 2H), 4,8 (m, 3H), 5,45 (s, 1H), 5,65 (d, 1H), 6,80 (d, 2H), 7,30 (d, 2H), 7,80 (d, 1H), 8,25 (s, 1H), 8,65 (d, 1H), 8,90 (s, 1H). |
| 46 | 2-Thienyl | 6-Methylsulfinyl-3--pyridyl | 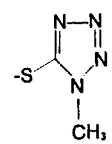 | 2x | 1760, 1655, 1610 | 2,80 (s, 3H), 3,45 (q, 2H), 3,90 (s, 3H), 4,35 (m, 2H), 4,85 (dd, 1H), 5,5 (dd, 1H,) 5,65 (breites s, 1H), 6,9 (m, 2H), 7,35 (m, 1H), 7,85 (d, 1H), 8,30 (s, 1H), 8,6 (d, 1H), 8,85 (s, 1H). |
| 47 | p-HO-Phenyl | 3-Pyridylmethyl | $OCOCH_3$ | 2e | 1760, 1655, 1605 | 2,05 (s, 3H), 3,45 (q, 2H), 4,5 (s, 2H), 4,85 (m, 3H), 5,45 (s, 1H), 5,60 (d, 1H), 6,75 (d, 2H), 7,35 (m, 2+1H), 7,7 (m, 1H), 8,10 (s, 1H), 8,5 (m, 2H). |
| 48 | 2-Thienyl | 3-Pyridylmethyl | 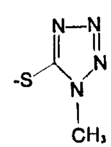 | 2ab | 1760, 1660, 1610 | 3,40 (q, 2H), 3,95 (s, 3H), 4,35 (m, 2H), 4,45 (m, 2H, 1H), 5,50 (dd, 1H), 5,70 (s, 1H), 7,0 (m, 2H), 7,3 (m, 1H), 7,65 (m, 1H), 8,05 (s, 1H), 8,45 (m, 2H). |

| Beispiel | A | $(CH_2)_nR_1$ | D | mit der Ureido-carbonsäure des Beispiels I | IR-Spektrum ($cm^{-1}$) | NMR-Spektrum ($DMSO/CD_3OD$) Signale bei ppm |
|---|---|---|---|---|---|---|
| 49 | 2-Furyl | 3-Pyridylmethyl | $OCOCH_3$ | 2aa | 1765, 1655, 1615 | 2,05 (s, 3H), 3,40 (q, 2H), 4,40 (m, 2+2H), 4,80 (dd, 1H), 5,45 (dd, 1H), 5,70 (s, 1H), 6,3 (m, 2H), 7,3 (m, 1H), 7,4 (m, 1H), 7,7 (m, 1H), 8,1 (s, 1H), 8,5 (m, 2H). |
| 50 | p-HO-Phenyl | 2-Methyl-5-pyrimidinyl | $OCOCH_3$ | 2ac | 1760, 1650, 1610 | 2,05 (s, 3H), 2,45 (s, 3H), 3,40 (q, 2H), 4,35 (m, 2H), 4,85 (d, 1H), 5,45 (s, 1H), 5,55 (d, 1H), 6,75 (d, 2H), 7,30 (d, 2H), 8,30 (s, 1H), 9,1 (s, 2H). |
| 51 | p-HO-Phenyl | 5-Aminosulfonyl-2--thienylmethyl | $OCOCH_3$ | 2ad | 1760, 1655, 1610 | 2,05 (s, 3H), 3,45 (m, 2H), 4,35 (m, 2H), 4,60 (s, 2H), 4,85 (d, 1H), 5,40 (s, 1H), 5,55 (d, 1H), 6,75 (d, 2H), 6,9 (d, 1H), 7,25 (d, 2H), 7,35 (d, 1H), 8,05 (s, 1H). |
| 52 | p-HO-Phenyl | 5-Aminosulfonyl-2--thienylmethyl | 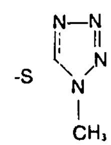 | 2ad | 1765, 1655, 1610 | 3,40 (m, 2H), 3,95 (s, 3H), 4,40 (m, 2+1H), 4,60 (s, 2H), 5,40 (s, 1H), 5,55 (d, 1H), 6,75 (d, 2H), 6,95 (d, 1H), 7,30 (d, 2H), 7,40 (d, 1H), 8,05 (s, 1H). |
| 53 | p-HO-Phenyl | 5-Aminosulfonyl-2--thienylmethyl | $OCOCH_3$ | 2ad | 1760, 1660, 1610 | 3,40 (q, 2H), 4,65 (s, 2H), 4,75 (m, 2H), 5,45 (s, 1H), 5.55 (d, 1H), 6,75 (d, 2H), 6,9 (d, 1H), 7,35 (d, 2H), 7,45 (s, 1H), 8,50 (s, 1H). |
| 54 | p-HO-Phenyl | 5-Aminosulfonyl-2--thienylmethyl | 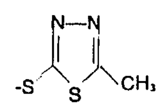 | 2ad | 1760, 1655, 1605 | 2,75 (s, 3H), 3,55 (q, 2H), 4,25 (q, 2H), 4,60 (s, 2H), 4,95 (d, 1H), 5,45 (s, 1H), 5,60 (d, 1H), 6,7 (d, 2H), 6,95 (d, 1H), 7,25 (d, 2H), 7,40 (d, 1H), 8,05 (s, 1H). |
| 55 | 2-Thienyl | 5-Aminosulfonyl-2--thienylmethyl | 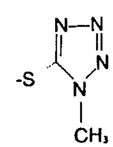 | 2ae | 1760, 1650, 1605 | 3,45 (q, 2H), 4,35 (m, 2H), 4,60 (s, 2H), 4,95 (dd, 1H), 5,50 (m, 1H), 5,65 (s, 1H), 7,0 (m, 3H), 7,4 (m, 2H), 8,05 (s, 1H). |

| Beispiel | A | $(CH_2)_nR_1$ | D | mit der Ureido-carbonsäure des Beispiels I | IR-Spektrum ($cm^{-1}$) | NMR-Spektrum ($DMSO/CD_3OD$) Signale bei ppm |
|---|---|---|---|---|---|---|
| 56 | p-HO-Phenyl | 6-Methylsulfinyl-3--pyridil | 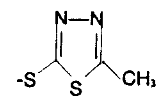 | 2v | 1760, 1660, 1610 | 2,75 (s, 3H), 2,80 (s, 3H), 3,50 (q, 2H), 4,25 (q, 2H), 5,45 (s, 1H), 5,60 (d, 1H), 6,75 (d, 2H), 7,25 (d, 2H), 7,7 (d, 1H), 8,25 (s, 1H), 8,5 (m, 1H), 8,9 (s, 1H). |
| 57 | p-HO-Phenyl | 6-Methylsulfonyl-3--pyridyl | 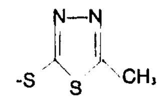 | 2y | 1760, 1655, 1615 | 2,75 (s, 3H), 3,10 (s, 3H), 3,55 (q, 2H), 4,30 (q, 2H), 5,0 (d, 1H), 5,50 (s, 1H), 5,60 (d, 1H), 6,75 (d, 2H), 7,25 (d, 2H), 7,85 (d, 1H), 8,25 (s, 1H), 8,55 (m, 1H), 8,95 (s, 1H). |
| 58 | p-HO-Phenyl | 5-Aminocarbonyl-2--thienyl | 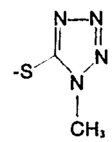 | 2af | 1760, 1660, 1600 | 3,50 (q, 2H), 3,95 (s, 3H), 4,30 (m, 2H), 4,95 (d, 1H), 5,40 (s, 1H), 5,55 (d, 1H), 6,6 (d, 1H), 6,75 (d, 2H), 7,25 (d, 2H), 7,45 (d, 1H), 8,40 (s, 1H). |
| 59 | p-HO-Phenyl | 5-Aminocarbonyl-2--thienyl | $OCOCH_3$ | 2af | 1765, 1665, 1620 | 2,05 (s, 3H), 3,40 (q, 2H), 4,80 (m, 2H), 4,90 (d, 1H), 5,45 (s, 1H), 5,55 (d, 1H), 6,65 (d, 1H), 6,75 (d, 2H), 7,25 (d, 2H), 7,40 (d, 1H), 8,35 (s, 1H). |
| 60 | 2-Thienyl | 5-Aminocarbonyl-2--thienyl | 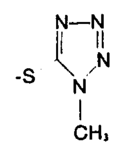 | 2ag | 1760, 1660, 1610 | 3,45 (q, 2H), 3,95 (s, 3H), 4,30 (m, 2H), 4,95 (dd, 1H), 5,50 (dd, 1H), 5,60 (s, 1H), 6,65 (d, 1H), 7,0 (m, 2H), 7,4 (m, 2H), 8,35 (s, 1H). |
| 61 | p-HO-Phenyl | 6-Hydroxy-3-pyridyl | 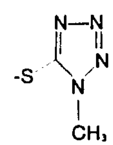 | 2z | 1760, 1660, 1615 | 3,40 (q, 2H), 3,95 (s, 3H), 4,30 (m, 2H), 4,90 (d, 1H), 5,45 (s, 1H), 5,55 (d, 1H), 6,45 (d, 1H), 6,75 (d, 2H), 7,25 (d, 2H), 7,6 (d, 1H), 7,9 (s, 2H), 8,20 (s, 1H). |
| 62 | p-HO-Phenyl | 6-Hydroxy-3-pyridyl | $OCOCH_3$ | 2z | 1765, 1665, 1610 | 2,05 (s, 3H), 3,50 (q, 2H), 4,80 (m, 3H), 5,40 (s, 1H), 5,55 (d, 1H), 6,50 (d, 1H), 6,80 (d, 2H), 7,30 (d, 2H), 7,6 (d, 1H), 7,9 (s, 2H), 8,20 (s, 1H). |

| | | | | | | |
|---|---|---|---|---|---|---|
| 63 | p-HO-Phenyl | 6-Hydroxy-3-pyridyl | 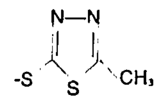 | 2z | 1760, 1660, 1610 | 2,70 (s, 3H), 3,50 (m, 2H), 4,25 (q, 2H), 4,95 (d, 1H), 5,45 (s, 1H), 5,55 (d, 1H), 6,40 (d, 1H), 6,75 (d, 2H), 7,30 (d, 2H), 7,6 (d, 1H), 7,95 (s, 2H), 8,20 (s, 1H). |
| 64 | p-HO-Phenyl | 2-Hydroxy-5--pyrimidinyl | $OCOCH_3$ | 2ah | 1765, 1660, 1615 | 2,05 (s, 3H), 3,45 (m, 2H), 4,85 (m, 2+1H), 5,50 (s, 1H), 5,60 (d, 1H), 6,80 (d, 2H), 7,30 (d, 2H), 8,35 (s, 1H), 8,70 (s, 2H). |
| 65 | p-HO-Phenyl | 2-Hydroxy-5--pyrimidinyl | 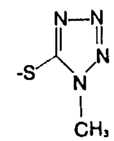 | 2ah | 1760, 1660, 1610 | 3,40 (q, 2H), 3,90 (s, 3H), 4,30 (m, 2H), 4,95 (d, 1H), 5,40 (s, 1H), 5,50 (d, 1H), 6,75 (d, 2H), 7,25 (d, 2H), 8,30 (s, 1H), 8,70 (s, 2H). |
| 66 | p-HO-Phenyl | 2,4-Dihydroxy-5--pyrimidinyl | 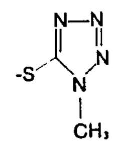 | 2ai | 1760, 1650, 1600 | 3,45 (m, 2H), 3,95 (s, 3H), 4,35 (m, 2H), 4,90 (d, 1H), 5,45 (s, 1H), 5,50 (d, 1H), 6,70 (d, 2H), 7,20 (d, 2H), 8,30 (s, 1H), 8,50 (s, 1H). |
| 67 | p-HO-Phenyl | 2,6-Dihydroxy-4--pyrimidinyl | 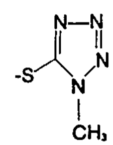 | 2ak | 1760, 1660, 1605 | 3,40 (m, 2H), 3,90 (s, 3H), 4,30 (m, 2H), 4,95 (d, 1H), 5,40 (s, 1H), 5,50 (d, 1H), 6,70 (d, 2H), 7,25 (d, 2H), 8,35 (s, 1H), 8,45 (s, 1H). |
| 68 | p-HO-Phenyl | 2,6-Dihydroxy-4--pyrimidinyl | $OCOCH_3$ | 2ak | 1760, 1655, 1610 | 2,05 (s, 3H), 3,50 (m, 2H), 4,85 (m, 3H), 5,40 (s, 1H), 5,50 (d, 1H), 6,75 (d, 2H), 7,30 (d, 2H), 8,30 (s, 1H), 8,50 (s, 1H). |
| 69 | p-HO-Phenyl | 4,6-Dihydroxy-2--pyrimidinyl | 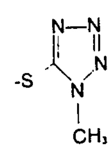 | 2al | 1765, 1660, 1610 | 3,45 (m, 2H), 3,90 (s, 3H), 4,30 (m, 2H), 4,90 (d, 1H), 5,40 (s, 1H), 5,50 (d, 1H), 6,75 (d, 2H), 7,35 (d, 2H), 8,35 (s, 1H), 8,80 (s, 1H). |
| 70 | p-HO-Phenyl | 4,6-Dihydroxy-2--pyrimidinyl | $OCOCH_3$ | 2al | 1760, 1655, 1610 | 2,10 (s, 3H), 3,45 (m, 2H), 4,80 (m, 2H+d, 1H), 5,45 (s, 1H), 5,55 (d, 1H), 6,75 (d, 2H), 7,30 (d, 2H), 8,35 (s, 1H), 8,75 (s, 1H). |

Beispiel 71

Natrium-7-{D-α-[3-‹4-hydroxy-2-(3'-pyridylamino)-5-pyrimidinyl)-ureido]-phenyl-acetamido}-3-methyl-ceph-3-em-4-carboxylat

Eine Suspension von 2,66 g Cefalexin-Monohydrat (0,0073 Mol) in 80 ml Tetrahydrofuran und 20 ml Wasser wird mit Triäthylamin unter Eiskühlung in Lösung gebracht.

1,48 g (0,0073 Mol) 5-Amino-4-hydroxy-2-(3'-pyridylamino)-pyrimidin werden in Tetrahydrofuran gelöst, mit 1 ml Triäthylamin versetzt und unter Eiskuühlung zu 750 mg Phosgen, gelöst in 18 ml Tetrahydrofuran, getropft. Das entstandene Gemisch wird im Vakuum auf 40 ml eingeengt und unter Eiskühlung zu der oben hergestellten Lösung getropft. Dabei wird der pH mit Hilfe von Triäthylamin auf 7,5 gehalten. Die erhaltene Lösung wird bei 5°C eine Stunde und bei Raumtemperatur eine weitere Stunde gerührt. Nach dieser Zeit wird Tetrahydrofuran im Vakuum abgezogen, der Rückstand mit 20 ml Wasser verdünnt und zweimal mit Essigester ausgeschüttelt. Dann wird die wässrige Phase mit Essigester überschichtet und unter Kühlen und Rühren langsam ein pH-Wert von 2,9 eingestellt. Die Essigesterschicht wird abgetrennt, die wässrige Phase noch einmal mit Essigester ausgeschüttelt, die beiden organischen Phasen vereinigt und das Lösemittel im Vakuum abdestilliert. Auf übliche Weise wird das Natriumsalz hergestellt.
Ausbeute: 2,94 g (68%);
IR-Spektrum: 1765, 1655, 1610, 1540 $cm^{-1}$;
NMR-Spektrum ($DMSO+CD_3OD$) Signale bei ppm: 2,0 (s, 3H), 3,40 (q, 2H), 5,05 (d, 1H), 5,45 (s, 1H), 5,65 (d, 1H), 7,45 (m, 6H), 8,3 (m, 3H), 8,75 (s, 1H).

Setzt man wie im Beispiel III/71 beschrieben, das p-Hydroxy-Analoge des Cefalexins ein, so erhält man folgende Cephalosporine:

Beispiel 72

Natrium-7-{D-α-[3-‹4-hydroxy-2-(3'-pyridylmethylamino)-5-pyrimidinyl›-ureido]-p-hydroxyphenyl-acetamido}-3-methyl-ceph-3-em-4-carboxylat

Mit dem Umsetzungsprodukt von 5-Amino-4-hydroxy-2-(3'-pyridylmethylamino)-pyrimidin mit Phosgen:
Ausbeute: 71%;
IR-Spektrum: 1765, 1660, 1610, 1540 $cm^{-1}$;
NMR-Spektrum ($DMSO+CD_3OD$) Signale bei ppm: 2,0 (s, 3H), 3,4 (q, 2H), 4,5 (s, 2H), 4,95 (d, 1H), 5,45 (s, 1H), 5,60 (d, 1H), 6,8 (d, 2H), 7,3 (m, 3H), 7,7 (m, 1H), 8,1 (s, 1H), 8,5 (m, 2H).

Beispiel 73

Natrium-7-{D-α-[3-‹4-hydroxy-2-(2'-thienylmethylamino)-5-pyrimidinyl›-ureido]-p-hydroxyphenyl-acetamido}-3-methyl-ceph-3-em-4-carboxylat

Mit dem Umsetzungsprodukt von 5-Amino-4-hydroxy-2-(3'-pyridylmethylamino)-pyrimidin mit Phosgen.
Ausbeute: 64%;
IR-Spektrum: 1770, 1660, 1610, 1550 $cm^{-1}$;
NMR-Spektrum ($DMSO+CD_3OD$) Signale bei ppm: 2,05 (s, 3H), 3,4 (q, 2H), 4,5 (s, 2H), 4,95 (d, 1H), 5,45 (s, 1H), 5,65 (d, 1H), 6,70 (d, 2H), 6,85 (m, 2H), 7,25 (d, 2H), 7,35 (m, 1H), 8,05 (s, 1H).

Beispiel 74

Natrium-7-{D-α-[3-‹4-hydroxy-2-(2'-furylmethylamino)-5-pyrimidinyl›-ureido]-p-hydroxyphenyl-acetamido}-3-methyl-ceph-3-em-4-carboxylat

Mit dem Umsetzungsprodukt von 5-Amino-4-hydroxy-2-(2'-furylmethylamino)-pyrimidin mit Phosgen.
Ausbeute: 71%;
IR-Spektrum: 1765, 1655, 1610, 1545 $cm^{-1}$;
NMR-Spektrum ($DMSO+CD_3OD$) Signale bei ppm: 2,0 (s, 3H), 3,35 (q, 2H), 4,4 (s, 2H), 4,95 (d, 1H), 5,4 (s, 1H), 5,65 (d, 1H), 6,3 (m, 2H), 6,7 (d, 2H), 7,2 (d, 2H), 7,5 (s, 1H), 8.05 (s, 1H).

Beispiel 75

Natrium-7-{D-α-[3-‹4-hydroxy-2-(3'-pyridylamino)-5-pyrimidinyl›-ureido]-phenylacetamido}-3-acetoxymethyl-ceph-3-em-4-carboxylat

2,03 g (0,01 Mol) 5-Amino-4-hydroxy-2-(3'-pyridylamino)-pyrimidin werden in Tetrahydrofuran gelöst, mit 1,35 ml Triäthylamin versetzt und zu einer Lösung von 1,0 g Phosgen in Tetrahydrofuran unter Eiskühlung getropft. Das entstandene Gemisch wird wie in Beispiel III/71 beschrieben mit 4,25 g (0,01 Mol) Cefaloglycin-dihydrat umgesetzt. Die Aufarbeitung erfolgt analog Beispiel III/71, nur wird das gewünschte Endprodukt bei pH 3,0 aus Wasser ausgefällt.
Ausbeute: 3,16 g Natriumsalz (48,5%);
NMR-Spektrum ($DMSO+CD_3OD$) Signale bei ppm: 2,05 (s, 3H), 3,45 (q, 2H), 4,85 (m, 3H), 5,45 (s, 1H), 5,65 (d, 1H), 7,45 (m, 6H), 8,25 (m, 3H), 8,75 (s, 1H).

Setzt man wie in Beispiel III/75 beschrieben, Cefaloglycindihydrat ein, so erhält man folgende Cephalosporine:

Beispiel 76

Natrium-7-{D-α-[3-‹4-hydroxy-2-(2'-thienylmethylamino)-5-pyrimidinyl›-ureido]-phenyl-acetamido}-3-acetoxymethyl-ceph-3-em-4-carboxylat

Mit dem Umsetzungsprodukt von 5-Amino-4-hydroxy-2-(2'-thienylmethylamino)-pyrimidin mit Phosgen.
Ausbeute: 62,5%;

IR-Spektrum: 1765, 1655, 1610, 1540 $cm^{-1}$; NMR-Spektrum ($DMSO+CD_3OD$) Signale bei ppm: 2,05 (s, 3H), 3,4 (q, 2H), 4,45 (s, 2H), 4,85 (m, 3H), 5,45 (s, 1H), 5,60 (d, 1H), 6,85 (m, 2H), 7,45 (m, 6H), 8,1 (s, 1H).

Beispiel 77

Natrium-7-{D-α-[‹4-hydroxy-2-(2'-furylmethyl-amino)-5-pyrimidinyl›-preido]-phenyl-acetamido}-3-acetoxymethyl-ceph-3-em-4-carboxylat

Mit dem Umsetzungsprodukt von 5-Amino-4--hydroxy-2-(2'-furylmethylamino)-pyrimidin mit Phosgen.
Ausbeute: 64%;
IR-Spektrum: 1770, 1660, 1610, 1550 $cm^{-1}$;
NMR-Spektrum ($DMSO+CD_3OD$) Signale bei ppm: 2,05 (s, 3H), 3,45 (q, 2H), 4,45 (s, 2H), 4,85 (m, 2 + 1H), 5,45 (s, 1H), 5,65 (d, 1H), 6,3 (m, 2H), 7,5 (m, 6H), 8,05 (s, 1H).

Beispiel 78

Natrium-7-{D-α-[4-‹4-hydroxy-2-(4'-methyl-2'--thiazolylmethylamino)-5-pydimidinyl›-ureido]-p--hydroxy-phenyl-acetamido}-3-[(1-methyl-tetrazol-5-yl)-thiomethyl]ceph-3-em-4-carboxylat

Herstellung analog Beispiel III/71 ausgehend von 405 mg 7-[D-α-Amino-(p-hydroxyphenyl-acetamido)]-3-[(1-methyl-tetrazol-5-yl)-thiomethyl]--ceph-3-em-4-carbonsäure (0,001 Mol) sowie dem Phosgen-Umsetzungsprodukt von 240 mg 5-Amino-4-hydroxy-2-(4'-methyl-2'-thiazolylmethylamino)-pyrimidin (0,001 Mol).

Bei der Aufarbeitung wird das entstandene Cephalosporin bei pH 2,9 aus Wasser ausgefällt, abgesaugt, getrocknet und in bekannter Weise in das Natriumsalz überführt.
Ausbeute: 315 mg (45,5%);
IR-Spektrum: 1765, 1655, 1610, 1540 $cm^{-1}$;
NMR-Spektrum ($DMSO+CD_3OD$) Signale bei ppm: 2,45 (s, 3H), 3,5 (q, 2H), 3,9 (s, 3H), 4,35 (q, 2H), 4,85 (d, 1H), 5,45 (s, 1H), 5,65 (d, 1H), 6,15 (s, 1H), 6,85 (d, 2H), 7,35 (d, 2H), 8,05 (s, 1H).

Analog wurde hergestellt:
Natrium-7-{D-α-[3-‹4-hydroxy-2-(3'-pyridylmethylamino)-5-pyrimidinyl›-ureido]-2-furylacetaamido}-3-acetoxymethyl-ceph-3-em-4-carboxylat
Natrium-7-{D-α-[3-‹4-hydroxy-2-(3'-pyridylmethylamino)-5-pyimidinyl›-ureido]-2-thienylacetamido}-3-acetoxymethyl-ceph-3-em-4-carboxylat
Natrium-7-{D-α-[3-‹4-hydroxy-2-(3'-pyridylmethylamino)-5-pyrimidinyl›-ureido]-3-furylacetamido}-3-acetoxymethyl-ceph-3-em-4-carboxylat

Natrium-7-{D-α-[3-‹4-hydroxy-2-(3'-pyridylmethylamino)-5-pyrimidinyl)-ureido]-phenylacetamido}-3-acetoxymethyl-ceph-3-em-4-carboxylat.

Beispiel 79

Natrium-7-{D-α-[3-‹4-hydroxy-2-(5'-methyl-2'--thiazolylamino)-5-pyrimidinyl›-ureido]-p-hydroxy-phenylacetamido}-3-[(1-methyl-tetrazol-5--yl)-thiomethyl]-ceph-3-em-4-carboxylat

1,14 g (0,005 Mol) 5-Amino-4-hydroxy-2-(5'-methyl-2'-thiadiazolyl-amino)-pyrimidin werden in 50 ml Tetrahydrofuran suspendiert und bis zur Lösung mit Trimethylsilyldiäthylamin behandelt. Es wird unter Stickstoff von etwas Unlöslichem abfiltriert, das Tetrahydrofuran abdestilliert und im Hochvakuum zur Trockne eingeengt. Das zurückbliebende Produkt wird in 30 ml Tetrahydrofuran gelöst und unter Eiskühlung zu einer Lösung von 500 mg Phosgen in Tetrahydrofuran getropft. Anschliessend wird zur Entfernung von nicht umgesetztem Phosgen Stickstoff durch die Lösung geblasen.

Die weitere Umsetzung mit 7-[D-α-Amino-p--hydroxyphenylacetamido]-3-(1-methyl-tetrazol--5-yl)-thiomethyl]-ceph-3-em-4-carbonsäure erfolgt analog Beispiel III/49.
Ausbeute: 980 mg (28,5%) Natriumsalz;
IR-Spektrum: 1765, 1665, 1605, 1545 $cm^{-1}$;
NMR-Spektrum ($DMSO+CD_3OD$) Signale bei ppm: 2,45 (s, 3H), 3,5 (q, 2H), 3,9 (s, 3H), 4,35 (q, 2H), 4,85 (d, 1H), 5,40 (s. 1H), 5,60 (d, 1H), 6,80 (d, 2H), 7,25 (d, 2H), 8,05 (s, 1H).

Analog wurde hergestellt:
Natrium-7-{D-α-[3-‹4-hydroxy-2-(5'-methyl-2'--triazolyl-amino)-5-pymidinyl›-ureido]-p-hydroxy--phenylacetamido}-3-[(1-methyltetrazol-5-yl)--thiomethyl]-ceph-3-em-4-carboxylat

Beispiel 80

Natrium-7-{D-α-[3-‹4-hydroxy-2-(2'-thienylmethylamino)-5-pyrimidinyl›-ureido]-p-hydroxy-phenylacetamido}-3-acetoxymethyl-ceph-3-em-4-carboxylat

Dieses Cephalosporin wird analog Beispiel III/71 hergestellt. Man geht aus von 810 mg 7--(D-α-Amino-p-hydroxy-phenylacetamido)-3-acetoxy-methylceph-3-em-4-carboxylat (0,002 Mol) sowie dem Umsetzungsprodukt von 440 mg 5-Amino-4-hydroxy-2-(2'-thienylmethylamino)-pyrimidin (0,002 Mol) mit 200 mg Phosgen.

Die Aufarbeitung erfolgte analog Beispiel III/49.
Ausbeute: 615 mg Natriumsalz (44,5%);
IR-Spektrum: 1765, 1660, 1540 $cm^{-1}$;
NMR-Spektrum ($DMSO+CD_3OD$) Signale bei ppm: 2,05 (s, 3H), 3,35 (q, 2H), 4,45 (s, 2H), 4,80 (m, 2 + 1H), 5,45 (s, 1H), 5,65 (d, 1H), 6,7 (d, 2H), 6,8 (m, 2H), 7,30 (d, 2H), 7,45 (m, 1H), 8,05 (s, 1H).

Analog wurden hergestellt:
Natrium-7-{D-α-[3-‹4-hydroxy-2-(2'furylmethylamino)-5-pyrimidinyl›-ureido]-m,p-dihydroxy--phenylacetamido}-3-acetoxymethyl-ceph-3-em--4-carboxylat
Natrium-7-{D-α-[3-‹4-hydroxy-2-(2'-furylmethyl-

amino)-5-pyrimidinyl›-ureido]-m,p-dihydroxy-phenylacetamido}-3-carbamoyloxymethyl-ceph-3-em-4-carboxylat

Natrium-7-{D-α-[3-‹4-hydroxy-2-(2'-furylmethylamino)-5-pyrimidinyl›-ureido]-m,p-dihydro-phenylacetamido}-3-[(1-methyl-tetrazol-5-yl)-thiomethyl]-ceph-3-em-4-carboxylat

Natrium-7-{D-α-[3-‹4-hydroxy-2-(3'-furylmethylamino)-5-pyrimidinyl›-ureido]-p-hydroxy-phenylacetamido}-3-acetoxymethyl-ceph-3-em-4-carboxylat

Natrium-7-{D-α-[3-‹4-hydroxy-2-(2'-furylmethylamino)-5-pyrimidinyl›-ureido]-m,p-dihydroxy-phenylacetamido}-3-[(1-methyl-tetrazol-5-yl)-thiomethyl]-ceph-3-em-4-carboxylat

Natrium-7-{D-α-[3-‹4-hydroxy-2-(5'-nitro-2'-furylmethylamino)-5-pyrimidinyl)-ureido]-p-hydroxy-phenylacetamido}-3-[(1-methyltetrazol-5-yl)-thiomethyl]-ceph-3-em-4-carboxylat

Beispiel 81

Natrium-7-{D-α-[3-‹4-hydroxy-2-(5'-pyrimidinyl-amino)-5-pyrimidinyl›-ureido]-p-hydroxy-phenylacetamido}-3-[(1-methyltetrazol-5-yl)-thiomethyl]-ceph-3-em-4-carboxylat

Herstellung analog Beispiel III/42, ausgehend von 5-Amino-4-hydroxy-2-(5'-pyrimidinylamino)-pyrimidin sowie dem Cephalosporinderivat des Beispiels III/50.
Ausbeute an Natriumsalz: 260 mg (35,5%);
IR-Spektrum: 1765, 1655, 1610, 1550 $cm^{-1}$;
NMR-Spektrum ($DMSO+CD_3OD$) Signale bei ppm: 3,5 (q, 2H), 3,9 (s, 3H), 4,40 (q, 2H), 4,85 (d, 1H), 5,45 (s, 1H), 5,65 (d, 1H), 6,85 (d, 2H), 7,35 (d, 2H), 8,0 (s, 1H), 8,3 (breites s, 2H), 8,8 (s, 1H).

Beispiel 82

Natrium-7-{D-α-[3-‹4-hydroxy-2-(3'-pyridylamino)-5-pyrimidinyl›-ureido]-p-hydroxy-phenylacetamido}-3-[(1,2,3-triazol-4-yl)-thiomethyl]-ceph-3-em-4-carboxylat

In 10 ml einer Phosphorsäure-Pufferlösung von pH 6,3 löst man 680 mg des in Beispiel III/1 erhaltenen Cephalosporinderivats. Zu dieser Lösung gibt man 100 mg 4-Mercapto-1,2,3-triazol und erhitzt 6 Stunden unter Stickstoff auf 70°C, wobei der pH-Wert auf 6,0-6,5 gehalten wird. Nach dieser Zeit wird die Reaktionsflüssigkeit abgekühlt und mit Essigester zweimal ausgeschüttelt. Anschliessend wird unter Kühlen 2n Salzsäure bis zu einem pH-Wert von 2,9 zugesetzt. Das ausgefallene Produkt wird abgesaugt, mit wenig Wasser gewaschen und getrocknet. Der Rückstand wird auf die übliche Weise in das Natriumsalz überführt.
Man erhält 460 mg (64%);
IR-Spektrum: 1765, 1660, 1615, 1545 $cm^{-1}$;
NMR-Spektrum ($DMSO+CD_3OD$) Signale bei ppm: 3,5 (q, 2H), 4,25 (q, 2H), 4,90 (d, 1H), 5,45 (s, 1H), 5,60 (d, 1H), 6,8 (d, 2H), 7,25 (d, 2H), 7,4 (m, 1H), 7,95 (s, 1H), 8,25 (m, 3H), 8,75 (s, 1H).

Beispiel 83

Natrium-7-{D-α-[3-‹4-hydroxy-2-(3'-pyridylmethylamino)-5-pyrimidinyl›-ureido]-p-hydroxy-phenylacetamido}-3-[(2-methyl-1,3,4-thiadiazol-5-yl)-thiomethyl]-ceph-3-em-4-carboxylat

Herstellung analog Beispiel III/82.
Man geht aus von 685 mg des entsprechenden Acetoxycephalosporinderivates (0,001 Mol) und setzt dieses mit 135 mg 5-Mercapto-2-methyl-1,3,4-thiadiazol um.
Ausbeute: 455 mg Natriumsalz (65%);
IR-Spektrum: 1765, 1670, 1615, 1550 $cm^{-1}$;
NMR-Spektrum ($DMSO+CD_3OD$) Signale bei ppm: 2,7 (s, 3H), 3,55 (q, 2H), 4,45 (s, 2H), 4,95 (d, 1H), 5,45 (s, 1H), 5,65 (d, 1H), 6,85 (d, 2H), 7,35 (m, 3H), 7,7 (m, 1H), 8,1 (s, 1H), 8,45 (m, 2H).

Beispiel 84

Natrium-7-{D-α-[3-‹4-hydroxy-2-(3'-pyridylmethylamino)-5-pyrimidinyl›-ureido]-2-furylacetamido}-3-[(1-methyl-tetrazol-5-yl)-thiomethyl]-ceph-3-em-4-carboxylat

Herstellung analog Beispiel III/82.
Aus Natrium-7-{D-α-[3-‹4-hydroxy-2-(3'-pyridylmethylamino)-5-pyrimidinyl›-ureido]-2-furylacetamido}-3-acetoxymethyl-ceph-3-em-4-carboxylat durch Umsetzung mit 1-Methyl-5-mercapto-tetrazol in 66,5%-iger Ausbeute.

Beispiel 85

Natrium-7-{D-α-[3-‹4-hydroxy-2-(2'-thienylmethylamino)-5-pyrimidinyl›-ureido]-p-hydroxy-phenylacetamido}-3-[(1,3,4-thiadiazol-2-yl)-thiomethyl]-ceph-3-em-4-carboxylat

Herstellung analog Beispiel III/82.
Durch Umsetzung des in Beispiel III/80 erhaltenen Cephalosporinderivates mit 2-Mercapto-1,3,4-thiadiazol in 71%iger Ausbeute.

Beispiel 86

Natrium-7-{D-α-[3-‹4-hydroxy-2-(2'-thienylmethylamino)-5-pyrimidinyl›-ureido]-p-hydroxy-phenylacetamido}-3-[(2-acetylamino-thiadiazol-5-yl)-thiomethyl]-ceph-3-em-4-carboxylat

Analog Beispiel III/82 aus 690 mg des Cephalosporin-derivates des Beispiels III/80 (0,001 Mol) und 175 mg 2-Acetylamino-5-mercapto-1,3,4-thiadiazol.
Ausbeute: 480 mg Natriumsalz (59%);
IR-Spektrum: 1765, 1655, 1620, 1540 $cm^{-1}$;
NMR-Spektrum $DMSO+CD_3OD$) Signale bei ppm: 2,4 (s, 3H), 3,70 (q, 2H), 4,25 (q, 2H), 4,45 (s, 2H), 4,95 (d, 1H), 5,45 (s, 1H), 5,65 (d, 1H), 6,7-7,45 (m, 7H), 8,05 (s, 1H).

Beispiel 87

Natrium-7-{D-α-[3-‹4-hydroxy-2-(2'-thienylmethylamino)-5-pyrimidinyl›-ureido]-p-hydroxy-phenylacetamido}-3-[(2-methylamino-thiadiazol-5-yl)-thiomethyl]-ceph-3-em-4-carboxylat

Analog Beispiel III/53 aus demselben Cephalosporin-Derivat. Durch Umsetzung mit 2-Methylamino-5-mercapto-1,3,4-thiadiazol in 61%iger Ausbeute.

Beispiel 88

Natrium-7-{D-α-[3-‹4-hydroxy-2-(2'-thienylmethylamino)-5-pyrimidinyl›-ureido]-p-hydroxy-phenylacetamido}-3-[(1,2,3-triazol-4-yl)-thiomethyl]-ceph-3-em-4-carboxylat

Analog Beispiel III/53 aus demselben Cephalosporin-Derivat. Durch Umsetzung mit 4-Mercapto-1,2,3-triazol in 68%iger Ausbeute.

Analog zu Beispiel III/53 werden, ausgehend von Natrium-7-{D-α-[3-‹4-hydroxy-2-(2'-furylmethylamino)-5-pyrimidinyl›-ureido]-p-hydroxy-phenylacetamido}-3-acetoxymethyl-ceph-3-em-4-carboxylat folgende Cephalosporine hergestellt:

Beispiel 89

Natrium-7-{-α-[3-‹4-hydroxy-2-(2'-furylmethylamino)-5-pyrimidinyl›-ureido]-p-hydroxy-phenylacetamido}-3-[(tetrazol-5-yl)-thiomethyl]-ceph-3-em-4-carboxylat

Mit 5-Mercapto-tetrazol in 57%iger Ausbeute.
IR-Spektrum: 1770, 1655, 1615, 1545 $cm^{-1}$;
NMR-Spektrum ($DMSO+CD_3OD$) Signale bei ppm: 3,45 (q, 2H), 4,45 (m, 4H), 4,90 (d, 1H), 5,45 (s, 1H), 5,60 (d, 1H), 6,3 (m, 2H), 6,7 (d, 2H), 7,2 (d, 2H), 7,5 (s, 1H), 8,05 (s, 1H).

Beispiel 90

Natrium-7-{D-α-[3-‹4-hydroxy-2-(2'-furylmethylamino)-5-pyrimidinyl›-ureido]-p-hydroxy-phenylacetamido}-3-[(1,2,4-thiadiazol-5-yl)-thiomethyl]-ceph-3-em-4-carboxylat

Durch Umsetzung mit 5-Mercapto-1,2,4-thiadiazol in 64,5%iger Ausbeute.

Beispiel 91

Natrium-7-{D-α-[3-‹4-hydroxy-2-(2'-furylmethylamino)-5-pyrimidinyl›-ureido]-p-hydroxy-phenylacetamido}-3-[(2-acetylamino-1,3,4-thiadiazol-5-yl)-thiomethyl]-ceph-3-em-4-carboxylat

Durch Umestzung mit 2-Acetylamino-1,3,4-thiadiazol in 56%iger Ausbeute.
NMR-Spektrum ($DMSO+CD_3OD$) Signale bei ppm: 2,45 (s, 3H), 3,70 (q, 2H), 4,3-4,4 (m, 4H, teilweise durch Lösungsmittel verdeckt), 4,95 (d, 1H), 5,45 (s, 1H), 5,60 (d, 1H), 6,3 (m, 2H), 6,7 (d, 2H), 7,2 (d, 2H), 7,5 (s, 1H), 8,05 (s, 1H).

Beispiel 92

Natrium-7-{D-α-[3-‹4-hydroxy-2-(2'-furylmethylamino)-5-pyrimidinyl›-ureido]-p-hydroxy-phenylacetamido}-3-[(1,3,4-triazol-2-yl)-thiomethyl]-ceph-3-em-4-carboxylat

Durch Umsetzung mit 2-Mercapto-1,3,4-triazol in 66%iger Ausbeute.
NMR-Spektrum ($DMSO+CD_3OD$) Signale bei ppm: 3,50 (q, 2H), 4,4 (m, 4H), 4,90 (d, 1H), 5,40 (s, 1H), 5,65 (d, 1H), 6,35 (m, 2H), 6,7 (d, 2H), 7,2 (d, 2H), 7,55 (s, 1H), 8,05 (s, 1H), 8,35 (s, 1H).

Beispiel 93

Natrium-7-{D-α-[3-‹4-hydroxy-2-(2'-furylmethylamino)-5-pyrimidinyl›-ureido]-p-hydroxy-phenylacetamido}-3-[(2-dimethylamino-1,3,4-thiadiazol-5-yl)-thiomethyl]-ceph-3-em-4-carboxylat

Durch Umsetzung mit 2-Dimethylamino-5-mercapto-1,3,4-thiadiazol in 60%iger Ausbeute.
NMR-Spektrum ($DMSO+CD_3OD$) Signale bei ppm: 3,05 (d, 6H), 3,50 (q, 2H), 4,35 (q, 2H), 4,45 (s, 2H), 5,0 (d, 1H), 5,5 (s, 1H), 5,70 (d, 1H), 6,30 (m, 2H), 6,7 (d, 2H), 7,2 (d, 2H), 7,55 (s, 1H), 8,05 (s, 1H).

Beispiel 94

Natrium-7-{D-α-[3-‹4-hydroxy-2-(2'-furylmethylamino)-5-pyrimidinyl›-ureido]-p-hydroxy-phenylacetamido}-3-[(2-methyl-1,3,4-oxadiazol-5-yl)-thiomethyl]-ceph-3-em-4-carboxylat

Durch Umsetzung mit 2-Methyl-5-mercapto-1,3,4-oxadiazol in 63,5%iger Ausbeute.
NMR-Spektrum ($DMSO+CD_3OD$) Signale bei ppm: 2,45 (s, 3H), 3,6 (q, 2H), 4,2 (2H), 4,45 (s, 2H), 4,95 (d, 1H), 5,45 (s, 1H), 5,60 (d, 1H), 6,35 (m, 2H), 6,8 (d, 2H), 7,25 (d, 2H), 7,5 (s, 1H), 8,05 (s, 1H).

Beispiel 95

Pivaloyloxymethyl-7-{D-α-[3-‹4-hydroxy-2-(3'-pyridylamino)-5-pyrimidinyl›-ureido]-p-hydroxy-phenylacetamido}-3-[1-methyl-tetrazol-5-yl)-thiomethyl]-ceph-3-em-4-carboxylat

Man rührt eine Lösung von 965 mg (0,0013 Mol) des Natriumsalzes des Beispiels III/2 und 325 mg Pivaloyloxymethyljodid in 15 ml Dimethylformamid während einer Stunde bei Raumtemperatur. Anschliessend gibt man 50 ml Essigsäureäthylester und 50 ml 0,1 m-Natriumhydrogencarbonat-Lösung zu. Die Essigesterschicht wird dann nacheinander mit Wasser, verdünnter Salzsäure, Magnesiumsulfat getrocknet und im Vakuum zur Trockne eingeengt. Der Rückstand wird mit wasserfreiem Äther verrührt und abgesaugt.
Ausbeute: 710 mg (66%);
IR-Spektrum: 1775, 1735;
NMR-Spektrum ($CDCl_3+CD_3OD$) Signale bei

ppm: 1,10 (s, 9H), 3,6 (m, 2H), 4,0 (s, 3H), 4,5 (m, 2H), 4,95 (d, 1H), 5,5 (s, 1H), 5,75 (d, 1H), 5,85 (dd, 2H), 6,85 (d, 2H), 7,3 (d, 2H), 7,45 (m, 1H), 8,3 (m, 3H), 8,75 (s, 1H).

Analog wurde hergestellt:
Pivaloyloxlmethyl-7-{D-α-[3-‹4-hydroxy-2-(3'-pyridylmethylamino)-5-pyrimidinyl›-uredio]-p-hydroxy-phenylacetamido}-3-[1-methyl-tetrazol-5-yl)-thiomethyl]-ceph-3-em-4-carboxylat.

Beispiel 96

Pivaloyloxymethyl-7-{D-α-[3-‹4-hydroxy-2-(2'-thienylmethylamino-5-pyrimidinyl›-ureido]-p-hydroxy-phenylacetamido}-3-[(1-methyl-tetrazol-5-yl)-thiomethyl]-ceph-3-em-4-carboxylat

Herstellung analog Beispiel III/6, ausgehend von 3,75 g des Natriumsalzes des Beispiels III/19, das mit 1,2 g Pivaloyloxy-methyljodid umgesetzt wurde.
Ausbeute: 2,79 g (68%);
IR-Spektrum: 1770, 1740 $cm^{-1}$;
NMR-Spektrum ($CDCl_3+CD_3OD$) Signale bei ppm: 1,10 (s, 9H), 3,55 (q, 2H), 3,95 (s, 3H), 4,45 (m, 4H), 4,95 (d, 1H), 5,55 (s, 1H), 5,65 (d, 1H), 5,8 (dd, 2H), 6,65-7,35 (m, 7H), 8,05 (s, 1H).
Analog wurde hergestellt:
Propionyloxy-1-äthyl-1-(7-{D-α-[‹4-hydroxy-2-(2'-thienylmethylamino-5-pyrimidinyl›-uredio]-p-hydroxy-phenylacetamido}-3-[(1-methyl-tetrazol-5-yl)-thiomethyl]-ceph-3-em-4-carboxylat.

Beispiel 97

Pivaloyloxymethyl-7-{D-α-[3-‹4-hydroxy-2-(2'-furylmethylamino-5-pyrimidinyl)-ureido]-p-hydroxy-phenylacetamido}-3-[(1-methyl-tetrazol-5-yl)-thiomethyl]-ceph-3-em-4-carboxylat

Herstellung analog Beispiel III/66, ausgehend von 735 mg des Natriumsalzes des Beispiels III/21, das mit 220 mg Pivaloyloxy-methyljodid umgesetzt wurde.
Ausbeute: 500 mg (61%);
IR-Spektrum: 1770, 1740 $cm^{-1}$;
NMR-Spektrum ($CDCl_3+CD_3OD$) Signale bei ppm: 1,05 (s, 9H), 3,55 (q, 2H), 3,95 (s, 3H), 4,45 (m, 4H), 4,95 (d, 1H), 5,55 (s, 1H), 5,65 (d, 1H), 5,75 (dd, 2H), 6,3 (m, 2H), 6,75 (d, 2H), 7,35 (d, 2H), 7,45 (s, 1H), 8,05 (s, 1H).
Analog wurde hergestellt:
Propionyloxy-1-äthyl-1-(7-{D-α-[‹4-hydroxy-2-(2'-furylmethylamino-5-pyrimidinyl›-ureido]-p-hydroxy-phenylacetamido}-3-[(1-methyl-tetrazol-5-yl)-thiomethyl]-ceph-3-em-4-carboxylat, Pivaloyloxymethyl-7-{D-α-[‹4-hydroxy-2-(2'-furylmethylamino-5-pyrimidinyl›-ureido]-p-hydroxy-phenylacetamido}-3-acetoxymethyl-ceph-3-em-4-carboxylat.

Beispiel 98

7-{D-α-[3-‹4-Hydroxy-2-(3'-pyridylmethylamino)-5-pyrimidinyl›-ureido]-p-hydroxy-phenylacetamido}-3-[(4'-aminocarbonylpyridino)-methyl]-ceph-3-em-4-carboxylat.

Ein Gemisch von 2mMol des Cephalosporins des Beispiels 47 sowie 2,5 mMol Pyridin-carboxamid, 4 g Kaliumthiocyanat und 10 ml Wasser werden 8 Stunden lang auf 50°C erhitzt. Die erhaltene Lösung wird über eine mit dem Ionenaustauscherharz Amberlite XAD-2 gefüllte Säule gegeben, die zunächst mit Wasser, anschliessend mit einem 7:3 Gemisch Wasser/Methanol eluiert wird. Aus den Fraktionen, die das gewünschte Produkt enthalten, wird Methanol im Vakuum abdestilliert und die Lösung gefriergetrocknet.
NMR-Spektrum ($D_2O$): 3,55 (m, 2H), 4,5 (s, 2H), 5,1 (d, 1H), 5,4 (q, 2H), 5,7 (s, 1H), 5,8 (d, 1H), 6,8 (d, 2H), 7,4 (m,2+1H), 7,7 (m, 1H), 8,15 (s, 1H), 8,3 (m, 2H), 8,5 (m, 2H), 9,0 (m, 2H).
Analog wurde hergestellt:
7-{D-α-[3-‹4-Hydroxy-2-(2'-thienylmethylamino)-5-pyrimidinyl›-ureido]-p-hydroxy-phenylacetamido}-3-[(pyridino)-methyl]-ceph-3-em-4-carboxylat
7-{D-α-[3-‹4-Hydroxy-2-(2'-thienylmethylamino)-5-pyrimidinyl›-ureido]-p-hydroxy-phenylacetamido}-3-[(4'-aminocarbonyl-pyridino)-methyl]-ceph-3-em-4-carboxylat
7-{D-α-[3-‹4-Hydroxy-2-(2'-furylmethylamino)-5-pyrimidinyl›-ureido]-p-hydroxy-phenylacetamido}-3-[(4'-aminocarbonyl-pyridino)-methyl]-ceph-3-em-4-carboxylat.

Beispiel 99

Natrium-7-{D-α-[‹4-hydroxy-2-(2'-methyl-5'-pyrimidinyl-methylamino)-5-pyrimidinyl›-ureido]-p-hydroxy-phenylacetamido}-3-acetoxymethyl-ceph-3-em-4-carboxylat

Synthese analog Beispiel III/1 ausgehend von 7-Amino-3-acetoxymethyl-ceph-3-em-4-carbonsäure-benzhydrylester sowie der Ureidocarbonsäure des Beispiels I/2am in 52%iger Ausbeute.
IR-Spektrum: 1760, 1660, 1610, 1540 $cm^{-1}$;
NMR-Spektrum ($DMSO+CD_3OD$) Signale bei ppm: 2,05 (s, 3H), 3,45 (q, 2H), 4,40 (s, 2H), 4,80 (m, 3H), 5,55 (s, 1H), 5,60 (d, 1H), 6,70 (d, 2H), 7,25 (d, 2H), 8,05 (s, 1H), 8,65 (s, 2H).

Beispiel 100

Natrium-7-{D-α-‹4-hydroxy-2-(2'-methyl-5'-pyrimidinylmethylamino)-5-pyrimidinyl›-ureido]-p-hydroxy-phenylacetamido}-3-[(1-methyl-tetrazol-5-yl)-thiomethyl]-ceph-3-em-4-carboxylat

Synthese analog Beispiel III/1 ausgehend von der Ureidocarbonsäure des Beispiels I/2am sowie 7-Amino-3-[(1-methyl-tetrazol-5-yl)-thiomethyl]-ceph-3-em-4-carbonsäurebenzhydrylester.
Ausbeute an Natriumsalz 58%;
IR-Spektrum: 1760, 1660, 1615 $cm^{-1}$;
NMR-Spektrum ($DMSO+CD_3OD$) Signale bei

ppm: 3,50 (q, 2H), 3,90 (s, 3H), 4,2-4,5 (m, 2+2H), 4,90 (d, 1H), 5,50 (s, 1H), 5,60 (d, 1H), 6,75 (d, 2H), 7,30 (d, 2H), 8,05 (s, 1H), 8,65 (s, 2H).

IV Darstellung pharmazeutischer Anwendungsformen

Die Verbindungen der allgemeinen Formeln I und I' lassen sich in die üblichen pharmazeutischen Anwendungsformen, wie Tabletten, Dragées, Kapseln oder Ampullen einarbeiten. Die Einzeldosis beträgt bei Erwachsenen im allgemeinen zwischen 100 und 1000 mg, vorzugsweise 200 bis 500 mg, die Tagesdosis zwischen 150 und 5000 mg, vorzugsweise 500 bis 2500 mg.

Beispiel I

Tabletten enthaltend Pivaloyloxymethyl-7-{D-α--[3-‹4-hydroxy-2-(3'-pyridylmethylamino)-5-pyrimidinyl›-ureido]-p-hydroxyphenylacetamido}-3--[(1-methyl-tetrazol-5-yl)-thiomethyl]-ceph-3-em--4-carboxylat

Ein Gemisch bestehend aus 2 kg Wirksubstanz, 5 kg Lactose, 1,8 kg Kartoffelstärke, 0,1 kg Magnesiumstearat und 0,1 kg Talk wird in üblicher Weise zu Tabletten gepresst, derart, dass jede Tablette 200 mg Wirkstoff enthält.

Beispiel II

Dragées enthaltend Pivaloyloxymethyl-7-{D-α-midinyl›-ureido]-p-hydroxyphenylacetamido}-3--[(1-methyl-tetrazol-5-yl)-thiomethyl]-ceph-3--[3-[(1-methyl-tetrazol-5-yl)-thiomethyl]-ceph-3--em-4-carboxylat

Analog Beispiel I werden Tabletten gepresst, die anschliessend in üblicher Weise mit einem Überzug, bestehend aus Zucker, Kartoffelstärke, Talk und Tragant überzogen werden.

Beispiel III

Kapseln enthaltend Pivalolyloxymethyl-7-{D-α--[3-‹4-hydroxy-2-(3'-pyridylmethylamino)-5-pyrimidinyl›-ureido]-p-hydroxy-phenylacetamido}-3--[(1-methyl-tetrazol-5-yl)-thiomethyl]-ceph-3--em-4-carboxylat

5 kg Wirksubstanz werden in üblicher Weise in Hartgelatinekapseln gefüllt, derart, dass jede Kapsel 500 mg des Wirkstoffes enthält.

Beispiel IV

Trockenampullen enthaltend Natrium-7-{D-α--[3-‹4-hydroxy-2-(3'-pyridylmethylamino)-5-pyrimidinyl›-ureido]-p-hydroxyphenylacetamido}-3--[(1-methyl-tetrazol-5-yl)-thiomethyl]-ceph-3-em--4-carboxylat

In einem aspetischen Bereich wurde 251 g Wirkstoff in 2008 ml destilliertem Wasser zur Injektion aufgelöst. Die Lösung wurde durch ein Millipore-Filter (Porengrösse 0,22 um, Produkt der Millipore Corporation, Bedford, USA) filtriert. Die Lösung wurde jeweils in einer Menge von 2,0 ml in 1000 Gläschen (Kapazität 10 ml) eingegossen und es wurde lyophilisiert. Die Gläschen wurden sodann mit einem Kautschukstöpsel und einer Aluminiumkappe verschlossen. Somit wurden Gläschen (Nr. A) jeweils mit 250 mg Wirkstoff erhalten.

Eine physiologische Kochsalzlösung zur Injektion wurde in einer Menge von jeweils 2,0 ml Ampullen abgefüllt und die Ampullen wurden verschlossen. Auf diese Weise wurden Ampullen (Nr. B) erhalten. Die physiologische Kochsalzlösung in den Ampullen (Nr. B) wurde in die Gläschen (Nr. A) gegossen, wodurch eine injizierbare Zubereitung für die intravenöse Verabreichung erhalten wurde.

Destilliertes Wasser zur Injektion wurde in einer Menge von 20 ml in die Gläschen (Nr. A) gegossen und die Lösung wurde in einer 5%igen Lösung von Glucose für Injektionen (250 ml) aufgelöst. Auf diese Weise wurden Lösungen für die kontinuierliche Infusion hergestellt.

Analog sind Tabletten, Dragées, Kapseln und Ampullen erhältlich, die einen oder mehrere der übrigen Wirkstoffe der Formel I oder die physiologisch unbedenklichen Salze dieser Verbindungen enthalten.

**Patentansprüche**
für die Vertragsstaaten: BE, CH, DE, FR, GB, IT, LI, LU, NL, SE

1. Neue β-Lactame der allgemeinen Formeln

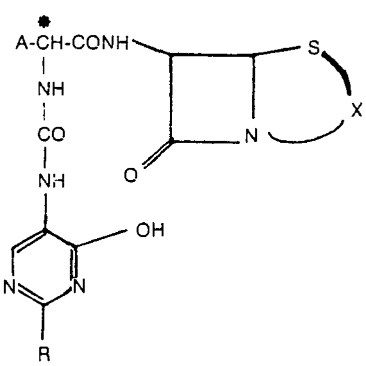

(I)

bzw.

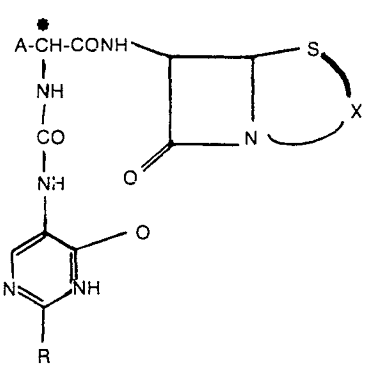

(I')

in welchen

A die Phenyl-, 4-Hydroxyphenyl-, 2- oder 3-Thienyl-, 2- oder 3-Furyl-, Cyclohexyl-, Cyclohexen-1-yl-, Cyclohexa-1,4-dien-1-ylgruppe sowie einen in 3,4-Stellung disubstituierten Phenylrest, wobei die Substituenten dieses Restes gleich oder voneinander verschieden und Chloratome, Hydroxy- oder Methoxygruppen sein können und

R eine Gruppe der allgemeinen Formel $NH-(CH_2)_nR_1$ darstellen, worin n die Zahlen 0 oder 1 und $R_1$ einen gegebenenfalls substituierten 5- oder 6-gliedrigen heterocyclischen Rest mit vorzugsweise 1 bis 4, insbesondere 1 oder 2 gleichen oder verschiedenen Heteroatomen, wie Sauerstoff, Schwefel oder Stickstoff bedeutet, beispielsweise eine gegebenenfalls substituierte Thienyl-, Furyl-, Pyrrolyl-, Oxazolyl-, Isooxazolyl-, Thiazolyl-, Isothiazolyl-, Imidazolyl-, Pyrazolyl-, Oxadiazolyl-, Thiadiazolyl-, Triazolyl-, Tetrazolyl-, Pyridyl-, Pyrazinyl-, Pyridazinyl-, Pyrimidinyl- oder Tetrahydrofuranylgruppe, wobei diese Gruppen durch folgende Reste substituiert sein können:

durch Alkylgruppen, durch Halogenatome, wie Fluor- Chlor oder Brom, durch Nitro-, Cyano-, Amino-, Alkyl- oder Dialkylaminogruppen, durch Alkylcarbonylamino- oder Alkoxycarbonylaminogruppen, durch Hydroxy-, Alkoxy-, Alkylthio-, Alkylsulfinyl- oder Alkylsulfonylgruppen, durch Methylsulfonylamino-, Aminocarbonyl-, Alkylcarbonyloxy- oder Alkoxycarbonylgruppen, Aminosulfonyl-, Alkylamino- oder Dialkylaminosulfonylgruppen, wobei die Alkylgruppen in diesen Resten jeweils 1-4 Kohlenstoffatome enthalten können, sowie durch die Carbonsäure- oder Sulfonsäuregruppe; und

X die Gruppen:

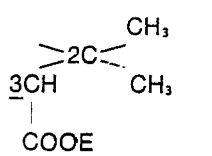 und 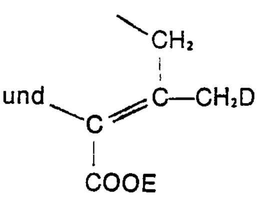

darstellt,

worin D ein Wasserstoffatom, eine Hydroxy-, Acetoxy-, Aminocarbonyloxy-, Pyridinium oder 4-Aminocarbonyl-pyridiniumgruppe oder die Gruppe -SHet bedeutet, wobei Het die Tetrazol-5-yl-, 1-Methyl-tetrazol-5-yl-, 1,2,4-Thiadiazol-5-yl-, 3-Methyl-1,2,4-thiadiazol-5-yl-, 1,3,4-Thiadiazol-2-yl-, 2-Methyl-1,3,4-thiadiazol-5-yl-, 2-Methylamino-1,3,4-thiadiazol-5-yl-, 2-Dimethylamino-1,3,4-thiadiazol-5-yl-, 2-Formylamino-1,3,4-thiadiazol-5-yl-, 2-Acetylamino-1,3,4-thiadiazol-5-yl-, 2-Methyl-1,3,4-oxadiazol-5-yl-, 1,2,3-Triazol-4-yl- oder die 1,2,4-Triazol-3-yl-gruppe darstellt und E ein Wasserstoffatom oder eine in vitro oder in vivo leicht spaltbare Schutzgruppe darstellt, und falls E ein Wasserstoffatom bedeutet, deren physiologisch verträgliche Salze mit anorganischen oder organischen Basen.

2. Neue β-Lactame der allgemeinen Formeln

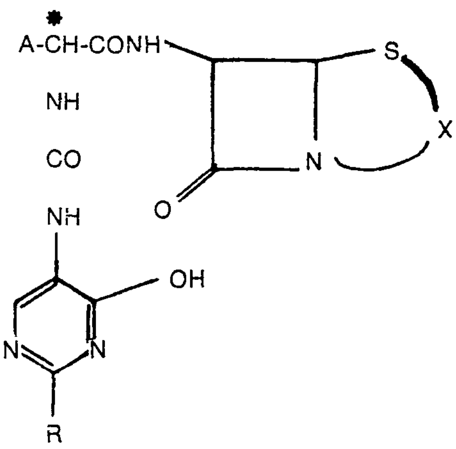

(I)

bzw.

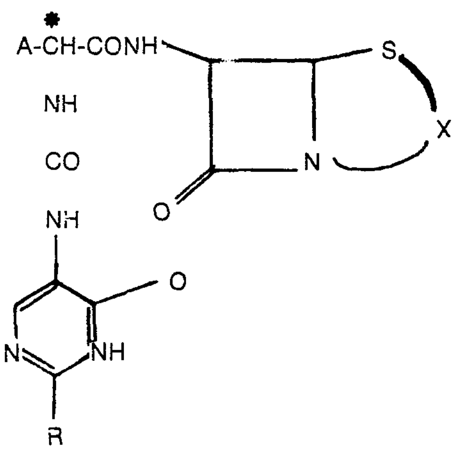

(I')

in welchen

A die Phenyl-, 4-Hydroxyphenyl-, 3,4-Dihydroxyphenyl-, 2- oder 3-Thienyl-, 2- oder 3-Furylgruppe bedeutet,

X wie im Anspruch 1 definiert ist, in X das Symbol E ein Wasserstoffatom, die 2-Acetoxyäthyl- oder Pivaloyloxymethylgruppe darstellt und D die im Anspruch 1 angegebenen Bedeutungen besitzt, sowie

R eine Gruppe der allgemeinen Formel $-NH-(CH_2)_nR_1$ darstellt, wobei die Gruppe $-(CH_2)_nR_1$ die folgenden Bedeutungen hat:

die 3-Pyridyl-, 6-subst.-3-Pyridyl-, 2-subst.-3-Pyridyl-, 2-, 3- oder 4-Pyridylmethyl-, 2-, 4- oder 5-Pyrimidinylmethyl-, 2-subst.-5-Pyridinylmethyl-, 4-Hydroxy-5-pyrimidinyl-, 2-subst.-4-hydroxy-5-pyrimidinyl-, 5-Pyrimidinyl-, 2-subst.-5-Pyrimidinyl-, 4-subst.-2-Pyrimidinyl-, 4,6-disubst.-2-Pyrimidinyl-, 2-subst.-4-Pyrimidinyl-, 2,6-disubst.-4-Pyrimidinyl-, 5-subst.-2-Furyl-, 5-subst.-2-Thienyl-, 2- oder 3-Furylmethyl-, 2- oder 3-Thienylmethyl-, 5-subst.-2-Thienylmethyl-, eine 5-subst.-2-Furylmethylgruppe oder eine 4-subst.-2-Thiazolylgruppe wobei diese Gruppen durch folgende Reste substituiert sein können:

durch Alkylgruppen mit 1 bis 6 Kohlenstoffatomen, durch Halogenatome, wie Fluor, Chlor oder Brom, durch Nitro-, Cyano-, Amino-, Alkyl- oder Dialkylaminogruppen, durch Alkylcarbonylamino- oder Alkoxycarbonylaminogruppen, durch Hydroxy-, Alkoxy-, Alkylthio-, Alkylsulfinyl- oder Alkylsulfonylgruppen, durch Methylsulfonylamino-, Aminocarbonyl-, Alkylcarbonyloxy- oder Alkoxycarbonylgruppen, Aminosulfonyl-, Alkylamino- oder Dialkylaminosulfonylgruppen, wobei die Alkylgruppen in diesen Resten jeweils 1-4 Kohlenstoffatome enthalten können, sowie durch die Carbonsäure- oder Sulfonsäuregruppe;

und falls E ein Wasserstoffatom bedeutet, deren physiologisch verträgliche Salze mit anorganischen oder organischen Basen.

3. Neue β-Lactame der allgemeinen Formeln I bzw. I' gemäss Anspruch 1 oder 2, dadurch gekennzeichnet, dass

A die Phenyl-, p-Hydroxyphenyl-, 2-Thienyl- oder die 2- oder 3-Furylgruppe bedeutet,

X wie im Anspruch 1 definiert ist, wobei

E für Wasserstoff oder die Pivaloyloxymethylgruppe steht und

D ein Wasserstoffatom, die Acetoxy- oder Aminocarbonyloxygruppe oder die Gruppe -SHet bedeutet, worin Het die Tetrazol-5-yl-, die 1-Methyltetrazol-5-yl-, die 1,3,4-Thiadiazol-5-yl- oder die 2-Methyl-1,3,4-thiadiazol-5-ylgruppe darstellt und die Gruppe

$(CH_2)_nR_1$ im Rest R die folgenden Bedeutungen hat:

die 3-Pyridyl-, die 6-Methylsulfinyl-3-pyridylgruppe, die 6-Methylsulfonyl-3-pyridylgruppe, die 6-Hydroxy-3-pyridylgruppe, die 5-Pyrimidinylmethyl- oder die 2-Methyl-5-pydimidinylmethylgruppe, die 2-Methyl- oder die 2-Hydroxy-5-pyrimidinylgruppe, die 6-Hydroxy-2-pyrimidinylgruppe, die 4,6-dihydroxy-2-pyrimidinylgruppe, die 3-Pyridylmethylgruppe, die 2-Furylmethylgruppe, die 2-Thienylgruppe, die 5-Aminosulfonyl-2-thienylmethylgruppe, die 5-Aminocarbonyl- oder die 5-Äthoxycarbonyl-thienylgruppe, und, falls E ein Wasserstoffatom bedeutet, deren physiologisch verträgliche Salze mit anorganischen oder organischen Basen.

4. Neue β-Lactame der allgemeinen Formeln I bzw. I' gemäss Anspruch 1, dadurch gekennzeichnet, dass

A die p-Hydroxyphenyl- oder 2-Thienylgruppe bedeutet,

X wie im Anspruch 1 definiert ist, wobei in X E für ein Wasserstoffatom oder die Pivaloyloxymethylgruppe und D für die Acetoxy- oder die 1-Methyl-tetrazol-5-ylgruppe steht und

$(CH_2)_nR_1$ die folgenden Bedeutungen besitzt:

die 3-Pyridylmethyl-, 6-Methylsulfinyl-3-pyridyl-, 6-Methylsulfonyl-3-pyridyl- oder 6-Hydroxy-3-pyridylgruppe, die 2-Methyl-5-pyridinylmethyl-, die 2-Hydroxy-5-pyrimidinyl-, die 4-Hydroxy-2-pyrimidinyl- oder die 4,6-Dihydroxy-2-pyrimidinylgruppe, die 5-Aminocarbonyl-thienyl-, 2-Thienylmethyl- oder 5-Aminosulfonyl-2-thienylmethyl- oder die 2-Furylmethylgruppe und deren physiologisch verträgliche Salze mit anorganischen oder organischen Basen.

5. Neue β-Lactame der allgemeinen Formeln I bzw. I' gemäss den Ansprüchen 1 bis 4, dadurch gekennzeichnet, dass A, R und X mit D die in den Ansprüchen 1 bis 4 angegebenen Bedeutungen besitzen und E als leicht abspaltbare Gruppe die Benzyl-, Diphenylmethyl-, Trityl-, t-Butyl-, die 2,2,2-Trichloräthyl-, die Trimethylsilyl-, eine Alkanoyloxyalkylgruppe mit 1 bis 5 Kohlenstoffatomen im Alkanoylrest und 1 bis 3 Kohlenstoffatomen im Alkylenrest, die Phthalidyl- oder Indanylgruppe darstellt.

6. Neue β-Lactame der allgemeinen Formel I bzw. I' gemäss Anspruch 1, dadurch gekennzeichnet, dass

A die p-Hydroxyphenylgruppe und

R die 5'-Aminosulfonyl-2'-thienylmethylamino-, 6'-Methylsulfonyl-3'-pyridylamino- oder die 2'-Methyl-5'-pyrimidylmethylaminogruppe bedeuten,

X und E wie im Anspruch 1 definiert sind und

D die (1-Methyl-tetrazol-5-yl)-thiogruppe darstellt und deren physiologisch verträglichen Salze mit anorganischen oder organischen Basen.

7. Verfahren zur Herstellung von neuen β-Lactamen der allgemeinen Formeln

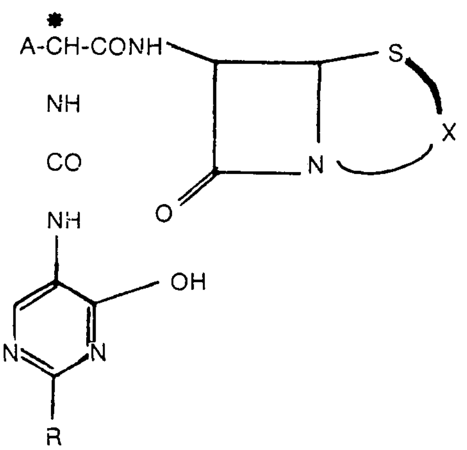

(I)

bzw.

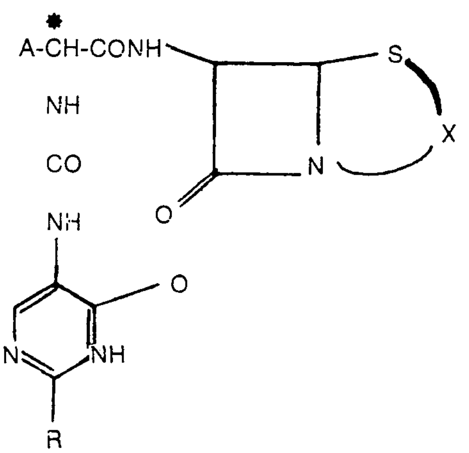

(I')

in welchen A, R, X, D und E wie im Anspruch I angegeben definiert sind, und falls E ein Wasserstoffatom bedeutet, von deren physiologisch verträglichen Salzen mit anorganischen oder organischen Basen, dadurch gekennzeichnet, dass

a) zur Herstellung einer Verbindung der allgemeinen Formel I, in der D die angegebenen Bedeutungen mit Ausnahme der einer Pyridinium- oder Aminocarbonylpyridiniumgruppe besitzt, eine Verbindung der allgemeinen Formel

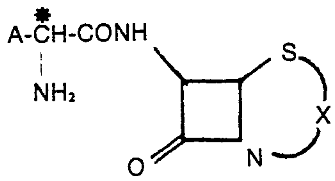

(II)

in der A und X die oben angegebenen Bedeutungen und D die oben angegebenen Bedeutungen mit Ausnahme der einer Pyridinium- oder Aminocarbonylpyridiniumgruppe besitzt, mit einem Pyridinderivat der allgemeinen Formel

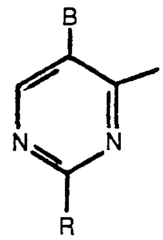

(III)

in der R wie oben definiert ist und B die Gruppen =NCO, -NHCOCl, -NHCOBr oder

-NH-COO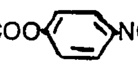$NO_2$, bedeutet, oder mit Gemischen solcher Pyrimidinderivaten der allgemeinen Formel III, in der B teils die eine und teils die andere der vorstehend genannten Bedeutungen besitzt, in einem Lösungsmittel und bei einem pH-Bereich zwischen 2,0 und 9,0 bei Temperaturen zwischen −20°C und +50°C, umgesetzt wird, oder

b) zur Herstellung einer Verbindung der allgemeinen Formel I, in der D die oben angegebenen Bedeutungen mit Ausnahme der einer Pyridinium- oder Aminocarbonylpyridiniumgruppe besitzt, eine Ureidocarbonsäure der allgemeinen Formel

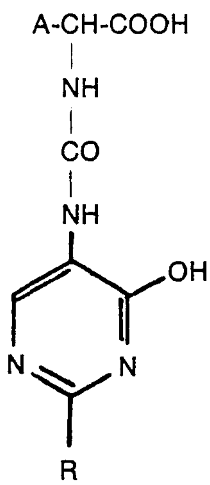

(IV)

in der A und R die oben angegebenen Bedeutungen besitzen, oder ihre Salze oder reaktiven Derivate, mit Verbindungen der allgemeinen Formel

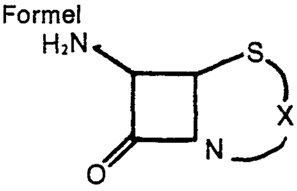

(V)

in der X die oben angegebenen Bedeutungen mit Ausnahme der Formel, in der D für Pyridinium oder Aminopyridinium steht, innehat, zwischen −40°C und +40°C in Gegenwart eines Lösungsmittels und gegebenenfalls in Gegenwart einer Base umgesetzt wird oder,

c) zur Herstellung von Cephalosporinderivaten der allgemeinen Formeln I bzw. I', in der D die Bedeutungen einer -S-Het-, Pyridinium- oder 4-Aminocarbonylpyridiniumgruppe besitzt, eine Verbindung der allgemeinen Formel

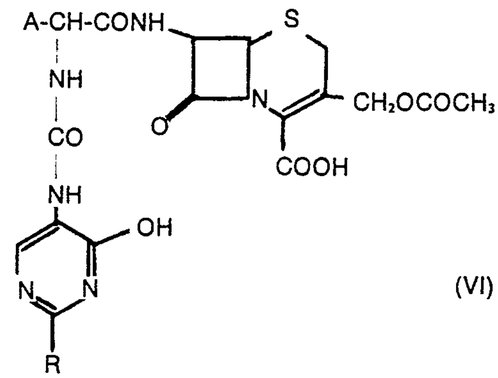

(VI)

in der A und R die oben angegebenen Bedeutungen haben, entweder mit einer Verbindung der allgemeinen Formel

Het-S-M (VII)

in der Het die oben angegebenen Bedeutungen hat und M für ein Wasserstoffatom oder ein Alkalimetall oder ein Erdalkalimetall steht oder mit Pyridin bzw. 4-Aminocarbonylpyridin in einem organischen Lösungsmittel oder in Wasser oder in Gemischen dieser Lösungsmittel bei einem pH-Wert der Reaktionslösung zwischen 2-10, vorteilhafterweise zwischen 4-8, bei einer Temperatur im Bereich von 0° bis 100°C umgesetzt wird und gewünschtenfalls anschliessend eine so hergestellte Verbindung der allgemeinen Formeln I oder I', in denen E für eine in vitro leicht abspaltbare Schutzgruppe steht, in die freie Carbonsäure der allgemeinen Formeln I bzw. I', in der E ein Wasserstoffatom ist, überführt wird und/oder eine Verbindung der allgemeinen Formeln I oder I', in der E ein Wasserstoffatom bedeutet, in ihre Ester oder, mittels anorganischen oder organischen Basen, in die entsprechenden Salze übergeführt wird.

8. Verfahren gemäss Anspruch 7 a, dadurch gekennzeichnet, dass eine Verbindung der allgemeinen Formel II, in der E Wasserstoff bedeutet, oder eines ihrer Salze mit anorganischen oder organischen Basen mit einer Verbindung der allgemeinen Formel III,

a) in Wasser oder in mit Wasser mischbaren Lösungsmitteln in Gegenwart von Wasser in einem pH-Bereich von 6,5 bis 8,0, oder

b) in wasserfreien Lösungsmitteln oder

c) in einem Gemenge aus Wasser und mit Wasser nicht mischbaren Lösungsmitteln in einem pH-Bereich zwischen 6,5 und 8,0

umgesetzt wird, oder, dass eine Verbindung

der allgemeinen Formel II, in der E eine Silylgruppe oder eine andere leicht abspaltbare Schutzgruppe bedeutet, mit einer Verbindung der allgemeinen Formel III in einem wasser- und hydroxylgruppenfreien bzw. aprotischen Lösungsmittel, gegebenenfalls in Gegenwart einer Base, umgesetzt wird.

9. Verfahren gemäss Anspruch 7b, dadurch gekennzeichnet, dass als reaktive Derivate der Ureidocarbonsäure der allgemeinen Formel IV deren Säureanhydride, deren reaktive Ester oder reaktive Amide oder deren Säurehalogenide, als reaktive Ester der Verbindungen der allgemeinen Formel V der Diphenylmethylester, der t-Butylester, der Trimethylsilylester oder das N,O--Bis-trimethylsilylderivat verwendet werden und die Umsetzung in Gegenwart einer Base und/oder eines organischen Lösungsmittels und/oder eines Kondensationsmittels erfolgt und, gewünschtenfalls, das so erhaltene Produkt der allgemeinen Formel I, in der E eine abspaltbare Schutzgruppe bedeutet, anschliessend in eine Verbindung der allgemeinen Formel I übergeführt wird, in der E ein Wasserstoffatom darstellt.

10. Verfahren gemäss Anspruch 7 zur Herstellung einer Verbindung der allgemeinen Formel I oder I', worin in X das Symbol E den Pivaloyloxymethylrest bedeutet, dadurch gekennzeichnet, dass eine Verbindung der allgemeinen Formel I oder I', in der E ein Wasserstoffatom darstellt, in ihr Aykalisalz übergeführt und dieses mit einem Pivaloyloxymethylhalogenid der allgemeinen Formel

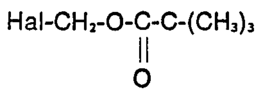

worin Hal für Chlor, Brom oder Jod steht, in einem Lösungsmittel bei Temperaturen zwischen 20 und 45°C umgesetzt wird.

11. Arzneimittel gekennzeichnet durch einen Gehalt an einer oder an mehreren Verbindungen der allgemeinen Formeln I bzw. I' gemäss den Ansprüchen 1 bis 6 neben den üblichen Träger- und/oder Hilfsstoffen.

**Patentansprüche**
für den Vertragsstaat: AT

1. Verfahren zur Herstellung von neuen β-Lactamen der allgemeinen Formeln

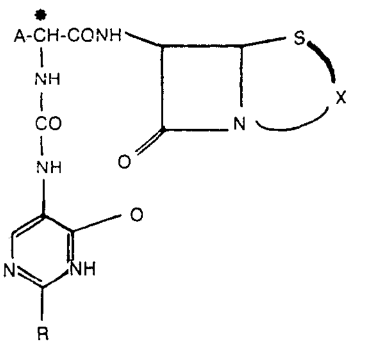

(I')

bzw.

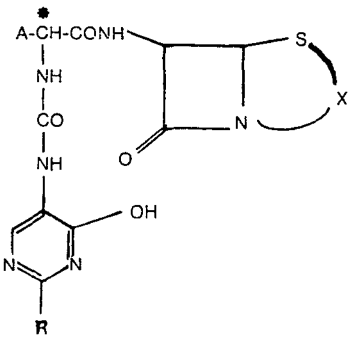

(I)

in welchen

A die Phenyl-, 4-Hydroxyphenyl-, 2- oder 3--Thienyl-, 2- oder 3-Furyl-, Cyclohexyl-, Cyclohexen-1-yl, Cyclohexa-1,4-dien-1-ylgruppe sowie einen in 3,4-Stellung disubstituierten Phenylrest, wobei die Substituenten dieses Restes gleich oder voneinander verschieden und Chloratome, Hydroxy- oder Methoxygruppen sein können und

R eine Gruppe der allgemeinen Formel $NH-(CH_2)_nR_1$ darstellen, worin n die Zahlen 0 oder 1 und $R_1$ einen gegebenenfalls substituierten 5- oder 6-gliedrigen heterocyclischen Rest mit vorzugsweise 1 bis 4, insbesondere 1 oder 2 gleichen oder verschiedenen Heteroatomen, wie Sauerstoff, Schwefel oder Stickstoff bedeutet, beispielsweise eine gegebenenfalls substituierte Thienyl-, Furyl-, Pyrrolyl-, Oxazolyl-, Isoxazolyl-, Thiazolyl-, Isothiazolyl-, Imidazolyl-, Pyrazolyl-, Oxdiazolyl-, Thiadiazolyl-, Triazolyl-, Tetrazolyl-, Pyridyl-, Pyrazinyl-, Pyridazinyl-, Pyrimidinyl- oder Tetrahydrofuranylgruppe, wobei diese Gruppen durch folgende Reste substituiert sein können:

durch Alkylgruppen, durch Halogenatome, wie Fluor, Chlor oder Brom, durch Nitro-, Cyano-, Amino-, Alkyl- oder Dialkylaminogruppen, durch Alkylcarbonylamino- oder Alkoxycarbonylaminogruppen, durch Hydroxy-, Alkoxy-, Alkylthio-, Alkylsulfinyl- oder Alkylsulfonylgruppen, durch Methylsulfonylamino-, Aminocarbonyl-, Alkylcarbonyloxy- oder Alkoxycarbonylgruppen, Aminosulfonyl-, Alkylamino- oder Dialkylaminosulfonylgruppen, wobei die Alkylgruppen in diesen Resten jeweils 1-4 Kohlenstoffatome enthalten können, sowie durch die Carbonsäure- oder Sulfonsäuregruppe; und

X die Gruppen:

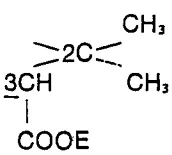

und

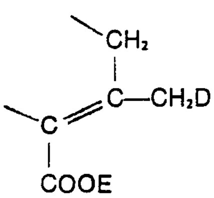

darstellt,

worin D ein Wasserstoffatom, eine Hydroxy-, Acetoxy-, Aminocarbonyloxy-, Pyridinium oder 4-Aminocarbonyl-pyridiniumgruppe oder die Gruppe -SHet bedeutet, wobei Het die Tetrazol--5-yl-, 1-Methyl-tetrazol-5-yl-, 1,2,4-Thiadiazol-5--yl-, 3-Methyl-1,2,4-thiadiazol-5-yl-, 1,3,4-Thiadiazol-2-yl-, 2-Methyl-1,3,4-thiadiazol-5-yl-, 2-Methylamino-1,3,4-thiadiazol-5-yl-, 2-Dimethylamino-1,3,4-thiadiazol-5-yl-, 2-Formylamino-1,3,4-thiadiazol-5-yl-, 2-Acetylamino-1,3,4-thiadiazol-5-yl-, 2-Methyl-1,3,4-oxadiazol-5-yl-, 1,2,3-Triazol-4-yl- oder die 1,2,4-Triazol-3-yl-gruppe darstellt und E ein Wasserstoffatom oder eine in vitro oder in vivo leicht spaltbare Schutzgruppe bedeutet, und falls E ein Wasserstoffatom bedeutet, von deren physiologisch verträglichen Salzen mit anorganischen oder organischen Basen, dadurch gekennzeichnet, dass

a) zur Herstellung einer Verbindung der allgemeinen Formel I, in der D die angegebenen Bedeutungen mit Ausnahme der einer Pyridinium- oder Aminocarbonylpyridiniumgruppe besitzt, eine Verbindung der allgemeinen Formel

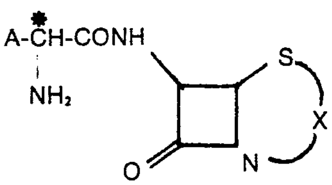

(II)

in der A und X die oben angegebenen Bedeutungen und D die oben angegebenen Bedeutungen mit Ausnahme der einer Pyridinium- oder Aminocarbonylpyridiniumgruppe besitzt, mit einem Pyrimidinderivat der allgemeinen Formel

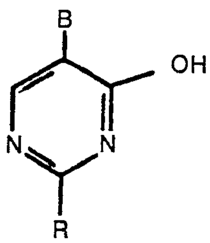

(III)

in der R wie oben definiert ist und B die Gruppen =NCO, -NHCOCl, -NHCOBr oder

-NH-COO 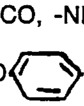 $NO_2$, bedeutet, oder mit Gemischen solcher Pyrimidinderivaten der allgemeinen Formel III, in der B teils die eine und teils die andere der vorstehend genannten Bedeutungen besitzt, in einem Lösungsmittel und bei einem pH-Bereich zwischen 2,0 und 9,0 bei Temperaturen zwischen −20°C und +50°C, umgesetzt wird, oder

b) zur Herstellung einer Verbindung der allgemeinen Formel I, in der D die oben angegebenen Bedeutungen mit Ausnahme der einer Pyridinium- oder Aminocarbonylpyridiniumgruppe besitzt, eine Ureidocarbonsäure der allgemeinen Formel

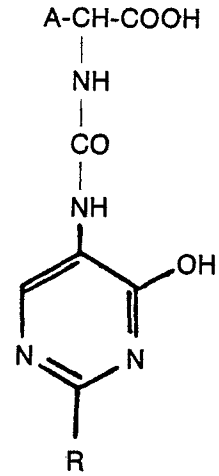

(IV)

in der A und R die oben angegebenen Bedeutungen besitzen, oder ihre Salze oder reaktiven Derivate, mit Verbindungen der allgemeinen Formel

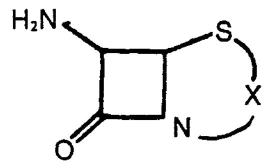

(V)

in der X die oben angegebenen Bedeutungen mit Ausnahme der Formel, in der D für Pyridinium oder Aminopyridinium steht, innehat, zwischen −40°C und +40°C in Gegenwart eines Lösungsmittels und gegebenenfalls in Gegenwart einer Base umgesetzt wird oder,

c) zur Herstellung von Cephalosporinderivaten der allgemeinen Formeln I bzw. I', in der D die Bedeutungen einer -S-Het-, Pyridinium- oder 4-Aminocarbonylpyridiniumgruppe besitzt, eine Verbindung der allgemeinen Formel

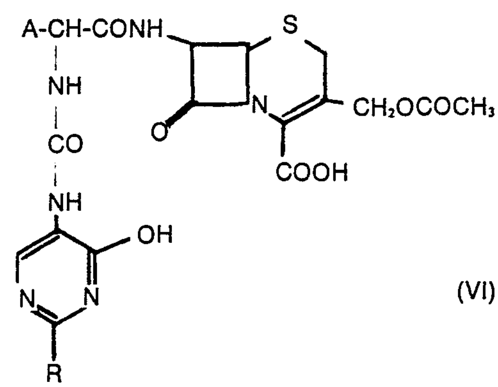

(VI)

die unter die allgemeine Formel I fällt und in der A und R die oben angegebenen Bedeutungen haben, entweder mit einer Verbindung der allgemeinen Formel

Het-S-M (VII)

in der Het die oben angegebenen Bedeutungen hat und M für ein Wasserstoffatom oder ein Alkalimetall oder ein Erdalkalimetall steht oder mit Pyridin bzw. 4-Aminocarbonylpyridin in einem organischen Lösungsmittel oder in Wasser oder in Gemischen dieser Lösungsmittel bei

einem pH-Wert der Reaktionslösung zwischen 2-10, vorteilhafterweise zwischen 4-8, bei einer Temperatur im Bereich von 0° bis 100°C umgesetzt wird und gewünschtenfalls anschliessend eine so hergestellte Verbindung der allgemeinen Formeln I oder I', in denen E für eine in vitro leicht abspaltbare Schutzgruppe steht, in die freie Carbonsäure der allgemeinen Formeln I bzw. I', in der E ein Wasserstoffatom ist, überführt wird und/oder eine Verbindung der allgemeinen Formeln I oder I', in der E ein Wasserstoffatom bedeutet, in ihre Ester oder, mittels anorganischen oder organischen Basen, in die entsprechenden Salze übergeführt wird.

2. Verfahren gemäss Anspruch 1 a, dadurch gekennzeichnet, dass eine Verbindung der allgemeinen Formel II, in der E Wasserstoff bedeutet, oder eines ihrer Salze mit anorganischen oder organischen Basen mit einer Verbindung der allgemeinen Formel III,

a) in Wasser oder in mit Wasser mischbaren Lösungsmitteln in Gegenwart von Wasser in einem pH-Bereich von 6,5 bis 8,0, oder

b) in wasserfreien Lösungsmitteln oder

c) in einem Gemenge aus Wasser und mit Wasser nicht mischbaren Lösungsmitteln in einem pH-Bereich zwischen 6,5 und 8,0

umgesetzt wird, oder, dass eine Verbindung der allgemeinen Formel II, in der E eine Silylgruppe oder eine andere leicht abspaltbare Schutzgruppe bedeutet, mit einer Verbindung der allgemeinen Formel III in einem wasser- und hydroxylgruppenfreien bzw. aprotischen Lösungsmittel, gegebenenfalls in Gegenwart einer Base, umgesetzt wird.

3. Verfahren gemäss Anspruch 1 b, dadurch gekennzeichnet, dass als reaktive Derivate der Ureidocarbonsäuren der allgemeinen Formel IV deren Säureanhydride, deren reaktive Ester oder reaktive Amide oder deren Säurehalogenide, als reaktive Ester der Verbindungen der allgemeinen Formel V der Diphenylmethylester, der t-Butylester, der Trimethylsilylester oder das N,O-Bis-trimethylsilylderivat verwendet werden und die Umsetzung in Gegenwart einer Base und/oder eines organischen Lösungsmittels und/oder eines Kondensationsmittels erfolgt und, gewünschtenfalls, das so erhaltene Produkt der allgemeinen Formel I, in der E eine abspaltbare Schutzgruppe bedeutet, anschiessend in eine Verbindung der allgemeinen Formel I übergeführt wird, in der E ein Wasserstoffatom darstellt.

4. Verfahren gemäss Anspruch 1 zur Herstellung einer Verbindung der allgemeinen Formel I oder I', worin in X das Symbol E den Pivaloyloxymethylrest bedeutet, dadurch gekennzeichnet, dass eine Verbindung der allgemeinen Formel I oder I', in der E ein Wasserstoffatom darstellt, in ihr Alkalisalz übergeführt und dieses mit einem Pivaloyloxymethylhalogenid der allgemeinen Formel

Hal-$CH_2$-O-C-C-$(CH_3)_3$
||
O

worin Hal für Chlor, Brom oder Jod steht, in einem Lösungsmittel bei Temperaturen zwischen 20 und 45°C umgesetzt wird.

**Claims**

for the Contracting states: BE, CH, DE, FR, GB, IT, LI, LU, NL, SE.

1. Novel β-lactams of general formulae

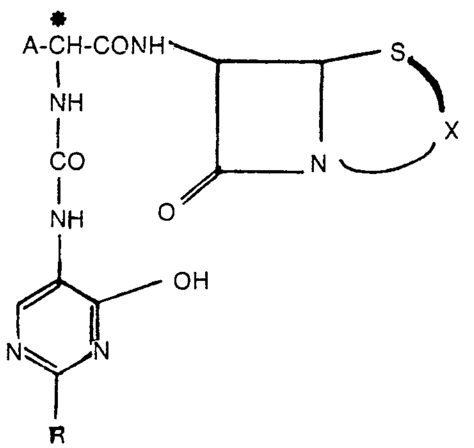

(I)

or

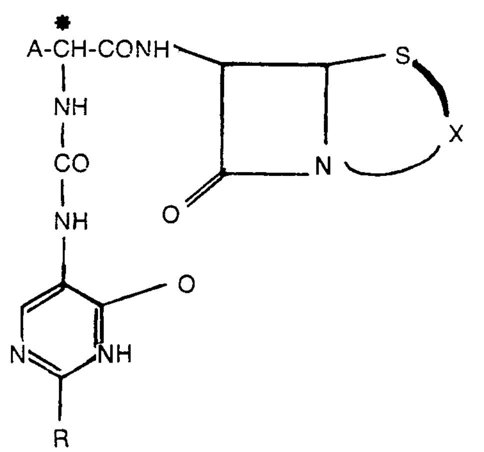

(I')

in which

A represents a phenyl, 4-hydroxyphenyl, 2-or 3-thienyl, 2- or 3-furyl, cyclohexyl, cyclohexen-1-yl, or cyclohexa-1,4-dien-1-yl group or a phenyl radical disubstituted in the 3 and 4 positions, whereby the substituents of this radical can be the same or different from one another and can be chlorine atoms or hydroxy or methoxy groups and

R represents a group of general formula $NH(CH_2)_nR_1$ in which n represents the numbers 0 or 1 and $R_1$ represents an optionally substituted 5- or 6-membered heterocyclic radical with preferably 1 to 4, especially 1 or 2, like or different heteroatoms such as oxygen, sulphur or nitrogen, for example an optionally substituted thienyl, furyl, pyrrolyl, oxazolyl, isooxazolyl, thiazolyl, isothiazolyl, imidazolyl, pyrazolyl, oxdiazolyl, thiadiazolyl, triazolyl, tetrazolyl, pyridyl, pyrazinyl, pyridazinyl, pyrimidinyl or tetrahydrofuranyl group, whereby these groups can be substituted by the following radicals:

by alkyl groups,
by halogen atoms such as fluorine, chlorine or bromine,
by nitro, cyano, amino, alkylamino or dialkylamino groups, by alkylcarbonylamino or alkoxycarbonylamino groups, by hydroxy, alkoxy, alkylthio, alkylsulfinyl or alkylsulfonyl groups, by methylsulfonylamino, aminocarbonyl, alkylcarbonyloxy or alkoxycarbonyl groups, or by aminosulfonyl, alkylaminosulfonyl or dialkylaminosulfonyl groups, whereby the alkyl groups in these radicals can each contain 1 to 4 carbon atoms, as well as by the carboxylic acid or sulphonic acid group; and

X represents the groups:

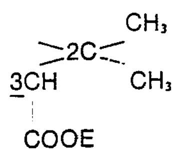

and

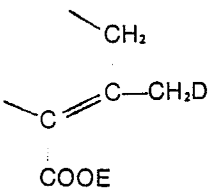

in which D represents a hydrogen atom, a hydroxy, acetoxy, aminocarbonyloxy, pyridinium or 4-aminocarbonylpyridinium group or the group -SHet, whereby Het represents a tetrazol-5-yl, 1-methyl-tetrazol-5-yl, 1,2,4-thiadiazol-5-yl, 3-meth -1,2,4-thiadiazol-5-yl, -,3,4-thiadiazol-2-yl, 2- hyl-1,3,4-thiadiazol-5-yl, 2-methylamino-1,3,4- azol-5-yl, 2-dimethylamino-1,3,4-thiadiazol- 2-formylamino-1,3,4-thiadiazol-5-yl, 2-acetyla -1,3,4-thiadiazol-5-yl, 2-methyl-1,3,4-oxadiazol-5-yl, 1,2,3-triazol-4-yl or 1,2,4-triazol-3-yl group, and E represents a hydrogen atom or a protective group easily separable in vitro or in vivo and, if E represents a hydrogen atom, their physiologically compatible salts with inorganic or organic bases.

2. Novel β-lactams of general formulae

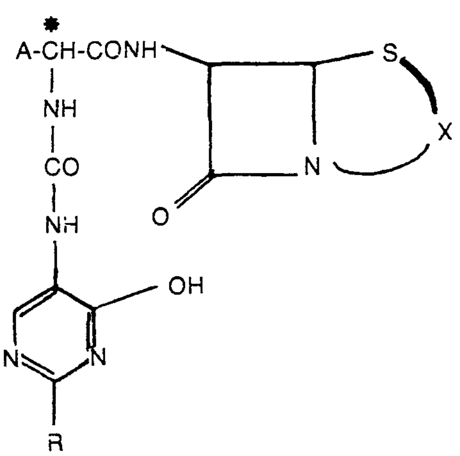

(I)

or

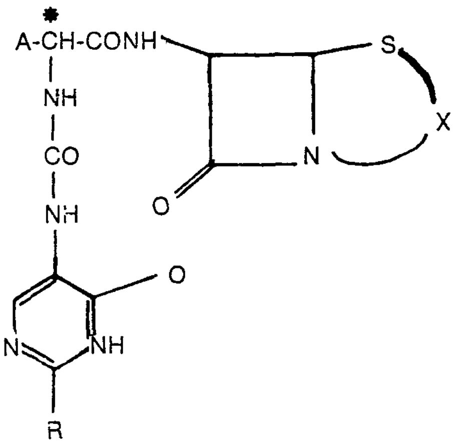

(I')

in which

A represents a phenyl, 4-hydroxyphenyl, 3,4-dihydroxyphenyl, 2- or 3-thienyl or 2- or 3-furyl group,

X is as defined in claim 1 and in X the symbol E represents a hydrogen atom or a 2-acetoxyethyl or pivaloyloxymethyl group and D has the meanings specified in claim 1, and

R represents a group of general formula $-NH(CH_2)_nR_1$, the group $-(CH_2)_nR_1$ having the following meanings:

a 3-pyridyl, 6-subst.-3-pyridyl, 2-subst.-3-pyridyl, 2-, 3- or 4-pyridylmethyl, 2-, 4- or 5-pyrimidinylmethyl, 2-subst.-5-pyrimidinylmethyl, 4-hydroxy-5-pyrimidinyl, 2-subst.-4-hydroxy-5-pyrimidinyl, 5-pyrimidinyl, 2-subst.-5-pyrimidinyl, 4-subst.-2-pyrimidinyl, 4,6-disubst.-2-pyrimidinyl, 2-subst.-4-pyrimidinyl, 2,6-disubst.-4-pyrimidinyl, 5-subst.-2-furyl, 5-subst.-2-thienyl, 2- or 3-furylmethyl, 2- or 3-thienylmethyl, 5-subst.-2-thienylmethyl, 5-subst.-2-furylmethyl group or 4-subst.-2-thiazolyl group, whereby these groups can be substituted by the following radicals:

by alkyl groups containing 1 to 6 carbon atoms, or by halogen atoms such as fluorine, chlorine or bromine, by nitro, cyano, amino, alkylamino or dialkylamino groups, by alkylcarbonylamino or alkoxycarbonylamino groups, by hydroxy, alkoxy, alkylthio, alkylsulfinyl or alkylsulfonyl groups, by methylsulfonylamino, aminocarbonyl, alkylcarbonyloxy or alkoxycarbonyl groups, or by aminosulfonyl, alkylaminosulfonyl or dialkylaminosulfonyl groups, whilst the alkyl groups in these radicals can each contain 1 to 4 carbon atoms, or by a carboxylic acid or sulphonic acid group, and, if E represents a hydrogen atom, their physiologically compatible salts with inorganic or organic bases.

3. Novel β-lactams of general formulae I or I' according to claim 1 or 2, characterised in that

A represents a phenyl, p-hydroxyphenyl, 2-thienyl or 2- or 3-furyl group,

X is as defined in claim 1, whilst

E represents hydrogen or a pivaloxymethyl group and

D represents a hydrogen atom, an acetoxy or aminocarbonyloxy group or the group -SHet, in which Het represents a tetrazol-5-yl, 1-methyl-tetrazol-5-yl, 1,3,4-thiadiazol-5-yl or 2-methyl-1,3,-4-thiadiazol-5-yl group and

the group $(CH_2)_nR_1$ in the radical R has the following meanings:

a 3-pyridyl, 6-methylsulfinyl-3-pyridyl, 6-methylsulfonyl-3-pyridyl, 6-hydroxy-3-pyridyl, 5-pyrimidinylmethyl, 2-methyl-5-pyrimidinylmethyl, 2-methyl-5-pyrimidinyl, 2-hydroxy-5-pyrimidinyl, 6-hydroxy-2-pyrimidinyl, 4,6-dihydroxy-2-pyrimidinyl, 3-pyridylmethyl, 2-furylmethyl, 2-thienyl, 5-aminosulfonyl-2-thienylmethyl, 5-aminocarbonylthienyl or 5-ethoxycarbonylthienyl group and, if E represents a hydrogen atom, their physiologically compatible salts with inorganic or organic bases.

4. Novel β-lactams of general formulae I or I' according to claim 1, characterised in that

A represents a p-hydroxyphenyl or 2-thienyl group,

X is as defined in claim 1, whilst in X

E represents a hydrogen atom or a pivaloyloxymethyl group and

D represents an acetoxy or 1-methyl-tetrazol-5-yl group and

$(CH_2)_nR_1$ has the following meanings:

a 3-pyridylmethyl, 6-methylsulfinyl-3-pyridyl, 6-methyl-sulphonyl-3-pyridyl or 6-hydroxy-3-pyridyl, 2-methyl-5-pyrimidinylmethyl, 2-hydroxy-5-pyrimidinyl, 4-hydroxy-2-pyrimidinyl, 4,6-dihydroxy-2-pyrimidinyl, 5-aminocarbonyl-thienyl, 2-thienylmethyl, 5-aminosulphonyl-2-thienylmethyl or 2-furylmethyl group and their physiologically compatible salts with inorganic or organic bases.

5. Novel β-lactams of general formula I or I' according to claims 1 to 4, characterised in that A, R, X and D have the meanings specified in claims 1 to 4 and E represents, as an easily separable group, a benzyl, diphenylmethyl, trityl, t-butyl, 2,2,2-trichloroethyl or trimethylsilyl group, an alkanoyloxyalkyl group with 1 to 5 carbon atoms in the alkanoyl radical and 1 to 3 carbon atoms in the alkylene radical, or a phthalidyl or indanyl group.

6. Novel β-lactams of general formula I or I' according to claim 1, characterised in that

A represents a p-hydroxyphenyl group and

R represents a 5'-aminosulphonyl-2'-thienylmethylamino, 6'-methylsulphonyl-3'-pyridylamino or 2-methyl-5'-pyrimidylmethylamino group,

X and E are defined as in claim 1 and

D represents a (1-methyl-tetrazol-5-yl)-thio group,

and the physiologically acceptable salts thereof with inorganic or organic bases.

7. Process for the preparation of novel β-lactams of general formulae

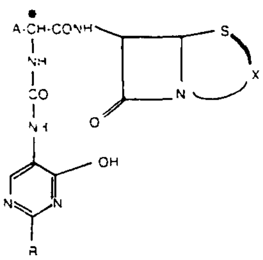

(I)

or

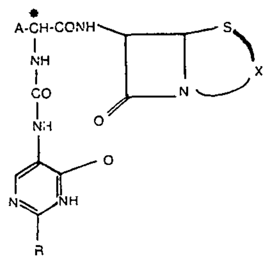

(I')

in which

A, R, X, D and E are defined as in claim 1, and, if E represents a hydrogen atom, the physiologically acceptable salts thereof with inorganic or organic bases, characterised in that

a) for the preparation of a compound of general formula I in which D has the specified meanings with the exception of a pyridinium or aminocarbonylpyridinium group, a compound of general formula

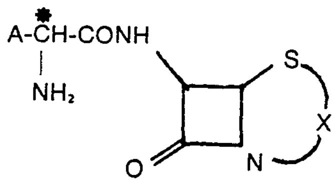

(II)

in which A and X have the above-specified meanings and D has the above-specified meanings with the exception of a pyridinium or aminocarbonylpyridinium group, is reacted with a pyrimidine derivative of general formula

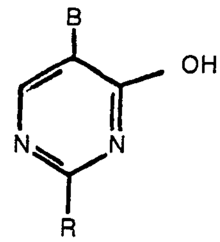

(III)

in which R is as defined above and B represents the groups =NCO, -NHCOCl, -NHCOBr or

-NH-COO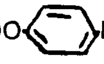$NO_2$, or with mixtures of such

pyrimidine derivatives of general formula III in which B has partly the one and partly the other of the above-mentioned meanings, in a solvent and in a pH range between 2.0 and 9.0 at temperatures between −20°C and +50°C, or

b) for the preparation of a compound of general formula I in which D has the above-specified meanings with the exception of a pyridinium or aminocarbonylpyridinium group, a ureidocarboxylic acid of general formula

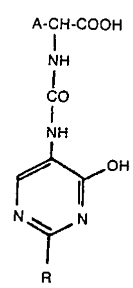

(IV)

in which A and R have the above-specified meanings, or its salts or reactive derivatives, is reacted with compounds of general formula

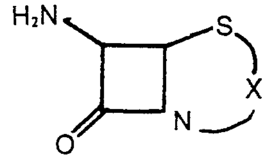

(V)

in which X has the above-specified meanings with the exception of the formula in which D represents pyridinium or aminopyridinium, between −40°C and +40°C in the presence of a solvent and optionally in the presence of a base, or

c) for the preparation of cephalosporin derivatives of general formulae I or I' in which D has the meanings of a -S-Het, pyridinium or 4-aminocarbonylpyridinium group, a compound of general formula

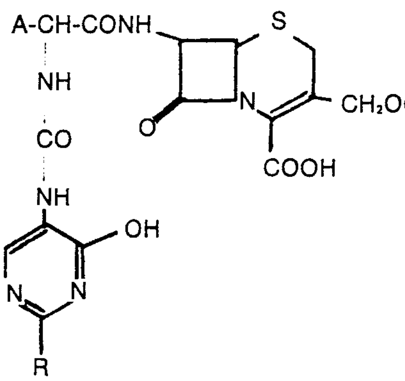

(VI)

in which A and R have the above-specified meanings, is reacted either with a compound of general formula

Het-S-M (VII)

in which Het has the above-specified meanings and H represents a hydrogen atom or an alkali metal or an alkaline-earth metal or with pyridine or 4-amino-carbonylpyridine in an organic solvent or in water or in mixtures of these solvents with a pH value of the reaction solution between 2 and 10, advantageously between 4 and 8, at a temperature in the range from 0° to 100°C, and, if desired, a compound of general formulae I or I' thus prepared, in which E represents a protective group easily separable in vitro, is subsequently converted into the free carboxylic acid of general formulae I or I' in which E represents a hydrogen atom and/or a compound of general formulae I or I' in which E represents a hydrogen atom is converted into its esters or, by means of inorganic or organic bases, into the corresponding salts.

8. Process according to claim 7a, characterised in that a compound of general formula II in which E represents hydrogen or one of its salts with inorganic or organic bases is reacted with a compound of general formula III

a) in water or in solvents miscible with water in the presence of water in a pH range from 6.5 to 8.0 or

b) in anhydrous solvents or

c) in a mixture of water and solvents immiscible with water in a pH range between 6.5 and 8.0 or in that a compound of general formula II in which E represents a silyl group or another easily separable protective group is reacted with a compound of general formula III in an anhydrous solvent free of hydroxyl groups or an aprotic solvent, optionally in the presence of a base.

9. Process according to claim 7b, characterised in that the reactive derivatives of the ureidocarboxylic acids of general formula IV which are used are their acid anhydrides, their reactive esters or reactive amides or their acid halides and the reactive esters of the compounds of general formula V which are used are the diphenylmethyl ester, the t-butyl ester, the trimethylsilyl ester or the N,O-bis-trimethylsilyl derivative and in that the reaction is effected in the presence of a base and/or an organic solvent and/or a condensation agent and, if desired, the product of general formula I thus obtained, in which E represents a separable protective group, is subsequently converted into a compound of general formula I in which E represents a hydrogen atom.

10. Process according to claim 7 for the preparation of a compound of general formula I or I' in which in X the symbol E represents the pivaloyloxymethyl radical, characterised in that a compound of general formula I or I' in which E represents a hydrogen atom is converted into its alkali metal salt and this is reacted with a pivaloyloxymethyl halide of general formula

$$Hal\text{-}CH_2\text{-}O\text{-}\underset{\underset{O}{\|}}{C}\text{-}C\text{-}(CH_3)_3$$

in which Hal represents chlorine, bromine or iodine, in a solvent at temperatures between 20 and 45°C.

11. Pharmaceutical agents, characterised by a content of one or more compounds of general formulas I or I' according to claims 1 to 6 together with the conventional carriers and/or excipients.

**Claims**

for the Contacting state: AT

1. Process for the preparation of novel β-lactams of general formulae

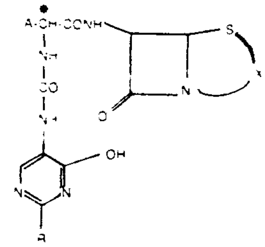

or

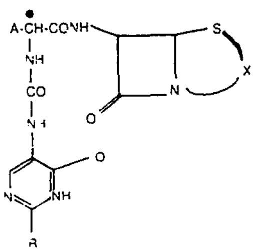

(I')

in which

A represents a phenyl, 4-hydroxyphenyl, 2-or 3-thienyl, 2- or 3-furyl, cyclohexyl, cyclohexen--1-yl or cyclohexa-1,4-dien-1-yl group or a phenyl radical disubstituted in the 3 and 4 positions, whereby the substituents of this radical can be the same or different from one another and can represent chlorine atoms, or hydroxy or methoxy groups and

R represents a group of general formula $NH(CH_2)_nR_1$ in which n represents the numbers 0 or 1 and $R_1$ represents an optionally substituted 5- or 6-membered heterocyclic radical with preferably 1 to 4, especially 1 or 2 like or different heteroatoms such as oxygen, sulphur or nitrogen, for example an optionally substituted thienyl, furyl, pyrrolyl, oxazolyl, isooxazolyl, thiazolyl, isothiazolyl, imidazolyl, pyrazolyl, oxdiazolyl, thiadiazolyl, triazolyl, tetrazolyl, pyridyl, pyrazinyl, pyridazinyl, pyrimidinyl or tetrahydrofuranyl group, whereby these groups can be substituted by the following radicals:

by alkyl groups, by halogen atoms such as fluorine, chlorine or bromine, by nitro, cyano, amino, alkylamino or dialkylamino groups, by alkylcarbonylamino, or alkoxycarbonylamino groups, by hydroxy, alkoxy, alkylthio, alkylsulfinyl or alkylsulphonyl groups, by methylsulfonylamino, aminocarbonyl, alkylcarbonyloxy or alkoxycarbonyl groups, or by aminosulfonyl, alkylaminosulfonyl or dialkylaminosulfonyl groups, whilst the alkyl groups in these radicals can each contain 1 to 4 carbon atoms, and by the carboxylic acid or sulfonic acid group; and

X represents the groups:

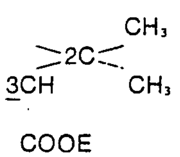 and 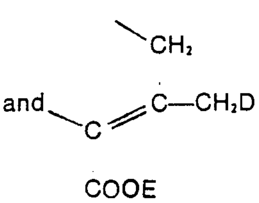

in which D represents a hydrogen atom, a hydroxy, acetoxy, aminocarbonyloxy, pyridinium or 4-aminocarbonylpyridinium group or the group -SHet, whereby Het represents the tetrazol-5-yl, 1-methyl-tetrazol-5-yl, 1,2,4-thiadiazol-5-yl, 3-methyl-1,2,4-thiadiazol-5-yl, 1,3,4-thiadiazol-2-yl, 2--methyl-1,3,4-thiadiazol-5-yl, 2-methylamino-1,3,4--thiadiazol-5-yl, 2-dimethylamino-1,3,4-thiadiazol-5-yl, 2-formylamino-1,3,4-thiadiazol-5-yl, 2-acetylamino-1,3,4-thiadiazol-5-yl, 2-methyl-1,3,4--oxadiazol-5-yl, 1,2,3-triazol-4-yl or the 1,2,4--triazol-3-yl group, and E represents a hydrogen atom or a protective group easily separable in vitro or in vivo and, if E represents a hydrogen atom, of their physiologically compatible salts with inorganic or organic bases, characterised in that

a) for the preparation of a compound of general formula I in which D has the specified meanings with the exception of a pyridinium or aminocarbonylpyridinium group, a compound of general formula

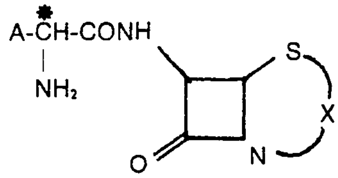

(II)

in which A and X have the above-specified meanings and D has the above-specified meanings with the exception of a pyridinium or aminocarbonylpyridinium group, is reacted with a pyrimidine derivative of general formula

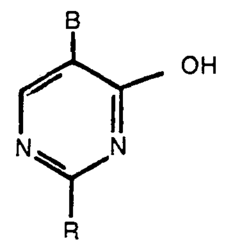

(III)

in which R is as defined above and B represents the groups =NCO, -NHCOCl, -NHCOBr or

-NH-COO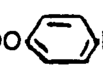$NO_2$, or with mixtures of such

pyrimidine derivatives of general formula III in which B has partly the one and partly the other of the above-mentioned meanings, in a solvent and in a pH range between 2.0 and 9.0 at temperatures between −20°C and +50°C, or

b) for the preparation of a compound of general formula I in which D has the above-specified meanings with the exception of a pyridinium or aminocarbonylpyridinium group, a ureidocarboxylic acid of general formula

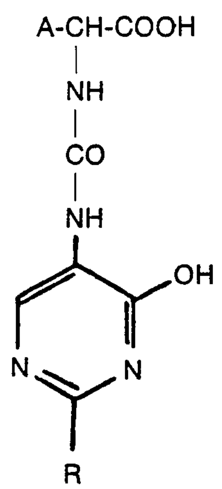

(IV)

in which A and R have the above-specified

meanings, or its salts or reactive derivatives, is racted with compounds of general formula

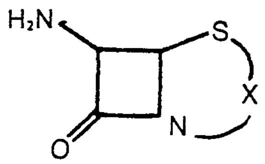

(V)

in which X has the above-specified meanings with the exception of the formula in which D represents pyridinium or aminopyridinium, between −40°C and +40°C in the presence of a solvent and optionally in the presence of a base or

c) for the preparation of cephalosporin derivatives of general formulae I or I' in which D has the meanings of a -S-Het, pyridinium or 4-aminocarbonylpyridinium group, a compound of general formula

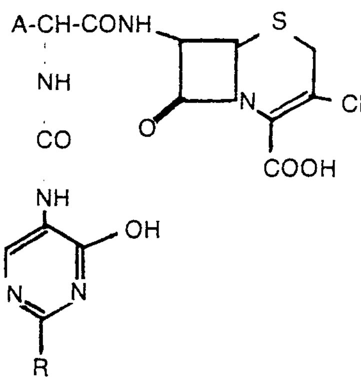

(VI)

which comes under general formula I and in which A and R have the above-specified meanings, is reacted either with a compound of general formula

Het-S-M (VII)

in which Het has the above-specified meanings and M represents a hydrogen atom or an alkali metal or an alkaline-earth metal or with pyridine or 4-amino-carbonylpyridine in an organic solvent or in water or in mixtures of these solvents with a pH value of the reaction solution between 2 and 10, advantageously between 4 and 8, at a temperature in the range from 0° to 100°C, and, if desired, a compound of general formulae I or I' thus prepared, in which E represents a protective group easily separable in vitro, is subsequently converted into the free carboxylic acid of general formulae I or I' in which E represents a hydrogen atom and/or a compound of general formulae I or I' in which E represents a hydrogen atom is converted into its esters or, by means of inorganic or organic bases, into the corresponding salts.

2. Process according to claim 1a, characterised in that a compound of general formula II in which E represents hydrogen or one of its salts with inorganic or organic bases is reacted with a compound of general formula III

a) in water or in solvents miscible with water in the presence of water in a pH range from 6.5 to 8.0 or

b) in anhydrous solvents or

c) in a mixture of water and solvents immiscible with water in a pH range between 6.5 and 8.0 or in that a compound of general formula II in which E represents a silyl group or another easily separable protective group is reacted with a compound of general formula III in an anhydrous solvent free of hydroxyl groups or an aprotic solvent, optionally in the presence of a base.

3. Process according to claim 1b, characterised in that the reactive derivatives of the ureidocarboxylic acids of general formula IV which are used are their acid anhydrides, their reactive esters or reactive amides or their acid halides and the reactive esters of the compounds of general formula V which are used are the diphenylmethyl ester, the t-butyl ester, the trimethylsilyl ester or the N,O-bis-trimethylsilyl derivative and in that the reaction is effected in the presence of a base and/or an organic solvent and/or a condensation agent and, if desired, the product of general formula I thus obtained, in which E represents a separable protective group, is subsequently converted into a compound of general formula I in which E represents a hydrogen atom.

4. Process according to claim 1 for the preparation of a compound of general formula I or I' in which in X the symbol E represents the pivaloyloxymethyl radical, characterised in that a compound of general formula I or I' in which E represents a hydrogen atom is converted into its alkali metal salt and this is reacted with a pivaloyloxymethyl halide of general formula

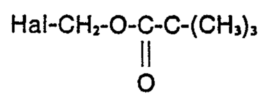

in which Hal represents chlorine, bromine or iodine, in a solvent at temperatures between 20 and 45°C.

**Revendications**

pour les Etats contractants: BE, CH, DE, FR, GB, IT, LI, LU, NL, SE.

1. Nouvelles β-lactames de formules générales

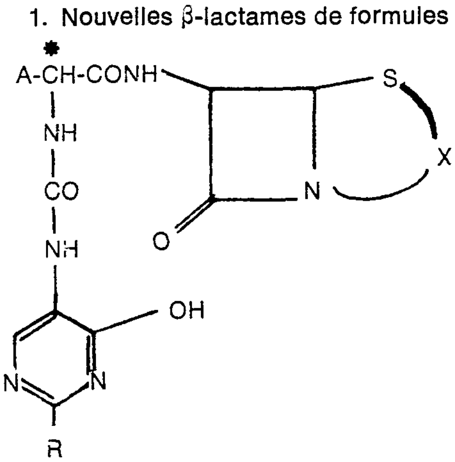

(I)

ou respectivement

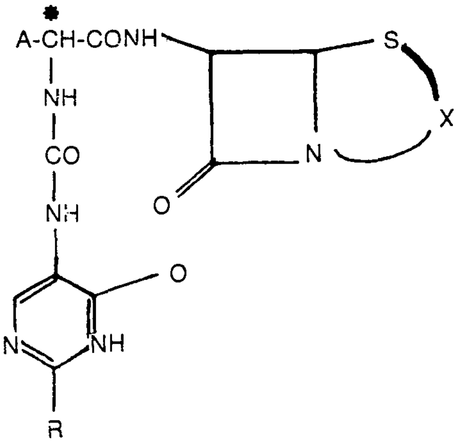

(I')

dans lesquelles

A représente le groupe phényle, 4-hydroxyphényle, 2- ou 3-thiényle, 2- ou 3- furyle, cyclohexyle, cyclohexène-1-yle, cyclohexa-1,4-diène-1-yle ainsi qu'un radical phényle disubstitué en position 3,4, les substituants de ce radical, identiques ou différents l'un de l'autre, pouvant être des atomes de chlore, des groupes hydroxy ou méthoxy,

R représente un groupe de formule générale $NH(CH_2)_nR_1$, dans laquelle n représente 0 ou 1 et $R_1$ représente un radical hétérocyclique à 5 ou 6 membres éventuellement substitué avec de préférence 1 à 4, en particulier 1 ou 2 hétéroatomes identiques ou différents tels que l'oxygène, le soufre ou l'azote, par exemple un groupe thiényle, furyle, pyrrolyle, oxazolyle, isoxazolyle, thiazolyle, isothiazolyle, imidazolyle, pyrazolyle, oxadiazolyle, thiadiazolyle, triazolyle, tétrazolyle, pyridyle, pyrazinyle, pyridazinyle, pyrimidinyle ou tétrahydrofuranyle éventuellement substitué, ces groupes pouvant être substitués par les radicaux suivants:

par des groupes alcoyle, par des atomes d'halogènes tels que le fluor, le chlore ou le brome, par des groupes nitro, cyano, amino, alcoyle, ou dialcoylamino, par des groupes alcoylcarbonylamino ou alcoxycarbonylamino, par des groupes hydroxy, alcoxy, alcoylthio, alcoylsulfinyle ou alcoylsulfonyle, par des groupes méthylsulfonylamino, aminocarbonyle, alcoylcarbonyloxy ou alcoxycarbonyle, des groupes aminosulfonyle, alcoylamino ou dialcoylaminosulfonyle, les groupes alcoyle dans ces radicaux pouvant à chaque fois contenir 1 à 4 atomes de carbone, ainsi que par le groupe acide carboxylique ou acide sulfonique; et

X représente les groupes

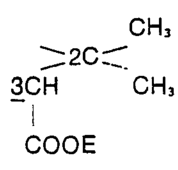 und 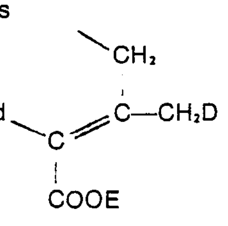

dans lesquels D représente un atome d'hydrogène un groupe hydroxy, acétoxy, aminocarbonyloxy, pyridinium ou 4-aminocarbonyl-pyridinium ou le groupe -SHet, où Het représente le groupe tétrazol-5-yle, 1-méthyl-tétrazol-5-yle, 1,2,4-thiadiazol-5-yle, 3-méthyl-1,2,4-thiadiazol-5-yle, 1,3,4- thiadiazol-2-yle, 2-méthyl-1,3,4-thiadiazol-5-yle, 2- méthylamino-1,3,4-thiadiazol-5-yle, 2-diméthylamino-1,3-4-thiadiazol-5-yle, 2-formylamino-1,3,4-thiadiazol-5-yle, 2-acétylamino-1,3,4-thiadiazol-5-yle, 2-méthyl-1,3,4-oxadiazol-5-yle, 1,2,3-triazol-4-yle ou 1,2,4-triazol-3-yle et

E représente un atome d'hydrogène ou un groupe protecteur facilement clivable in vitro ou in vivo, et, au cas où E représente un atome d'hydrogène, leurs sels physiologiquement supportables avec des bases minérales ou organiques.

2. Nouvelles β-lactames de formules générales

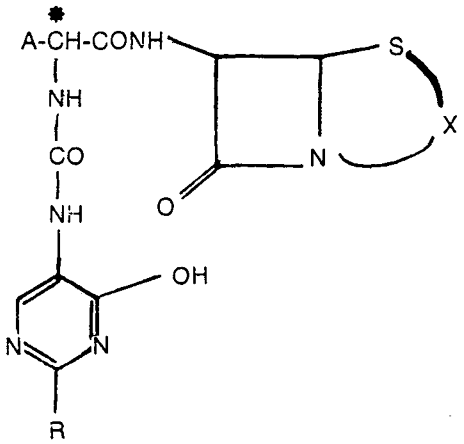

(I)

ou respectivement

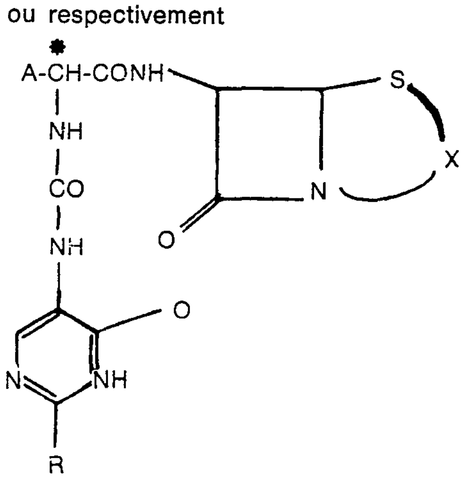

(I')

dans lesquelles

A représente le groupe phényle, 4-hydroxyphényle, 3,4-dihydroxyphényle, 2- ou 3-thiényle, 2- ou 3-furyle,

X est défini comme dans la revendication 1, dans X le symbole

E représente un atome d'hydrogène, le groupe 2-acétoxy-éthyle ou pivaloyloxyméthyle et

D possède les significations données dans la revendication 1, ainsi que

R représente un groupe de formule générale $-NH(CH_2)_nR_1$, le groupe $-(CH_2)_nR_1$ ayant les significations suivantes: le groupe 3-pyridyle,

6-subst.-3-pyridyle, 2-subst.-3-pyridyle, 2-, 3- ou 4-pyridylméthyle, 2-, 4- ou 5-pyrimidinylméthyle, 2-subst.-5-pyrimidinylméthyle, 4-hydroxy-5-pyrimidinyle, 2-subst.-4-hydroxy-5-pyrimidinyle, 5-pyrimidinyle, 2-subst.-5-pyrimidinyle, 4-subst.-2-pyrimidinyle, 4,6-disubst.-2-pyrimidinyle, 2-subst.-4--pyrimidinyle, 2,6-disubst.-4-pyrimidinyle, 5-subst. -2-furyle, 5-subst.-2-thiènyle, 2- ou 3-furylméthylle, 2- ou 3- thiénylméthyle, 5-subst.-2-thiénylméthyle, un groupe 5-subst.-2-furylméthyle ou un groupe 4-subst.-2-thiazole, ces groupes pouvant être substitués par les radicaux suivants:

par des groupes alcoyle avec 1 à 6 atomes de carbone, par des atomes d'halogène, tels que le fluor, le chlore ou le brome, par des groupes nitro, cyano, amino, alcoyle ou dialcoylamino, par des groupes acoylcarbonylamino ou alcoxycarbonylamino, par des groupes hydroxy, alcoxy, alcoylthio, ou alcoylsulfinyle ou alcoylsulfonyle, par des groupes méthylsulfonylamino, aminocarbonyle, alcoylcarbonyloxy ou alcoxycarbonyle, par des groupes aminosulfonyle, alcoylamino- ou dialcoylaminosulfonyle, les groupes alcoyle dans ces radicaux pouvant contenir à chaque fois 1 à 4 atomes de carbone, ainsi que par le groupe acide carboxylique ou acide sulfonique;

et dans le cas où E représente un atome d'hydrogène, leurs sels physiologiquement supportables avec des bases minérales ou organiques.

3. Nouvelles β-lactames de formules générales I ou respectivement I' selon la revendication 1 ou 2, caractérisées en ce que

A représente le groupe phényle, p-hydroxyphényle, 2-thiényle ou le groupe 2- ou 3-furyle,

X est défini comme dans la revendication 1,

E représentant un atome d'hydrogène ou le groupe pivaloyloxyméthyle et

D représentant un atome d'hydrogène, le groupe acétoxy ou aminocarbonyloxy ou le groupe -SHet, dans lequel Het représente le groupe tétrazol-5-yle, le groupe 2-méthyltétrazol-5-yle, le groupe 1,3,4-thiadiazol-5-yle ou le groupe 2-méthyl-1,3,4-thiadiazol-5-yle et le groupe $(CH_2)_nR_1$ dans le radical R ayant les significations suivantes:

le groupe 3-pyridyle, le groupe 6-méthylsulfinyl-3-pyridyle, le groupe 6-méthylsulfonyl-3-pyridyle, le groupe 6-hydroxy-3-pyridyle, le groupe 5-pyrimidinylméthyle ou 2-méthyl-5-pyrimidinylméthyle, le groupe 2-méthyl- ou 2-hydroxy-5-pyrimidinyle, le groupe 6-hydroxy-2-pyrimidinyle, le groupe 4,6-dihydroxy-2-pyrimidinyle, le groupe 3-pyridylméthyle, le groupe 2- furylméthyle, le groupe 2-thiénylméthyle, le groupe 5-aminosulfonyl-2-thiénylméthyle, le groupe 5-aminocarbonyl- ou 5-éthoxycarbonyl-thiényle, et, pour le cas où E représente un atome d'hydrogène leurs sels physiologiquement supportables avec des bases minérales ou organiques.

4. Nouvelles β-lactames de formules générales I ou respectivement I' selon la revendication 1, caractérisées en ce que

A représente le groupe p-hydroxyphényle ou 2-thiényle,

X est défini comme dans la revendication 1, dans X

E représentant un atome d'hydrogène ou le groupe pivaloyloxyméthyle et D représentant le groupe acétoxy ou 1-méthyltétrazol-5-yle et

$(CH_2)_nR_1$ possédant les significations suivantes:

3-pyridylméthyle, 6-méthylsulfinyl-3-pyridyle, 6-méthylsulfonyl-3-pyridyle ou 6-hydroxy-3-pyridyle, le groupe 2-méthyl-5-pyrimidinylméthyle, le groupe 2-hydroxy-5-pyrimidinyle, le groupe 4-hydroxy-2-pyrimidinyle ou le groupe 4,6-dihydroxy--2-pyrimidinyle, le groupe 5-aminocarbonylthiényle, 2-thiénylméthyle ou 5-aminosulfonyl-2-thiénylméthyle ou le groupe 2-furylméthyle et leurs sels physiologiquement supportables avec des bases minérales ou organiques.

5. Nouvelles β-lactames de formules générales I ou respectivement I' selon les revendications 1 à 4, caractérisées en ce que A, R et X avec D possèdent les significations indiquées dans les revendications 1 à 4 et E représente, en tant que groupe facilement clivable, le groupe benzyle, diphénylméthyle, trityle, t-butyle, le groupe 2,2,2-trichloroéthyle, le groupe triméthylsilyle, un groupe alcanoyloxyalcoyle avec 1 à 5 atomes de carbone dans le radical alcanoyle et 1 à 3 atomes de carbone dans le radical alcoylène, le groupe phtalidyle ou indanyle.

6. Nouvelles β-lactames de formule générale I ou respectivement I' selon la revendication 1 caractérisées en ce que

A représente le groupe p-hydroxyphényle et

R réprésente le groupe 5'-aminosulfonyl-2'--thiénylméthylamino, 6'-méthylsulfonyl-3'-pyridylamino ou 2'-méthyl-5'-pyrimidylméthylamino,

X et E sont définis comme dans la revendication 1 et

D représente le groupe (1-méthyl-tétrazol-5--yle)-thio et leurs sels physiologiquement supportables avec des bases minérales ou organiques.

7. Procédé pour la préparation de nouvelles β-lactames de formules générales

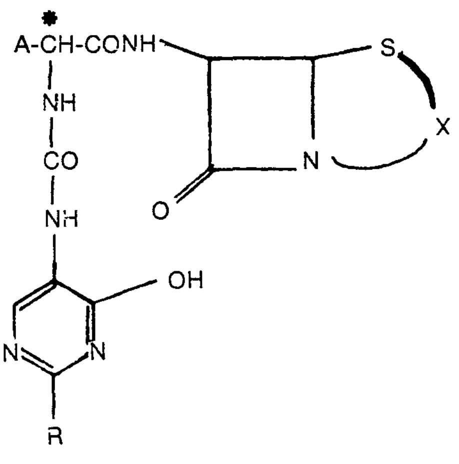

(I)

ou respectivement

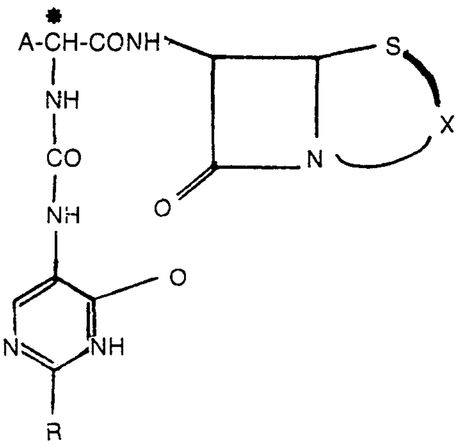

(I')

dans lesquelles

A, R, X, D et E sont définis comme indiqué dans la revendication 1, et pour le cas où E représente un atome d'hydrogène, de leurs sels physiologiquement supportables avec des bases minérales ou organiques, caractérisé en ce que

a) pour préparer un composé de formule générale I dans laquelle D possède les significations indiquées à l'exception de celle d'un groupe pyridinium ou aminocarbonylpyridinium, on fait réagir un composé de formule générale

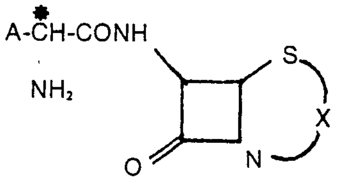

(II)

dans laquelle A et X possèdent les significations indiquées plus haut et D possède les significations indiquées plus haut à l'exception de celle d'un groupe pyridinium ou aminocarbonylpyridinium, avec un dérivé de pyrimidine de formule générale

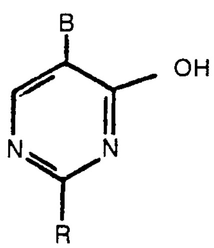

(III)

dans laquelle R est défini comme plus haut et B répresente les groupes =NCO, -NHCOCl, -NHCOBr ou -NH-COO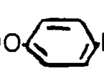$NO_2$, ou avec des mélanges te tels dérivés de pyrimidine de formule générale III dans laquelle B possède en partie l'une et en partie l'autre des significations mentionnées plus haut, dans un solvant et dans un domaine de pH compris entre 2,0 et 9,0 à des températures comprises entre −20°C et +50°C, ou

b) pour préparer un composé de formule générale I dans laquelle D possède les significations indiquées plus haut à l'exception de celle d'un groupe pyridinium ou aminocarbonylpyridinium, on fait réagir un acide uréidocarboxylique de formule générale

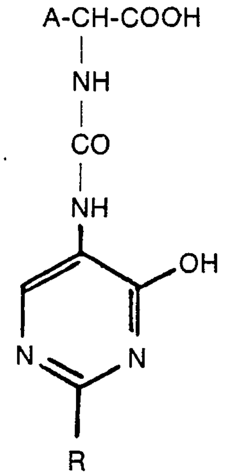

(IV)

dans laquelle A et R possèdent les significations indiquées plus haut, ou ses sels ou dérivés réactifs, avec des composés de formule générale

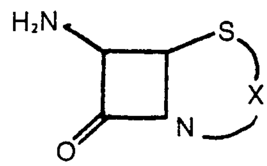

(V)

dans laquelle X possède les significations indiquées plus haut à l'exception de la formule dans laquelle D représente pyridinium ou aminopyridinium, entre −40°C et +40°C en présence d'un solvant et éventuellement en présence d'une base, ou

c) pour préparer des dérivés de céphalosporines de formules générales I ou respectivement I' dans lesquelles D possède les significations d'un groupe -S-Het, pyridinium ou 4-aminocarbonylpyridinium, on fait réagir un composé de formule générale

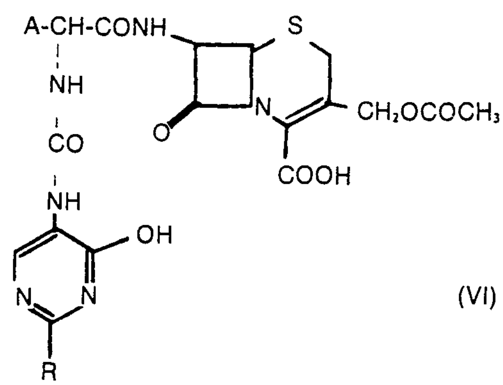

(VI)

dans laquelle A et R ont les significations indiquées plus haut, soit avec un composé de formule générale

Het-S-M (VII)

dans laquelle Het a les significations indiquées plus haut et M représente un atome d'hydrogène ou un métal alcalin ou un métal alcalino--terreux, soit avec la pyridine ou respectivement la 4-aminocarbonylpyrimidine dans un solvant organique ou dans l'eau ou dans des mélanges de ces solvants à une valeur de pH de la solution de réaction entre 2 et 10, de façon préférable entre 4 et 8, à une température dans le domaine de 0° à 100°C et si on le désire on transforme ensuite un composé ainsi obtenu de formules générales I ou I' dans lesquelles E représente un groupe protecteur facilement clivable in vitro, en l'acide carboxylique libre de formules générales I ou respectivement I' dans lesquelles E est un atome d'hydrogène et/ou on transforme un composé de formules générales I ou I' dans lesquelles E représente un atome d'hydrogène en ses éthers ou, au moyen de bases minérales ou organiques, en les sels correspondants.

8. Procédé selon la revendication 7a, caractérisé en ce que qu'un composé de formule générale II dans laquelle E représente l'hydrogène, ou l'un des ses sels avec des bases minérales ou organiques est mis à réagir avec un composé de formule générale III,

a) dans l'eau ou dans des solvants miscibles à l'eau en présence d'eau dans un domaine de pH de 6,5 et 8,0, ou

b) dans des solvants anhydres ou

c) dans un mélange d'eau et de solvants non miscibles à l'eau dans un domaine de pH compris entre 6,5 et 8,0, ou en ce qu'on fait réagir un composé de formule générale II dans laquelle E représente un groupe silyle ou un autre groupe protecteur facilement clivable, avec un composé de formule générale III dans un solvant aprotique anhydre et ne comportant pas de groupe hydroxyle, éventuellement en présence d'une base.

9. Procédé selon la revendication 7b, caractérisé en ce qu'on utilise en tant que dérivés réactifs des acides uréidocarboxyliques de formule générale IV leurs anhydrides d'acides, leurs esters réactifs ou amides réactifs ou leurs halogénures d'acides, en tant qu'esters réactifs des composés de formule générale V l'ester diphénylméthylé, l'ester t-butylé, l'ester triméthylsilylé ou le dérivé N,O-bis-triméthylsilylé et en ce que la réaction a lieu en présence d'une base et/ou d'un solvant organique et/ou d'un agent de condensation, et si on le désire on transforme ensuite le produit obtenu de formule générale I dans laquelle E représente un groupe protecteur clivable, en un composé de formule générale I dans laquelle E représente un atome d'hydrogène.

10. Procédé selon la revendication 7 pour la préparation d'un composé de formule générale I ou I' dans laquelle dans X le symbole E représente le radical pivaloyloxyméthyle, caractérisé en ce qu'on transforme un composé de formule générale I ou I' dans laquelle E représente un atome d'hydrogène en son sel alcalin et on fait réagir celui-ci avec un halogènure de pivaloyloxyméthyle de formule générale

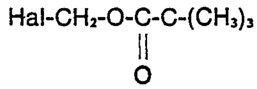

dans laquelle Hal représente le chlore, le brome ou l'iode, dans un solvant à des températures entre 20 et 45°C.

11. Médicament caractérisé par une tenueur en l'un ou plusieurs des composés de formules générales I ou respectivement I' selon les revendications 1 à 6 conjointement aux excipients et/ou adjuvants habituels.

**Revendications**
pour l'Etat contractant: AT

1. Procédé de préparation de nouvelles β-lactames de formules générales

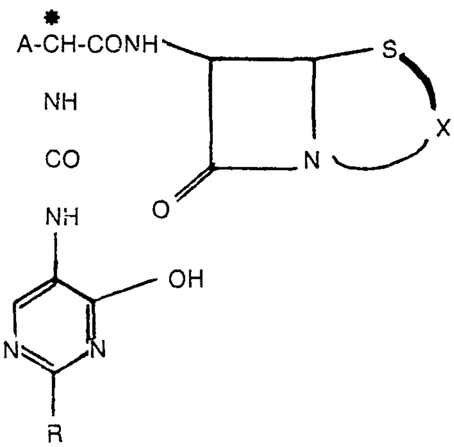
(I)

ou respectivement

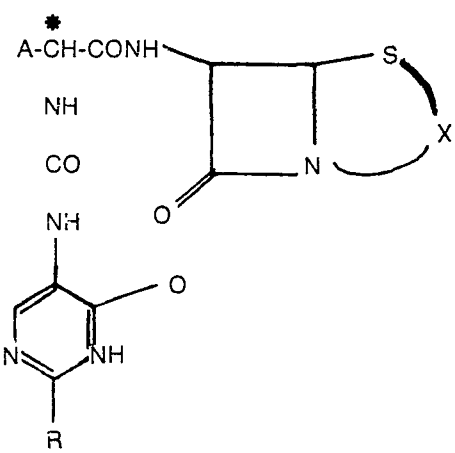
(I')

dans lesquelles

A représente le groupe phényle, 4-hydroxyphényle, 2- ou 3-thiényle, 2- ou 3- furyle, cyclohexyle, cyclohexène-1-yle, cyclohexa-1,4-diène--1-yle ainsi qu'un radical phényle disubstitué en position 3,4, les substituants de ce radical, identiques ou différents l'un de l'autre, pouvant être des atomes de chlore, des groupes hydroxy ou méthoxy, et

R représente un groupe de formule $NH(CH_2)_n-R_1$, dans laquelle n représente 0 ou 1 et $R_1$ représente un radical hétérocyclique à 5 ou 6 membres éventuellement substitué avec de préférence 1 à 4, en particulier 1 ou 2 hétéroatomes identiques ou différents tels que l'oxygène, le soufre ou l'azote, par exemple un groupe thiényle, furyle, pyrrolyle, oxazolyle, isoxazolyle, thiazolyle, isothiazolyle, imidazolyle, pyrazolyle, oxdiazolyle, thiadiazolyle, triazolyle, tétrazolyle, pyridyle, pyrazinyle, pyridazinyle, pyrimidinyle ou tétrahydrofuranyle éventuellement substitué, ces groupes pouvant être substituées par les radicaux suivants:

par des groupes alcoyle, par des atomes d'halogènes tels que le fluor, le chlore ou le brome, par des groupes nitro, cyano, amino, alcoyle, ou dialcoylamino, par des groupes alcoylcarbonylamino ou alcoxycarbonylamino, par des groupes hydroxy, alcoxy, alcoylthio, alcoylsulfinyle ou alcoylsulfonyle, par des groupes méthylsulfonylamino, aminocarbonyle, alcoylcarbonyloxy ou alcoxycarbonyle, des groupes aminosulfonyle, alcoylamino ou dialcoylaminosulfonyle, les groupes alcoyle dans ces radicaux pouvant à chaque fois contenir 1 à 4 atomes de carbone, ainsi que par le groupe acide carboxylique ou acide sulfonique; et

X représente les groupes

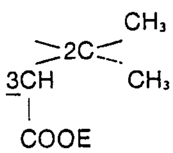 und 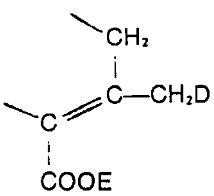

dans lesquels D représente un atome d'hydrogène un groupe hydroxy, acétoxy, aminocarbonyloxy, pyridinium ou 4-aminocarbonyl-pyridinium ou le groupe -SHet, où Het représente le groupe tétrazol-5-yle, 1-méthyl-tétrazol-5-yle, 1,2,4-thiadiazol-5-yle, 3-méthyl-1,2,4-thiadiazol-5--yle, 1,3,4-thiadiazol-2-yle, 2-méthyl-1,3,4-thiadiazol-5-yle, 2-méthylamino-1,3,4-thiadiazol-5-yle, 2--diméthylamino-1,3,4-thiadiazol-5-yle, 2-formylamino-1,3,4-thiadiazol-5-yle, 2-acéthylamino-1,3,-4-thiadiazol-5-yle, 2-méthyl-1,3,4-oxadiazol-5-yle, 1,2,3-triazol-4-yle ou 1,2,3-triazol-4-yle ou 1,2,4--triazol-3-yle et

E représente un atome d'hydrogène ou un groupe protecteur facilement clivable in vitro ou in vivo, et lorsque E représente un atome d'hydrogène, de leurs sels physiologiquement supportables avec des bases minérales ou organiques, caractérisé en ce que

a) pour préparer un composé de formule générale I dans laquelle D possède les significations indiquées à l'exception de celle d'un groupe pyridinium ou aminocarbonylpyridinium, on fait réagir un composé de formule

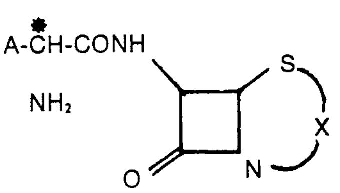

(II)

dans laquelle A et X possèdent les significations indiquées plus haut et D possède les significations indiquées plus haut à l'exception de celle d'un groupe pyridinium ou aminocarbonylpyridinium, avec un dérivé de pyrimidine de formule générale

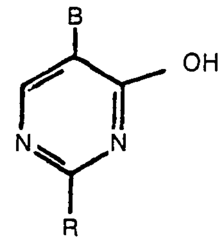

(III)

dans laquelle R est défini comme plus haut et B représente les groupes =NCO, -NHCOCl, -NH-COBr ou -NH-COO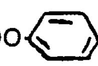NO₂, ou avec des mélanges de tels dérivés de pyrimidine de formule générale III, dans laquelle B possède en partie l'une et en partie l'autre des significations mentionnées plus haut, dans un solvant et dans un domaine de pH compris entre 2,0 et 9,0 à des températures comprises entre −20°C et +50°C, ou

b) pour préparer un composé de formule générale I dans laquelle D possède les significations indiquées plus haut à l'exception de celle d'un groupe pyridinium ou aminocarbonylpyridinium, on fait réagir un acide uréidocarboxylique de formule générale

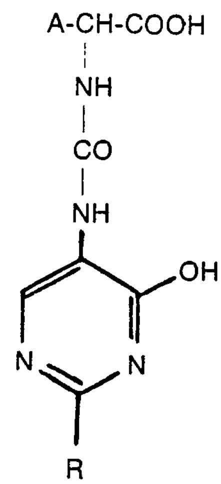

(IV)

dans laquelle A et R possèdent les significations indiquées plus haut, ou ses sels ou dérivés réactifs, avec des composés de formule générale

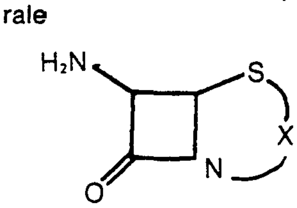

(V)

dans laquelle X possède les significations indiquées plus haut à l'exception de la formule dans laquelle D représente pyridinium ou aminopyridinium, entre −40°C et +40°C en présence

d'un solvant et éventuellement en présence d'une base, ou

c) pour préparer des dérivés de céphalosporines de formules générales I ou respectivement I' dans lesquelles D possède les significations d'un groupe -S-Het, pyridinium ou 4-aminocarbonylpyridinium, on fait réagir un composé de formule générale

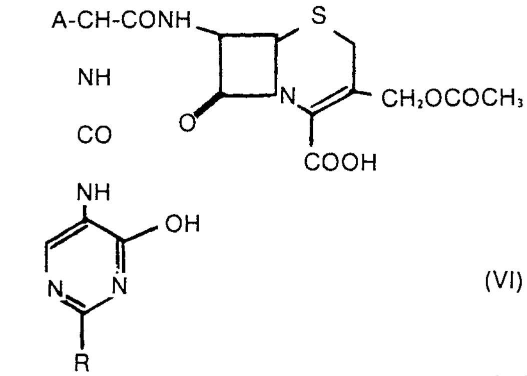

(VI)

qui tombe dans le cadre de la formule générale I et dans laquelle A et R ont les significations indiquées plus haut, soit avec un composé de formule générale

Het-S-M (VII)

dans laquelle Het a les significations indiquées plus haut et M représente un atome d'hydrogène ou un métal alcalin ou un métal alcalino-terreux, soit avec la pyridine ou respectivement la 4-aminocarbonylpyridine dans und solvant organique ou dans l'eau ou dans des mélanges de ces solvants à une valeur de pH de la solution de réaction compris entre 2 et 10, de manière avantageuse entre 4 et 8, à une température dans le domaine de 0° à 100°C et si on le désire on transforme ensuite un composé ainsi obtenu de formules générales I ou I', dans lesquelles E représente un groupe protecteur facilement clivable in vitro en l'acide carboxylique libre de formules générales I ou respectivement I' dans lesquelles E est un atome d'hydrogène et/ou on transforme un composé de formules générales I ou I' dans lesquelles E représente un atome d'hydrogène en leurs esters ou, au moyen de bases minérales ou organiques, en les sels correspondants.

2. Procédé selon la revendication 1a, caractérisé en ce qu'on fait réagir un composé de formule générale II dans laquelle E représente l'hydrogène, ou l'un de ses sels avec des bases minérales ou organiques, avec un composé de formule générale III,

a) dans l'eau ou dans des solvants miscibles à l'eau en présence d'eau dans un domaine de pH de 6,5 à 8,0, ou

b) dans des solvants anhydres ou

c) dans un mélange d'eau et de solvants non miscibles à l'eau dans un domaine de pH compris entre 6,5 et 8,0, ou, en ce qu'on fait réagir un composé de formule générale II dans laquelle E représente un groupe silyle ou un autre groupe protecteur facilement clivable, avec un composé de formule générale III dans un solvant aprotique anhydre et ne comportant pas de groupe hydroxyle, éventuellement en présence d'une base.

3. Procédé selon la revendication 1b, caractérisé en ce qu'on utilise en tant que dérivés réactifs des acides uréidocarboxyliques de formule IV leurs anhydrides d'acides, leurs esters réactifs ou amides réactifs ou leurs halogènures d'acides, en tant qu'esters réactifs des composés de formule générale V l'ester diphénylméthylé, l'ester t-butylé, l'ester triméthylsilylé ou le dérivé N,O-bis-triméthylsilylé et en ce que la réaction a lieu en présence d'une base et/ou d'un solvant organique et/ou d'un agent de condensation et, si on le désire, on transforme ensuite le produit ainsi obtenu de formule générale I dans laquelle E représente un groupe protecteur clivable en un composé de formule générale I dans laquelle E représente un atome d'hydrogène.

4. Procédé selon la revendication 1 pour préparer un composé de formule générale I ou I' ou, dans X, le symbole E représente le radical pivaloyloxyméthyle, caractérisé en ce qu'on transforme un composé de formule générale I ou I' dans laquelle E représente un atome d'hydrogène en son sel alcalin et ce qu'on fait réagir celui-ci avec un halogènure de pivaloyloxyméthyle de formule générale

$$Hal\text{-}CH_2\text{-}O\text{-}\underset{\underset{O}{\|}}{C}\text{-}C\text{-}(CH_3)_3$$

dans laquelle Hal représente le chlore, le brome ou l'iode, dans un solvanta à des températures entre 20 et 45°C.